# EUROPEAN PATENT APPLICATION

(11) **EP 4 379 054 A1**
(43) Date of publication of application: **05.06.2024**
(21) Application number: 22849476.1
(22) Date of filing: 26.07.2022
(51) Int. Cl.: C12N 15/12, C07K 7/00, C07K 14/00, C12N 1/15, C12N 1/19, C12N 1/21, C12N 5/10, C12N 15/13, C12N 15/62, C12N 15/63

(54) **PEPTIDE TAG AND NUCLEIC ACID ENCODING SAME**

(30) Priority: 27.07.2021 JP 2021122510
(71) Applicant: Stand Therapeutics Co., Ltd., Tokyo 107-0062 (JP)
(72) Inventor: KABAYAMA, Hiroyuki, Tokyo 107-0062 (JP)
(74) Representative: Marks & Clerk LLP
(86) International application number: PCT/JP2022/028746
(87) International publication number: WO 2023/008415

(57) **Abstract**

The present disclosure provides a peptide tag, and a nucleic acid encoding the peptide tag. The peptide tag of the present disclosure can reduce an aggregation property of a protein in a cell. Specifically, the peptide tag of the present disclosure can be a peptide tag in which 5% or more and less than 45% of amino acids contained in an amino acid sequence thereof are acidic amino acids, and (b) 20% or more of the amino acids contained in the amino acid sequence are amino acids selected from the group consisting of F, P, Y, G, S, Q, N, and A.

## Description

### Technical Field

The present disclosure relates to a peptide tag, and a nucleic acid encoding the peptide tag.

### Background Art

An antibody functioning in a cell, namely, an intrabody (intracellular antibody) can affect the function of the cell by recognizing and binding to an antigen (target molecule) in the cell of a higher organism. Such an antigen can be a significant intracellular therapeutic target that can be inactivated by binding to an intracellular antibody. As a research method, use of an intracellular antibody attracts attention as means for specifically inhibiting the function of a protein directly by binding to the antibody in the cell.

In case of an intracellular antibody, a hybridoma producing a monoclonal antibody recognizing an antigen is first produced by a standard method, and from the cDNA thereof, an intracellular expression vector containing a DNA encoding a single chain antibody (single chain Fv: scFv) is constructed to obtain a complex of a heavy chain (VH) and a light chain (VL) as an intracellular antibody. Recently, a phage library for presenting a human scFv from an antibody isolated from a human B cell is produced, and is used for isolation of a scFv binding to an intracellular antigen in some cases.

An antibody usually moves around in an extracellular space such as blood in a body, and recognizes an extracellular antigen to function, and hence works in the extracellular space as a premise. Accordingly, if an antibody is expressed in the cytoplasm, there arise problems of reduction of the expression level, folding causing limitation of a half-life of an antibody domain, and stability. The problem of stability of an intracellular antibody in the cytoplasm can lead to formation of an aggregation of the intracellular antibody in the cytoplasm. The formation of the aggregation can lead to reduction of a production amount of the intracellular antibody, and inhibition of expression of normal function. The same applies to a protein except for the intracellular antibody. The intracellular antibody has a characteristic of easily aggregating in particular, but a protein except for the intracellular antibody also can form an aggregation in the cytoplasm when produced in the cytoplasm.

By contrast, it has been shown that a peptide tag having an amino acid sequence containing 45% or more of acidic amino acids improves stability of an intracellular antibody (Patent Literature 1, and Non Patent Literature 1). In proposing the effectiveness of a peptide tag having an amino acid sequence containing 45% or more of acidic amino acids, Patent Literature 1 and Non Patent Literature 1 point out, as a design guideline for the peptide tag, significance of designing the peptide tag in such a manner that a charge value and a pI value are sufficiently low based not on the pH environment of the cytoplasm but on the pH environment on the surface of an endosome on the side of the cytoplasm. In Non Patent Literature 3, a membrane localization signal of HRAS is added to a heavy chain variable region of an antibody.

### Citation List

### Patent Literature

Patent Literature 1: WO2019/004213

### Non Patent Literature

Non Patent Literature 1: Kabayama et al., 2020, Nature Communication, 11, 336
Non Patent Literature 2: Shubhada et al., 2012, Biochemical genetics, Vol. 50, No. 7-8, pp. 625-41
Non Patent Literature 3: Tanaka et al., 2007, EMBO Journal, 26:3250-3259

### Summary of Invention

The present disclosure provides a peptide tag, and a nucleic acid encoding the peptide tag. The peptide tag of the present disclosure can reduce the aggregation property of a protein in a cell.

The present inventors made earnest studies on peptide tags having various amino acid sequences, resulting in finding a peptide tag having an effect of reducing an aggregation property of a protein in a cell.

The present disclosure provides the following inventions:
[1] A peptide having an amino acid sequence with a length of, for example, 600 amino acids or less, for example, 10 to 200 amino acids (for example, 10 to 90 amino acids),
   wherein (a) 5% or more and less than 45% of amino acids contained in the amino acid sequence are acidic amino acids, and
   (b) 20% or more, and preferably 30% or more of the amino acids contained in the amino acid sequence are amino acids selected from the group consisting of F, P, Y, G, S, Q, N, and A, and
   the peptide is preferably capable of reducing an aggregation property in a cell of a protein linked to the peptide, wherein 10% or 15% or more of the amino acids contained in the amino acid sequence are N or P.
[2] The peptide according to [1] above, wherein 30% or less, preferably 20% or less, more preferably 15% or less, and further preferably 10% or less of the amino acids contained in the amino acid sequence are amino acids selected from the group consisting of M, T, W, C, I, V, and L.
[3] The peptide according to [1] or [2] above, wherein each of A and G constitutes less than 10% of the amino acids contained in the amino acid sequence thereof.
[4] The peptide according to any one of [1] to [3] above, wherein
   (a) 20% or more and less than 45% of the amino acids contained in the amino acid sequence are acidic amino acids,
   (b) 30% or more and less than 70% of the amino acids contained in the amino acid sequence are amino acids selected from the group consisting of F, P, Y, G, S, Q, N, and A,
   (c) 20% or less of the amino acids contained in the amino acid sequence are amino acids selected from the group consisting of M, T, W, C, I, V, and L, and
   (d) each of A and G constitutes less than 10% of the amino acids contained in the amino acid sequence.
[5] A peptide having an amino acid sequence set forth in any one of SEQ ID NOs: 2 to 11.
[6] A nucleic acid encoding the peptide according to any one of [1] to [5] above.
[7] A protein expression vector comprising: the nucleic acid according to [6] above operably linked to a regulatory sequence; and a nucleic acid encoding a protein of interest in-frame to the nucleic acid according to [6] above.
[8] The protein expression vector according to [7] above, wherein the protein of interest is an antibody, or an antigen-binding fragment of an antibody.
[9] The protein expression vector according to [8] above, wherein the antigen-binding fragment of the antibody is a single chain Fv (scFv).
[10] A fusion protein of the peptide according to any one of [1] to [5] above and a protein of interest.
[11] The fusion protein according to [10] above, wherein the protein of interest is an antibody, or an antigen-binding fragment of an antibody.
[12] The fusion protein according to [11] above, wherein the antigen-binding fragment of the antibody is a single chain Fv (scFv).
[13] A protein-producing cell comprising: the nucleic acid according to [6] above operably linked to a regulatory sequence; and a nucleic acid encoding a protein of interest in-frame to the nucleic acid according to [6] above.
[14] A method for selecting or identifying an amino acid sequence having a length of 600 amino acids or less, for example, 10 to 200 amino acids (for example, 10 to 90 amino acids), the method comprising:
   acquiring, from an amino acid sequence (group) (that can include an amino acid sequence (group) having a length of 10 to 200 amino acids (for example, 10 to 90 amino acids)), an amino acid sequence (group) in which (a) 5% or more and less than 45% of amino acids contained in the amino acid sequence are acidic amino acids, and (b) 20% or more of the amino acids contained in the amino acid sequence are amino acids selected from the group consisting of F, P, Y, G, S, Q, N, and A;
   selecting or identifying an amino acid sequence of a peptide tag that, when the fusion protein of a peptide tag having the selected or identified amino acid sequence and a reference protein is expressed in a mammal cell (preferably in a human cell), provides reduction of a proportion of cells in which the fusion protein forms an aggregation (for example, the proportion which is not more than a predetermined value); and
   obtaining a peptide tag having the amino acid sequence, or a nucleic acid encoding the peptide tag.
[15] The method according to claim 14, wherein the amino acid sequence group to be acquired is the peptide according to any one of [1] to [5] above.
[16] The method according to [14] or [15] above, wherein the amino acid sequence group to be acquired is a group of amino acid sequences encoded by coding regions of human genome.
[17] The method according to any one of [14] to [16] above, wherein the amino acid sequence to be acquired contains a neo-antigen.
[18] A peptide satisfying one or more selected from the group consisting of (a) to (h) described below, and capable of reducing an aggregation property in a cell of a protein linked to the peptide.
[19] A peptide selected from the group consisting of (A) to (AE) and (AF) to (AU) described below, and capable of reducing an aggregation property in a cell of a protein linked to the peptide.
[20] A nucleic acid encoding the peptide according to [18] above.
[21] A protein expression vector comprising: the nucleic acid according to [20] above operably linked to a regulatory sequence; and a nucleic acid encoding a protein of interest in-frame to the nucleic acid according to [20] above.
[22] A fusion protein of the peptide according to [18] above and a protein of interest.
[23] The fusion protein according to [22] above, wherein the protein of interest is an antibody, or an antigen-binding fragment of an antibody.
[24] The fusion protein according to [23] above, wherein the antigen-binding fragment of the antibody is a single chain Fv (scFv).
[25] A nucleic acid encoding the peptide according to [19] above.
[26] A protein expression vector comprising: the nucleic acid according to [25] above operably linked to a regulatory sequence; and a nucleic acid encoding a protein of interest in-frame to the nucleic acid according to [25] above.
[27] A fusion protein of the peptide according to [25] above, and a protein of interest.
[28] The fusion protein according to [26] above, wherein the protein of interest is an antibody, or an antigen-binding fragment of an antibody.
[29] The fusion protein according to [27] above, wherein the antigen-binding fragment of the antibody is a single chain Fv (scFv).
[30] The method according to [14] above, wherein the amino acid sequence (group) includes the peptide according to [18] above.
[31] The method according to [14] above, wherein the amino acid sequence (group) includes the peptide according to [19] above.
[32] The method according to [14] above, wherein the reference protein is a scFv, and the predetermined value is a value of 30% or less.
[33] The method according to [14] above, wherein the reference protein is a scFv, and the predetermined value is a value of 20% or less.
[34] The method according to [14] above, wherein the reference protein is a scFv, and the predetermined value is a value of 15% or less.
[35] The method according to [14] above, wherein the reference protein is a scFv, and the predetermined value is a value of 10% or less.
[36] The method according to [14] above, wherein the reference protein is a scFv, and the predetermined value is a value of 5% or less.
[37] The method according to [14] above, wherein the reference protein is a scFv, the amino acid sequence group includes the peptide according to (A) described below, and the amino acid sequence to be acquired preferably further satisfies a condition of (A) described below.
[38] The method according to [14] above, wherein the reference protein is a scFv, the amino acid sequence group includes the peptide according to (B) described below, and the amino acid sequence to be acquired preferably further satisfies a condition of (B) described below.
[39] The method according to [14] above, wherein the reference protein is a scFv, the amino acid sequence group includes the peptide according to (C) described below, and the amino acid sequence to be acquired preferably further satisfies a condition of (C) described below.
[40] The method according to [14] above, wherein the reference protein is a scFv, the amino acid sequence group includes the peptide according to (D) described below, and the amino acid sequence to be acquired preferably further satisfies a condition of (D) described below.
[41] The method according to [14] above, wherein the reference protein is a scFv, the amino acid sequence group includes the peptide according to (E) described below, and the amino acid sequence to be acquired preferably further satisfies a condition of (E) described below.
[42] The method according to [14] above, wherein the reference protein is a scFv, the amino acid sequence group includes the peptide according to (F) described below, and the amino acid sequence to be acquired preferably further satisfies a condition of (F) described below.
[43] The method according to [14] above, wherein the reference protein is a scFv, the amino acid sequence group includes the peptide according to (G) described below, and the amino acid sequence to be acquired preferably further satisfies a condition of (G) described below.
[44] The method according to [14] above, wherein the reference protein is a scFv, the amino acid sequence group includes the peptide according to (H) described below, and the amino acid sequence to be acquired preferably further satisfies a condition of (H) described below.
[45] The method according to [14] above, wherein the reference protein is a scFv, the amino acid sequence group includes the peptide according to (I) described below, and the amino acid sequence to be acquired preferably further satisfies a condition of (I) described below.
[46] The method according to [14] above, wherein the reference protein is a scFv, the amino acid sequence group includes the peptide according to (J) described below, and the amino acid sequence to be acquired preferably further satisfies a condition of (J) described below.
[47] The method according to [14] above, wherein the reference protein is a scFv, the amino acid sequence group includes the peptide according to (K) described below, and the amino acid sequence to be acquired preferably further satisfies a condition of (K) described below.
[48] The method according to [14] above, wherein the reference protein is a scFv, the amino acid sequence group includes the peptide according to (L) described below, and the amino acid sequence to be acquired preferably further satisfies a condition of (K) described below.
[49] The method according to [14] above, wherein the reference protein is a scFv, the amino acid sequence group includes the peptide according to (M) described below, and the amino acid sequence to be acquired preferably further satisfies a condition of (M) described below.
[50] The method according to [14] above, wherein the reference protein is a scFv, the amino acid sequence group includes the peptide according to (N) described below, and the amino acid sequence to be acquired preferably further satisfies a condition of (N) described below.
[51] The method according to [14] above, wherein the reference protein is a scFv, the amino acid sequence group includes the peptide according to (O) described below, and the amino acid sequence to be acquired preferably further satisfies a condition of (O) described below.
[52] The method according to [14] above, wherein the reference protein is a scFv, the amino acid sequence group includes the peptide according to (P) described below, and the amino acid sequence to be acquired preferably further satisfies a condition of (P) described below.
[53] The method according to [14] above, wherein the reference protein is a scFv, the amino acid sequence group includes the peptide according to (Q) described below, and the amino acid sequence to be acquired preferably further satisfies a condition of (Q) described below.
[54] The method according to [14] above, wherein the reference protein is a scFv, the amino acid sequence group includes the peptide according to (R) described below, and the amino acid sequence to be acquired preferably further satisfies a condition of (R) described below.
[55] The method according to [14] above, wherein the reference protein is a scFv, the amino acid sequence group includes the peptide according to (S) described below, and the amino acid sequence to be acquired preferably further satisfies a condition of (S) described below.
[56] The method according to [14] above, wherein the reference protein is a scFv, the amino acid sequence group includes the peptide according to (T) described below, and the amino acid sequence to be acquired preferably further satisfies a condition of (T) described below.
[57] The method according to [14] above, wherein the reference protein is a scFv, the amino acid sequence group includes the peptide according to (U) described below, and the amino acid sequence to be acquired preferably further satisfies a condition of (U) described below.
[58] The method according to [14] above, wherein the reference protein is a scFv, the amino acid sequence group includes the peptide according to (V) described below, and the amino acid sequence to be acquired preferably further satisfies a condition of (V) described below.
[59] The method according to [14] above, wherein the reference protein is a scFv, the amino acid sequence group includes the peptide according to (W) described below, and the amino acid sequence to be acquired preferably further satisfies a condition of (W) described below.
[60] The method according to [14] above, wherein the reference protein is a scFv, the amino acid sequence group includes the peptide according to (X) described below, and the amino acid sequence to be acquired preferably further satisfies a condition of (X) described below.
[61] The method according to [14] above, wherein the reference protein is a scFv, the amino acid sequence group includes the peptide according to (Y) described below, and the amino acid sequence to be acquired preferably further satisfies a condition of (Y) described below.
[62] The method according to [14] above, wherein the reference protein is a scFv, the amino acid sequence group includes the peptide according to (Z) described below, and the amino acid sequence to be acquired preferably further satisfies a condition of (Z) described below.
[63] The method according to [14] above, wherein the reference protein is a scFv, the amino acid sequence group includes the peptide according to (AA) described below, and the amino acid sequence to be acquired preferably further satisfies a condition of (AA) described below.
[64] The method according to [14] above, wherein the reference protein is a scFv, the amino acid sequence group includes the peptide according to (AB) described below, and the amino acid sequence to be acquired preferably further satisfies a condition of (AB) described below.
[65] The method according to [14] above, wherein the reference protein is a scFv, the amino acid sequence group includes the peptide according to (AC) described below, and the amino acid sequence to be acquired preferably further satisfies a condition of (AC) described below.
[66] The method according to [14] above, wherein the reference protein is a scFv, the amino acid sequence group includes the peptide according to (AD) described below, and the amino acid sequence to be acquired preferably further satisfies a condition of (AD) described below.
[67] The method according to [14] above, wherein the reference protein is a scFv, the amino acid sequence group includes the peptide according to (AE) described below, and the amino acid sequence to be acquired preferably further satisfies a condition of (AE) described below.
   [67A] The method according to [14] above, wherein the reference protein is a scFv, the amino acid sequence group includes the peptide according to (AF) described below, and the amino acid sequence to be acquired preferably further satisfies a condition of (AF) described below.
   [67B] The method according to [14] above, wherein the reference protein is a scFv, the amino acid sequence group includes the peptide according to (AG) described below, and the amino acid sequence to be acquired preferably further satisfies a condition of (AG) described below.
   [67C] The method according to [14] above, wherein the reference protein is a scFv, the amino acid sequence group includes the peptide according to (AH) described below, and the amino acid sequence to be acquired preferably further satisfies a condition of (AH) described below.
   [67D] The method according to [14] above, wherein the reference protein is a scFv, the amino acid sequence group includes the peptide according to (AI) described below, and the amino acid sequence to be acquired preferably further satisfies a condition of (AI) described below.
   [67E] The method according to [14] above, wherein the reference protein is a scFv, the amino acid sequence group includes the peptide according to (AJ) described below, and the amino acid sequence to be acquired preferably further satisfies a condition of (AJ) described below.
   [67F] The method according to [14] above, wherein the reference protein is a scFv, the amino acid sequence group includes the peptide according to (AK) described below, and the amino acid sequence to be acquired preferably further satisfies a condition of (AK) described below.
   [67G] The method according to [14] above, wherein the reference protein is a scFv, the amino acid sequence group includes the peptide according to (AL) described below, and the amino acid sequence to be acquired preferably further satisfies a condition of (AL) described below.
   [67H] The method according to [14] above, wherein the reference protein is a scFv, the amino acid sequence group includes the peptide according to (AM) described below, and the amino acid sequence to be acquired preferably further satisfies a condition of (AM) described below.
   [67I] The method according to [14] above, wherein the reference protein is a scFv, the amino acid sequence group includes the peptide according to (AN) described below, and the amino acid sequence to be acquired preferably further satisfies a condition of (AN) described below.
   [67J] The method according to [14] above, wherein the reference protein is a scFv, the amino acid sequence group includes the peptide according to (AO) described below, and the amino acid sequence to be acquired preferably further satisfies a condition of (AO) described below.
   [67K] The method according to [14] above, wherein the reference protein is a scFv, the amino acid sequence group includes the peptide according to (AP) described below, and the amino acid sequence to be acquired preferably further satisfies a condition of (AP) described below.
   [67L] The method according to [14] above, wherein the reference protein is a scFv, the amino acid sequence group includes the peptide according to (AQ) described below, and the amino acid sequence to be acquired preferably further satisfies a condition of (AQ) described below.
   [67M] The method according to [14] above, wherein the reference protein is a scFv, the amino acid sequence group includes the peptide according to (AR) described below, and the amino acid sequence to be acquired preferably further satisfies a condition of (AR) described below.
   [67N] The method according to [14] above, wherein the reference protein is a scFv, the amino acid sequence group includes the peptide according to (AS) described below, and the amino acid sequence to be acquired preferably further satisfies a condition of (AS) described below.
   [670] The method according to [14] above, wherein the reference protein is a scFv, the amino acid sequence group includes the peptide according to (AT) described below, and the amino acid sequence to be acquired preferably further satisfies a condition of (AT) described below.
   [67P] The method according to [14] above, wherein the reference protein is a scFv, the amino acid sequence group includes the peptide according to (AU) described below, and the amino acid sequence to be acquired preferably further satisfies a condition of (AU) described below.
[68] The method according to any one of [37] to [67] and [67A] to [67N] above, wherein the reference protein is a scFv, and the predetermined value is a value of 15% or less.
[69] The method according to any one of [37] to [67] and [67A] to [67N] above, wherein the reference protein is a scFv, and the predetermined value is a value of 10% or less.
[70] The method according to any one of [37] to [67] and [67A] to [67N] above, wherein the reference protein is a scFv, and the predetermined value is a value of 5% or less.
[71] The method according to any one of [37] to [70] above, wherein a proportion of cells in which the reference protein forms an aggregation is a value more than 30%.
[72] The method according to any one of [37] to [70] above, wherein a proportion of cells in which the reference protein forms an aggregation is a value in a range of 30 to 40%.
[73] The method according to any one of [37] to [70] above, wherein a rate of cells in which the reference protein forms an aggregation is a value in a range of 40 to 50%.
[74] The method according to any one of [37] to [70] above, wherein a rate of cells in which the reference protein forms an aggregation is a value in a range of 50 to 60%.
[75] The method according to any one of [37] to [70] above, wherein a rate of cells in which the reference protein forms an aggregation is a value in a range of 60 to 70%.
[76] The method according to any one of [37] to [70] above, wherein a rate of cells in which the reference protein forms an aggregation is a value in a range of 70 to 80%.
[77] The method according to any one of [37] to [70] above, wherein a rate of cells in which the reference protein forms an aggregation is a value in a range of 80 to 90%.
[78] The method according to any one of [37] to [70] above, wherein a rate of cells in which the reference protein forms an aggregation is a value in a range of 90 to 95%.
[79] The method according to any one of [37] to [70] above, wherein a rate of cells in which the reference protein forms an aggregation is a value in a range of 95 to 99%.
[80] The method according to any one of [37] to [70] above, wherein a rate of cells in which the reference protein forms an aggregation is a value in a range of 99 to 99.9%.
[81] The method according to any one of [37] to [70] above, wherein a rate of cells in which the reference protein forms an aggregation is a value in a range of 99.9 to 100%.
[82] The method according to [69] above, wherein a proportion of cells in which the reference protein forms an aggregation is a value more than 30%.
[83] The method according to [69] above, wherein a proportion of cells in which the reference protein forms an aggregation is a value in a range of 30 to 40%.
[84] The method according to [69] above, wherein a rate of cells in which the reference protein forms an aggregation is a value in a range of 40 to 50%.
[85] The method according to [69] above, wherein a rate of cells in which the reference protein forms an aggregation is a value in a range of 50 to 60%.
[86] The method according to [69] above, wherein a rate of cells in which the reference protein forms an aggregation is a value in a range of 60 to 70%.
[87] The method according to [69] above, wherein a rate of cells in which the reference protein forms an aggregation is a value in a range of 70 to 80%.
[88] The method according to [69] above, wherein a rate of cells in which the reference protein forms an aggregation is a value in a range of 80 to 90%.
[89] The method according to [69] above, wherein a rate of cells in which the reference protein forms an aggregation is a value in a range of 90 to 95%.
[91] The method according to [69] above, wherein a rate of cells in which the reference protein forms an aggregation is a value in a range of 99 to 99.9%.
[92] The method according to [69] above, wherein a rate of cells in which the reference protein forms an aggregation is a value in a range of 99.9 to 100%.
[93] The method according to [70] above, wherein a proportion of cells in which the reference protein forms an aggregation is a value more than 30%.
[94] The method according to [70] above, wherein a proportion of cells in which the reference protein forms an aggregation is a value in a range of 30 to 40%.
[95] The method according to [70] above, wherein a rate of cells in which the reference protein forms an aggregation is a value in a range of 40 to 50%.
[96] The method according to [70] above, wherein a rate of cells in which the reference protein forms an aggregation is a value in a range of 50 to 60%.
[97] The method according to [70] above, wherein a rate of cells in which the reference protein forms an aggregation is a value in a range of 60 to 70%.
[98] The method according to [70] above, wherein a rate of cells in which the reference protein forms an aggregation is a value in a range of 70 to 80%.
[99] The method according to [70] above, wherein a rate of cells in which the reference protein forms an aggregation is a value in a range of 80 to 90%.
[100] The method according to [70] above, wherein a rate of cells in which the reference protein forms an aggregation is a value in a range of 90 to 95%.
[101] The method according to [70] above, wherein a rate of cells in which the reference protein forms an aggregation is a value in a range of 95 to 99%.
[102] The method according to [70] above, wherein a rate of cells in which the reference protein forms an aggregation is a value in a range of 99 to 99.9%.
[103] The method according to [70] above, wherein a rate of cells in which the reference protein forms an aggregation is a value in a range of 99.9 to 100%.
[104] The peptide according to any one of [1] to [5] above, wherein a peptide tag is capable of reducing an aggregation property of a scFv having at least an amino acid sequence set forth in SEQ ID NO: 1.
[105] The nucleic acid according to [6] above, wherein a peptide tag is capable of reducing an aggregation property of a scFv having at least an amino acid sequence set forth in SEQ ID NO: 1.
[106] The protein expression vector according to any one of [7] to [9] above, wherein a peptide tag is capable of reducing an aggregation property of a scFv having at least an amino acid sequence set forth in SEQ ID NO: 1.
[107] The protein expression vector according to any one of [7] to [9] above, wherein the protein expression vector is a virus vector.
[108] The protein expression vector according to [107] above, wherein the virus vector is selected from the group consisting of a retrovirus vector, a lentivirus vector, an adenovirus vector, an adeno-associated virus vector, a herpes simplex virus vector, a vaccinia virus vector, a Sendai virus vector, and a vesicular stomatitis virus vector.
[109] The nucleic acid according to [6] above, wherein the nucleic acid is an mRNA.
[110] The nucleic acid according to [109] above, wherein the nucleic acid has a cap structure at the 5' end, and a poly A chain at the 3' UTR.
[111] The nucleic acid according to [109] or [110] above, wherein the nucleic acid contains pseudouridine as U.
[112] A nanoparticle, comprising the nucleic acid according to any one of [109] to [111] above.
[113] The nanoparticle according to [112], wherein the nanoparticle is a lipid nanoparticle.
[114] The method according to any one of [14] to [17] and [30] to [103] above, wherein the reference protein has an amino acid sequence set forth in SEQ ID NO: 1.
[115] The method, the peptide, the fusion protein, the nucleic acid, or the vector according to any one of those described above, wherein the cell is a eukaryotic cell.
[116] The method, the peptide, the fusion protein, the nucleic acid, or the vector according to any one of those described above, wherein the cell is a human cell.
[117] The method, the peptide, the fusion protein, the nucleic acid, or the vector according to any one of those described above, wherein a peptide tag does not prevent free localization of a protein of interest.

A peptide tag of the present disclosure can cause an intracellular stability of a tagged protein. Accordingly, the peptide tag of the present disclosure can be a more highly biocompatible peptide tag.

### Brief Description of Drawings

[Figure 1] Figure 1 illustrates an effect of a peptide tag Tag4-1 on an aggregation property of a single chain Fv (scFv) in a cell.
[Figure 2A] Figure 2A illustrates a scheme for constructing a model of an intracellular accumulation of α-synuclein, that is, an amyloid.
[Figure 2B] Figure 2B illustrates fluorescence microscope images showing influence on intracellular synuclein fibril caused by intracellular expression of scFv-E6-CMA peptide fusion protein having Tag18-1, that is, one of peptide tags of the present disclosure.
[Figure 2C] Figure 2C illustrates an effect of removing synuclein fibril by intracellular expression of scFv-E6-CMA peptide fusion protein having Tag4-8 or Tag18-1, that is, one of peptide tags of the present disclosure.
[Figure 3A] Figure 3A illustrates fluorescence microscope images showing intracellular localization of a scFv-C2 itself having Tag18-1, that is, one of peptide tags of the present disclosure expressed in the cell.
[Figure 3B] Figure 3B illustrates a stabilizing action of the scFv-C2 having Tag18-1, that is, one of peptide tags of the present disclosure.

### Description of Embodiments

In the present invention, the term "subject" is a vertebrate, examples include birds and mammals, and specific examples include mammals such as a mouse, a rat, a hamster, a guinea pig, a horse, a cow, a pig, a goat, sheep, a donkey, a dog, and a cat, and primates such as a monkey, a chimpanzee, a gorilla, an orangutan, a bonobo, and a human, and particularly a human. Herein, the term "subject" is used in the meaning including a human as described above, and when a human is excluded, the term "non-human" is used.

Herein, the term "antibody" means an immunoglobulin, and refers to a protein having a structure in which two heavy chains (H chains) and two light chains (L chains) stabilized through a disulfide bond are associated with each other. The heavy chain contains a heavy chain variable region VH, heavy chain constant regions CH1, CH2, and CH3, and a hinge region positioned between the CH1 and the CH2, and the light chain contains a light chain variable region VL (wherein VL can be Vκ or Vλ), and a light chain constant region CL. Among these regions, a variable region fragment (Fv) consisting of the VH and the VL is a region directly involved in an antigen bond, and imparting variety to the antibody. An antigen binding region consisting of the VL, the CL, the VH, and the CH1 is designated as a Fab region, and a region consisting of the hinge region, the CH2 and the CH3 is designated as a Fc region.

Among the variable regions, a region directly contacting an antigen is particularly largely changed, and is designated as a complementarity-determining region (CDR). A portion except for the CDRs that is comparatively less mutated is designated as a framework region (FR). There are three CDRs in each variable region of the heavy chain and the light chain, and these are designated, successively from the N terminal side, heavy chain CDR1 to CDR3, and light chain CDR1 to CDR3, respectively. Each CDR is incorporated into the framework regions. The heavy chain variable region of the antibody includes, from the N terminal side to the C terminal side, a heavy chain framework region 1, the heavy chain CDR1, a heavy chain framework region 2, the heavy chain CDR2, a heavy chain framework region 3, the heavy chain CDR3, and a heavy chain framework region 4 in the stated order. The light chain variable region of the antibody includes, from the N terminal side to the C terminal side, a light chain framework region 1, the light chain CDR1, a light chain framework region 2, the light chain CDR2, a light chain framework region 3, the light chain CDR3, and a light chain framework region 4 in the stated order. The antibody may be a recombinant protein (recombinant antibody), and can be produced in an animal cell such as a Chinese hamster ovarian cell (CHO cell). The derivation of the antibody is not especially limited, and examples include an antibody of a non-human animal, an antibody of a non-human mammal (such as a mouse antibody, a rat antibody, or a camel antibody), and a human antibody. The antibody may be a chimeric antibody, a humanized antibody, or a fully humanized antibody. The antibody may be a polyclonal antibody or a monoclonal antibody, and is preferably a monoclonal antibody. A "chimeric antibody" refers to an antibody in which a heavy chain variable region and a light chain variable region are respectively linked to a heavy chain constant region and a light chain constant region of different species. A humanized antibody means an antibody in which an amino acid sequence characteristic to a non-human-derived antibody is substituted in the corresponding position of a human antibody, and an example includes an antibody having heavy chain CDR1 to CDR3 and light chain CDR1 to CDR3 of an antibody produced by immunizing a mouse or a rat, and having the other regions including four framework regions (FR) each of the heavy chain and the light chain all derived from a human antibody. Such an antibody is designated as a CDR-grafted antibody in some cases. A "humanized antibody" encompasses a human chimeric antibody in some cases. A "human chimeric antibody" refers to a non-human-derived antibody in which a constant region of the non-human-derived antibody is substituted with a constant region of a human antibody. The antibody can be an isolated antibody, or a purified antibody. The antibody can be, for example, an IgG.

A variable region of an immunoglobulin chain generally has the same entire structure including relatively preserved framework regions (FR) linked through three hypervariable regions (more frequently designated as "complementarity-determining regions" or CDRs). The CDRs obtained from the two chains of each heavy chain/light chain pair are typically arranged parallel by the framework region for forming a structure specifically binding to a specific epitope on a protein of interest (such as PCSK9). Light chain and heavy chain variable regions present in nature all usually have these elements in the following order from the N terminal to the C terminal: FR1, CDR1, FR2, CDR2, FR3, CDR3 and FR4. In order to assign numbers to amino acids positioned in these respective domains, a numbering system has been devised. This numbering system is defined in "Kabat Sequences of Proteins of Immunological Interest (1987 and 1991, NIH, Bethesda, MD)", or "Chothia & Lesk, 1987, J. Mol. Biol. 196: 901-917; Chothia et al., 1989, Nature, 342: 878-883".

Herein, the antibody encompasses an antigen-binding fragment of an antibody. Herein, an antibody not fragmented may be referred to as a full length antibody. A full length antibody can contain the full length of the antibody excluding a signal sequence.

Herein, the term "antigen-binding fragment" means a part of an antibody maintaining a binding property to an antigen. The antigen-binding fragment can contain either or both of a heavy chain variable region and a light chain variable region of the antibody of the present disclosure. The antigen-binding fragment may be chimerized or humanized. Examples of the antigen-binding fragment include Fab, Fab', F(ab')₂, and Fv. The antigen-binding fragment may contain a bonded product or functional equivalent produced by recombination (for example, a part of another antibody in the form of a scFv (single chain Fv), a diabody, a scDb, a tandem scFv, a leucine zipper type, or a sc(Fv)₂ (single chain (Fv)₂)). . Such an antigen-binding fragment of an antibody can be obtained, for example, by treating the antibody with an enzyme, although not especially limited. For example, when an antibody is digested with papain, a Fab can be obtained. Alternatively, when an antibody is digested with pepsin, a F(ab')₂ can be obtained, and when this is further reduced, a Fab' can be obtained. Herein, such an antigen-binding fragment of the antibody can be used. In an scFv, the VL and the VH are linked via an artificial polypeptide linker, and thus, the same antigen specificity as that of the original antibody can be maintained. The VL and the VH can be linked in the order of the VH and the VL, or the VL and the VH from the N terminal side. The linker can have a length of about 10 to 25 amino acids. The linker may contain glycine in a large amount, and may contain an amino acid such as serine or threonine for purpose of increasing water solubility.

Herein, the term "intracellular antibody" (intrabody) refers to an antibody expressed in a cell (for example, in the cytoplasm or in the nucleus). Although an antibody is extracellularly secreted to function, an intracellular antibody is different in that it is designed to be expressed in a cell to function. The intracellular antibody can affect the function of an intracellular protein, and can inhibit the function thereof in the cytoplasm, the nucleus, or the secretory pathway. A cancer gene product can be a target of the intracellular antibody (Biocca, S., Pierandrei-Amaldi, P., and Cattaneo, A. (1993), Biochem Biophys Res Commun, Vol. 197, p. 422 to 427; Biocca, S., Pierandrei-Amaldi, P., Campioni, N., and Cattaneo, A. (1994), Biotechnology (NY), Vol. 12, p. 396 to 399; Cochet, O. et al., (1998), Cancer Res, Vol. 58, p. 1170 to 1176). The intracellular antibody directly binds to a protein for purpose of inhibiting the protein function. The bond may directly inhibit the function of the protein in some cases, and may inhibit the protein from binding to another protein in other cases.

Examples of the intracellular antibody include various antibodies and antigen-binding fragments thereof, and although not especially limited, a scFv, a tandem scFv, a VHH antibody (nanobody), a minibody, and a diabody can be preferably used. A scFv is typically an antibody fragment having a heavy chain variable region and a light chain variable region of an antibody, and the heavy chain variable region and the light chain variable region are linked via a linker. A tandem scFv is typically an antibody fragment having two scFvs having different antigen specificities, and these are linked via a linker. A diabody is typically a dimer of a scFv. Diabodies are roughly divided into bivalent monospecific diabodies and bispecific diabodies. A minibody is typically dimerized two fusion proteins each of a dimerized domain and a scFv via the dimerized domain. A VHH antibody is an antibody fragment containing a heavy chain variable domain of a heavy chain antibody. The VHH antibody is typically a heavy chain variable domain of a heavy chain antibody derived from a camelid (such as a camel, a llama, or an alpaca). Although a general antibody is extracellularly expressed, and hence can be caused to function only extracellularly, the intracellular antibody is superior because it can be caused to exhibit the antibody function in a cell. The intracellular antibody can be used in various applications in a cell such as activation and inactivation of a target protein, and neutralization and block of protein-protein interaction. A scFv tends to exhibit an aggregation property when expressed in a cell. Accordingly, in such a case, it is useful to reduce the aggregation property by obtaining a fusion protein by linking a peptide tag of the present disclosure to the intracellular antibody. When the aggregation property of a protein is reduced, the protein can be caused to exhibit functions inherent to the protein in the cell.

Herein, the term "peptide tag" refers to one that labels a protein of interest, or changes a biochemical property of the protein of interest when fused with the protein of interest. Examples of the peptide tag include various tags such as a FLAG tag, a 3×_{FLAG} tag, a Myc tag, an HA tag, T7, a 6×His tag, a PA tag, an S tag, an E tag, VSV-G, Glu-Glu, Strep-tag II, a HSV tag, a Chitin Binding Domain (CBD) tag, a Calmodulin Binding Peptide (CBP) tag, a V5 tag, a GST tag, a maltose binding protein (MBP) tag, a thioredoxin (Trx) tag, and a mini-AID tag. These can be used for affinity purification of a protein of interest by utilizing affinity for the tag, or for detection of the protein of interest with an antibody to the tag produced. An antibody recognizing a tag is generally designated as a tag antibody, and a tag sequence corresponding to an epitope of the tag antibody is designated as an epitope tag. A tag can have a polypeptide chain generally with a length of several amino acids to several tens amino acids.

Herein, the term "protein of interest" refers to a protein to be expressed in a cell. The protein of interest may be an aggregating protein or a non-aggregating protein. In either case, when the peptide tag of the present disclosure is added thereto, the stability is further increased, and robustness against formation of aggregation can be obtained. Even when added to an aggregating protein, however, the peptide tag of the present disclosure can reduce the aggregation property thereof in a cell, and therefore, the protein of interest can be preferably an aggregating protein. Even when the protein of interest is a secretory protein, aggregation may be formed in a cell before the secretory protein is secreted extracellularly in some cases. The peptide tag of the present disclosure can be advantageously used also for a secretory protein, preferably a secretory protein having an aggregation property.

Herein, the term " aggregating protein" refers to a protein that forms aggregation (particularly, an insoluble aggregation) in a cell. Herein, the term "aggregation property" means a property of forming aggregation, and the term "non-aggregation property" means a property of not forming aggregation. Attenuation of the aggregation property can be promotion of the non-aggregation property, and promotion of the aggregation property can be attenuation of the non-aggregation property. Herein, the term "non-aggregation property" is used interchangeably with the term "stability". Aggregation can be observed, for example, as a bright point under a microscope by immunocytochemistry (IC). An aggregation rate can be calculated, for example, as a proportion of cells exhibiting aggregation in cells forcedly expressing a protein. Reduction of the aggregation rate thus calculated means increase of cells that forcedly express a protein and are not affected by the aggregation, and therefore can be an index of physiological favorability. Reduction of the aggregation property (for example, reduction of the aggregation rate) and increase of solubility are different indexes. The increase of solubility means increase of a concentration in an aqueous solution of available protein, and does not directly lead to the number of aggregations, or a proportion of cells having the aggregations. Accordingly, the increase of solubility does not always mean the reduction of the aggregation property (for example, the reduction of the aggregation rate).

Herein, an amino acid sequence is described by one letter amino acid code. Specifically, A denotes alanine, R denotes arginine, N denotes asparagine, D denotes aspartic acid, C denotes cysteine, Q denotes glutamine, E denotes glutamic acid, G denotes glycine, H denotes histidine, I denotes isoleucine, L denotes leucine, K denotes lysine, M denotes methionine, F denotes phenylalanine, P denotes proline, S denotes serine, T denotes threonine, W denotes tryptophan, Y denotes tyrosine, and V denotes valine. Amino acids are usually 20 types of L-amino acids mentioned above.

Herein, the term "regulatory sequence" refers to a sequence having activity of driving a gene operably linked thereto to transcribe RNA from the gene. The regulatory sequence is, for example, a promoter. Examples of the promoter include a class I promoter (usable for transcription of an rRNA precursor), a class II promoter (containing a core promoter and an upstream promoter element, and usable for transcription of an mRNA), and a class III promoter (further roughly divided into type I, type II, and type III).

The present invention provides a peptide tag that reduces aggregation tendency of an aggregating protein. The present invention provides a protein expression vector operably linked to a regulatory sequence, and containing a gene encoding the peptide tag. The present invention provides a protein of interest fused with the peptide tag. The present invention provides a protein expression vector operably linked to a regulatory sequence, and containing a gene encoding a protein of interest fused with the peptide tag. The protein of interest can be an intracellular protein in one embodiment. The protein of interest can be an intracellular antibody in one embodiment. The protein of interest can be an scFv in one embodiment.

Hereinafter, the peptide tag of the present disclosure that reduces aggregation tendency of an aggregating protein will be described in detail. The peptide tag of the present disclosure can reduce aggregation tendency of a protein of interest in a eukaryotic cell, particularly, in a human cell. In examination of pharmaceutical application and the like, it can be useful to reduce the aggregation tendency of a protein of interest in a human cell.

The length of the peptide tag of the present disclosure is not especially limited, and can be, for example, 600 amino acids or less, 500 amino acids or less, 400 amino acids or less, 300 amino acids or less, or 200 amino acids or less, and for example, 5 amino acids to 100 amino acids, such as 10 amino acids to 90 amino acids, 20 amino acids to 80 amino acids, 30 amino acids to 70 amino acids, 40 amino acids to 60 amino acids, 10 amino acids to 50 amino acids, 10 amino acids to 40 amino acids, or 10 amino acids to 30 amino acids. In this embodiment, the lower limit of the length of the peptide tag of the present disclosure can be 5 amino acids or more, 10 amino acids or more, 15 amino acids or more, 20 amino acids or more, 30 amino acids or more, 40 amino acids or more, 50 amino acids or more, 60 amino acids or more, 70 amino acids or more, or 80 amino acids or more, and/or the upper limit can be 100 amino acids or less, 90 amino acids or less, 80 amino acids or less, 70 amino acids or less, 60 amino acids or less, 50 amino acids or less, 40 amino acids or less, 30 amino acids or less, or 20 amino acids or less.

(a) The peptide tag of the present disclosure can contain acidic amino acids (amino acids belonging to Element 1) in the following ratio.

In the peptide tag of the present disclosure, 45% or more of amino acids contained in the amino acid sequence thereof can be acidic amino acids.

In the peptide tag of the present disclosure, less than 45% of amino acids contained in the amino acid sequence thereof can be acidic amino acids. In a preferable embodiment, in the peptide tag of the present disclosure, 5% or more and less than 45% of amino acids contained in the amino acid sequence thereof can be acidic amino acids, more preferably, 10% or more and less than 45% of amino acids contained in the amino acid sequence thereof can be acidic amino acids, further preferably, 20% or more and less than 45% of amino acids contained in the amino acid sequence thereof can be acidic amino acids, further preferably, 30% or more and less than 45% of amino acids contained in the amino acid sequence thereof can be acidic amino acids, still further preferably, 35% or more and less than 45% of amino acids contained in the amino acid sequence thereof can be acidic amino acids, and particularly preferably, 40% or more and less than 45% of amino acids contained in the amino acid sequence thereof can be acidic amino acids. The acidic amino acids are D or E. For example, an acidic amino acid content in the peptide tag of the present disclosure can be 44% or less, 43.5% or less, 43% or less, 42.5% or less, 42% or less, 41.5% or less, 410 or less, 40% or less, 35% or less, 30% or less, 25% or less, or 20% or less. Thus, in one embodiment, a risk of occurrence of unexpected interaction with an intracellular molecule or the like having a positive charge based on a high acidic amino acid ratio in the peptide tag can be reduced.

(b) The peptide tag of the present disclosure can contain basic amino acids (amino acids belonging to Element 2) in the following ratio.

The peptide tag of the present disclosure can contain basic amino acids in a rate of preferably 25% or less or 20% or less, and more preferably can contain basic amino acids in a rate of 15% or less of amino acids, can contain basic amino acids further preferably in a rate of 10% or less, can contain basic amino acids further preferably in a rate of 5% or less, and can contain basic amino acids particularly preferably in a rate less than 3%, less than 2%, or less than 1%. In a most preferable embodiment, the peptide tag of the present disclosure does not contain a basic amino acid in the amino acid sequence thereof. The basic amino acids are K, R, or H.

(c) The peptide tag of the present disclosure can contain amino acids belonging to Element 3 in the following ratio.

The amino acids belonging to the Element 3 can be F, P, Y, G, S, Q, N, and A.

In the peptide tag of the present disclosure, 10% or more, preferably 20% or more, more preferably 30% or more, or 40% or more of amino acids contained in the amino acid sequence thereof can be preferably the amino acids of the Element 3. In the peptide tag of the present disclosure, 50% or more, 60% or more, or 70% or more of amino acids contained in the amino acid sequence thereof can be amino acids of the Element 3. In the peptide tag of the present disclosure, preferably 80% or less, more preferably 70% or less, and further preferably 60% or less of amino acids contained in the amino acid sequence thereof can be the amino acids of the Element 3. 50% or less, 40% or less, 30% or less, or 20% or less of amino acids contained in the amino acid sequence thereof can be the amino acids of the Element 3. In a preferable embodiment, in the peptide tag of the present disclosure, 20% or more and 80% or less, 30% or more and 70% or less, 30% or more and 60% or less, 30% or more and 50% or less, 30% or more and 40% or less, 40% or more and 70% or less, 40% or more and 60% or less, 40% or more and 50% or less, 50% or more and 70% or less, 50% or more and 60% or less, 60% or more and 80% or less, or 60% or more and 70% or less of amino acids contained in the amino acid sequence thereof can be the amino acids of the Element 3.

In a particularly preferable embodiment, in the peptide tag of the present disclosure, 5% or more, 10% or more, 15% or more, or 20% or more (preferably 210 or more, 25% or more, or 30% or more) of amino acids contained in the amino acid sequence thereof are either N or P. In the peptide tag of the present disclosure, for example, 90% or less, 80% or less, 70% or less, 60% or less, 50% or less, 45% or less, 40% or less, 35% or less, 30% or less, or 25% or less of amino acids contained in the amino acid sequence thereof can be either N or P. In one embodiment, in the peptide tag of the present disclosure, 10% or more and 20% or less of amino acids contained in the amino acid sequence thereof can be either N or P. In one embodiment, in the peptide tag of the present disclosure, 55% or more and 90% or less of amino acids contained in the amino acid sequence thereof can be either N or P. In one embodiment, in the peptide tag of the present disclosure, more than 10% and 20% or less of amino acids contained in the amino acid sequence thereof can be either N or P. In one embodiment, in the peptide tag of the present disclosure, more than 20% and 30% or less of amino acids contained in the amino acid sequence thereof can be either N or P. In one embodiment, in the peptide tag of the present disclosure, more than 30% and 40% or less of amino acids contained in the amino acid sequence thereof can be either N or P. In one embodiment, in the peptide tag of the present disclosure, more than 40% and 50% or less of amino acids contained in the amino acid sequence thereof can be either N or P. In one embodiment, in the peptide tag of the present disclosure, more than 50% and 60% or less of amino acids contained in the amino acid sequence thereof can be either N or P.

For example, in the peptide tag of the present disclosure, 5% or more, 10% or more, 15% or more, or 20% or more (preferably 210 or more, 25% or more, or 30% or more) of amino acids contained in the amino acid sequence thereof are N. In the peptide tag of the present disclosure, for example, 90% or less, 80% or less, 70% or less, 60% or less, 50% or less, 45% or less, 40% or less, 35% or less, 30% or less, or 25% or less of amino acids contained in the amino acid sequence thereof can be N. In one embodiment, in the peptide tag of the present disclosure, 10% or more and 20% or less of amino acids contained in the amino acid sequence thereof can be N. In one embodiment, in the peptide tag of the present disclosure, 55% or more and 90% or less of amino acids contained in the amino acid sequence thereof can be N. In one embodiment, in the peptide tag of the present disclosure, more than 10% and 20% or less of amino acids contained in the amino acid sequence thereof can be N. In one embodiment, in the peptide tag of the present disclosure, more than 20% and 30% or less of amino acids contained in the amino acid sequence thereof can be N. In one embodiment, in the peptide tag of the present disclosure, more than 30% and 40% or less of amino acids contained in the amino acid sequence thereof can be N. In one embodiment, in the peptide tag of the present disclosure, more than 40% and 50% or less of amino acids contained in the amino acid sequence thereof can be N. In one embodiment, in the peptide tag of the present disclosure, more than 50% and 60% or less of amino acids contained in the amino acid sequence thereof can be N.

For example, in the peptide tag of the present disclosure, 5% or more, 6% or more, 7% or more, 8% or more, 9% or more, 100 or more, 15% or more, or 20% or more (preferably 210 or more, 25% or more, or 30% or more) of amino acids contained in the amino acid sequence thereof are P. In the peptide tag of the present disclosure, for example, 90% or less, 80% or less, 70% or less, 60% or less, 50% or less, 45% or less, 40% or less, 35% or less, 30% or less, or 25% or less of amino acids contained in the amino acid sequence thereof can be P. In one embodiment, in the peptide tag of the present disclosure, 55% or more and 90% or less of amino acids contained in the amino acid sequence thereof can be P. In one embodiment, in the peptide tag of the present disclosure, 10% or more and 20% or less of amino acids contained in the amino acid sequence thereof can be P. In one embodiment, in the peptide tag of the present disclosure, more than 10% and 20% or less of amino acids contained in the amino acid sequence thereof can be P. In one embodiment, in the peptide tag of the present disclosure, more than 20% and 30% or less of amino acids contained in the amino acid sequence thereof can be P. In one embodiment, in the peptide tag of the present disclosure, more than 30% and 40% or less of amino acids contained in the amino acid sequence thereof can be P. In one embodiment, in the peptide tag of the present disclosure, more than 40% and 50% or less of amino acids contained in the amino acid sequence thereof can be P. In one embodiment, in the peptide tag of the present disclosure, more than 50% and 60% or less of amino acids contained in the amino acid sequence thereof can be P.

In a particularly preferable embodiment, in the peptide tag of the present disclosure, 5% or less, 10% or less, 15% or less, or 20% or less of amino acids contained in the amino acid sequence thereof are F or Y. In a particularly preferable embodiment, in the peptide tag of the present disclosure, 5% or less, 10% or less, 15% or less, or 20% or less of amino acids contained in the amino acid sequence thereof are F and/or Y. In a particularly preferable embodiment, in the peptide tag of the present disclosure, 5% or less, 10% or less, 15% or less, or 20% or less of amino acids contained in the amino acid sequence thereof are F and Y. In a particularly preferable embodiment, 5% or more, 10% or more, 15% or more, or 20% or more of amino acids contained in the amino acid sequence thereof are either N or P, and 5% or less, 10% or less, 15% or less, or 20% or less thereof are F and/or Y.

(d) The peptide tag of the present disclosure can contain amino acids belonging to Element 4 in the following ratio.

The amino acids belonging to the Element 4 can be amino acids that are none of an acidic amino acid, a basic amino acid, and the amino acids of the Element 3. The amino acids of the Element 4 can be, for example, M, T, W, C, I, V, and L.

In the peptide tag of the present disclosure, 80% or less, 70% or less, 60% or less, 50% or less, 40% or less, 30% or less, 20% or less, 15% or less, 10% or less, or 5% or less of amino acids contained in the amino acid sequence thereof can be preferably the amino acids of the Element 4. In a preferable embodiment, the peptide tag of the present disclosure does not contain the amino acids of the Element 4.

(e) In the peptide tag of the present disclosure, 40% or less, 30% or less, 20% or less, 15% or less, 10% or less, or 5% or less of amino acids contained in the amino acid sequence thereof can be preferably G. In one embodiment, the peptide tag of the present disclosure does not contain G.

(f) In the peptide tag of the present disclosure, 40% or less, 30% or less, 20% or less, 15% or less, 10% or less, or 5% or less of amino acids contained in the amino acid sequence thereof can be preferably A. In one embodiment, the peptide tag of the present disclosure does not contain A.

(g) In the peptide tag of the present disclosure, 40% or less, 30% or less, 20% or less, 15% or less, 10% or less, or 5% or less of amino acids contained in the amino acid sequence thereof can be preferably G, and 40% or less, 30% or less, 20% or less, 15% or less, 10% or less, or 5% or less of amino acids contained in the amino acid sequence thereof can be A.

(h) The peptide tag of the present disclosure can preferably contain S. The peptide tag of the present disclosure preferably does not contain S. The peptide tag of the present disclosure can contain S in a rate of 10% or more, 20% or more, 30% or more, 40% or more, or 50% or more of amino acids contained in the amino acid sequence thereof. The peptide tag of the present disclosure can contain S in a rate of 60% or less, 50% or less, 40% or less, 30% or less, 20% or less, 15% or less, 10% or less, or 5% or less of amino acids contained in the amino acid sequence thereof.

(A) In the peptide tag of the present disclosure, less than 45% of amino acids contained in the amino acid sequence thereof can be acidic amino acids, and 20% or more and 80% or less (preferably 30% or more and 70% or less) of the amino acids contained in the amino acid sequence thereof can be the amino acids of the Element 3.

(B) In the peptide tag of the present disclosure, less than 45% of amino acids contained in the amino acid sequence thereof can be acidic amino acids, 20% or more and 80% or less (preferably 30% or more and 70% or less) of the amino acids contained in the amino acid sequence thereof can be the amino acids of the Element 3, and 10% or less of the amino acids contained in the amino acid sequence thereof can be A.

(C) In the peptide tag of the present disclosure, less than 45% of amino acids contained in the amino acid sequence thereof can be acidic amino acids, 20% or more and 80% or less (preferably 30% or more and 70% or less) of the amino acids contained in the amino acid sequence thereof can be the amino acids of the Element 3, and 10% or less of the amino acids contained in the amino acid sequence thereof can be G.

(D) In the peptide tag of the present disclosure, less than 45% of amino acids contained in the amino acid sequence thereof can be acidic amino acids, 20% or more and 80% or less (preferably 30% or more and 70% or less) of the amino acids contained in the amino acid sequence thereof can be the amino acids of the Element 3, 10% or less of the amino acids contained in the amino acid sequence thereof can be A, and 10% or less of the amino acids contained in the amino acid sequence thereof can be G.

(E) In the peptide tag of the present disclosure, less than 45% of amino acids contained in the amino acid sequence thereof can be acidic amino acids, 20% or more and 80% or less (preferably 30% or more and 70% or less) of the amino acids contained in the amino acid sequence thereof can be the amino acids of the Element 3, and 30% or less of the amino acids contained in the amino acid sequence thereof can be the amino acids of the Element 4.

(F) In the peptide tag of the present disclosure, less than 45% of amino acids contained in the amino acid sequence thereof can be acidic amino acids, 20% or more and 80% or less (preferably 30% or more and 70% or less) of the amino acids contained in the amino acid sequence thereof can be the amino acids of the Element 3, 30% or less of the amino acids contained in the amino acid sequence thereof can be the amino acids of the Element 4, and 10% or less of the amino acids contained in the amino acid sequence thereof can be A.

(G) In the peptide tag of the present disclosure, less than 45% of amino acids contained in the amino acid sequence thereof can be acidic amino acids, 20% or more and 80% or less (preferably 30% or more and 70% or less) of the amino acids contained in the amino acid sequence thereof can be the amino acids of the Element 3, 30% or less of the amino acids contained in the amino acid sequence thereof can be the amino acids of the Element 4, and 10% or less of the amino acids contained in the amino acid sequence thereof can be G.

(H) In the peptide tag of the present disclosure, less than 45% of amino acids contained in the amino acid sequence thereof can be acidic amino acids, 20% or more and 80% or less (preferably 30% or more and 70% or less) of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be A, and 10% or less of the amino acids contained in the amino acid sequence thereof can be G.

(I) In the peptide tag of the present disclosure, less than 45% of amino acids contained in the amino acid sequence thereof can be acidic amino acids, and 30% or more and 70% or less of the amino acids contained in the amino acid sequence thereof can be the amino acids of the Element 3.

(J) In the peptide tag of the present disclosure, less than 45% of amino acids contained in the amino acid sequence thereof can be acidic amino acids, 30% or more and 70% or less of the amino acids contained in the amino acid sequence thereof can be the amino acids of the Element 3, and 10% or less of the amino acids contained in the amino acid sequence thereof can be A.

(K) In the peptide tag of the present disclosure, less than 45% of amino acids contained in the amino acid sequence thereof can be acidic amino acids, 30% or more and 70% or less of the amino acids contained in the amino acid sequence thereof can be the amino acids of the Element 3, and 10% or less of the amino acids contained in the amino acid sequence thereof can be G.

(L) In the peptide tag of the present disclosure, less than 45% of amino acids contained in the amino acid sequence thereof can be acidic amino acids, 30% or more and 70% or less of the amino acids contained in the amino acid sequence thereof can be the amino acids of the Element 3, 10% or less of the amino acids contained in the amino acid sequence thereof can be A, and 10% or less of the amino acids contained in the amino acid sequence thereof can be G.

(M) In the peptide tag of the present disclosure, less than 45% of amino acids contained in the amino acid sequence thereof can be acidic amino acids, 30% or more and 70% or less of the amino acids contained in the amino acid sequence thereof can be the amino acids of the Element 3, and 30% or less of the amino acids contained in the amino acid sequence thereof can be the amino acids of the Element 4.

(N) In the peptide tag of the present disclosure, less than 45% of amino acids contained in the amino acid sequence thereof can be acidic amino acids, 30% or more and 70% or less of the amino acids contained in the amino acid sequence thereof can be the amino acids of the Element 3, 30% or less of the amino acids contained in the amino acid sequence thereof can be the amino acids of the Element 4, and 10% or less of the amino acids contained in the amino acid sequence thereof can be A.

(O) In the peptide tag of the present disclosure, less than 45% of amino acids contained in the amino acid sequence thereof can be acidic amino acids, 30% or more and 70% or less of the amino acids contained in the amino acid sequence thereof can be the amino acids of the Element 3, 30% or less of the amino acids contained in the amino acid sequence thereof can be the amino acids of the Element 4, and 10% or less of the amino acids contained in the amino acid sequence thereof can be G.

(P) In the peptide tag of the present disclosure, less than 45% of amino acids contained in the amino acid sequence thereof can be acidic amino acids, 30% or more and 70% or less of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be A, and 10% or less of the amino acids contained in the amino acid sequence thereof can be G.

(Q) In the above-described peptide tag, preferably 20% or more and less than 45% of amino acids contained in the amino acid sequence thereof can be acidic amino acids, more preferably 30% or more and less than 45% of the amino acids contained in the amino acid sequence thereof can be acidic amino acids, further preferably 35% or more and less than 45% of the amino acids contained in the amino acid sequence thereof can be acidic amino acids, and particularly preferably 40% or more and less than 45% of the amino acids contained in the amino acid sequence thereof can be acidic amino acids.

(R) In the above-described peptide tag, 10% or less of the amino acids contained in the amino acid sequence thereof are preferably basic amino acids. The peptide tag can contain preferably 5% or less of basic amino acids, and particularly preferably less than 3%, less than 2%, or less than 1% of basic amino acids. Alternatively, the peptide tag does not contain a basic amino acid in a preferable embodiment.

(S) In the above-described peptide tag, 40% or more and 60% or less of the amino acids contained in the amino acid sequence thereof are preferably the amino acids of the Element 3.

(T) In the above-described peptide tag, 20% or less, 15% or less, 10% or less, or 5% or less of the amino acids contained in the amino acid sequence thereof are preferably the amino acids of the Element 4. In a preferable embodiment, the above-described peptide tag does not contain the amino acids of the Element 4.

(U) In the peptide tag of the present disclosure, less than 45% of amino acids contained in the amino acid sequence thereof can be acidic amino acids, 10% or less of the amino acids contained in the amino acid sequence thereof can be basic amino acids, 20% or more and 80% or less (preferably 30% or more and 70% or less) of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, preferably 5% or more and less than 45% of the amino acids contained in the amino acid sequence thereof can be acidic amino acids, more preferably 10% or more and less than 45% of the amino acids contained in the amino acid sequence thereof can be acidic amino acids, further preferably 20% or more and less than 45% of the amino acids contained in the amino acid sequence thereof can be acidic amino acids, further preferably 30% or more and less than 45% of the amino acids contained in the amino acid sequence thereof can be acidic amino acids, still further preferably 35% or more and less than 45% of the amino acids contained in the amino acid sequence thereof can be acidic amino acids, and particularly preferably 40% or more and less than 45% of the amino acids contained in the amino acid sequence thereof can be acidic amino acids.

(V) In the peptide tag of the present disclosure, less than 45% of amino acids contained in the amino acid sequence thereof can be acidic amino acids, 5% or less of the amino acids contained in the amino acid sequence thereof can be basic amino acids, 20% or more and 80% or less (preferably 30% or more and 70% or less) of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, in the peptide tag of the present disclosure, it is preferable that less than 3%, less than 2%, or less than 1% of the amino acids contained in the amino acid sequence thereof can be basic amino acids, or that it does not contain a basic amino acid.

(W) In the peptide tag of the present disclosure, less than 45% of amino acids contained in the amino acid sequence thereof can be acidic amino acids, 10% or less of the amino acids contained in the amino acid sequence thereof can be basic amino acids, 30% or more and 70% or less of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, in the peptide tag of the present disclosure, 40% or more and 60% or less of the amino acids contained in the amino acid sequence thereof can be preferably the amino acids of the Element 3.

(X) In the peptide tag of the present disclosure, less than 45% of amino acids contained in the amino acid sequence thereof can be acidic amino acids, 10% or less of the amino acids contained in the amino acid sequence thereof can be basic amino acids, 20% or more and 80% or less (preferably 30% or more and 70% or less) of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, in the peptide tag of the present disclosure, 20% or less, 15% or less, 10% or less, or 5% or less of the amino acids contained in the amino acid sequence thereof can be preferably the amino acids of the Element 4. In a preferable embodiment, the peptide tag of the present disclosure does not contain the amino acids of the Element 4.

(Y) In the peptide tag of the present disclosure, 20% or more and less than 45% of amino acids contained in the amino acid sequence thereof can be acidic amino acids, 10% or less of the amino acids contained in the amino acid sequence thereof can be basic amino acids, 20% or more and 80% or less (preferably 30% or more and 70% or less) of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, preferably 30% or more and less than 45% of the amino acids contained in the amino acid sequence thereof are acidic amino acids, more preferably 35% or more and less than 45% of the amino acids of the amino acid sequence are acidic amino acids, and further preferably 40% or more and less than 45% of the amino acids contained in the amino acid sequence thereof are acidic amino acids.

(Z) In the peptide tag of the present disclosure, 20% or more and less than 45% of amino acids contained in the amino acid sequence thereof can be acidic amino acids, 10% or less of the amino acids contained in the amino acid sequence thereof can be basic amino acids, 30% or more and 70% or less of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, preferably 40% or more and 60% or less of the amino acids contained in the amino acid sequence thereof are the amino acids of the Element 3.

(AA) In the peptide tag of the present disclosure, 20% or more and less than 45% of amino acids contained in the amino acid sequence thereof can be acidic amino acids, 10% or less of the amino acids contained in the amino acid sequence thereof can be basic amino acids, 20% or more and 80% or less (preferably 30% or more and 70% or less) of the amino acids contained in the amino acid sequence thereof can be the amino acids of the Element 3, 20% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, it is preferable that 15% or less of the amino acids contained in the amino acid sequence thereof are the amino acids of the Element 4, that 10% or less of the amino acids contained in the amino acid sequence thereof are the amino acids of the Element 4, and that 5% or less of the amino acids contained in the amino acid sequence thereof are the amino acids of the Element 4.

(AB) In the peptide tag of the present disclosure, 30% or more and less than 45% of amino acids contained in the amino acid sequence thereof can be acidic amino acids, 10% or less of the amino acids contained in the amino acid sequence thereof can be basic amino acids, 30% or more and 70% or less of the amino acids contained in the amino acid sequence thereof can be the amino acids of the Element 3, 30% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, preferably 40% or more and 60% or less of the amino acids contained in the amino acid sequence thereof are the amino acids of the Element 3. In this embodiment, more preferably 35% or more and less than 45% of the amino acids contained in the amino acid sequence thereof can be acidic amino acids, and further preferably 40% or more and less than 45% of the amino acids contained in the amino acid sequence thereof can be acidic amino acids. In this embodiment, preferably 40% or more and 60% or less of the amino acids contained in the amino acid sequence thereof are the amino acids of the Element 3, and 35% or more and less than 45% of the amino acids contained in the amino acid sequence thereof are acidic amino acids, and further preferably 40% or more and 60% or less of the amino acids contained in the amino acid sequence thereof can be the amino acids of the Element 3, and 40% or more and less than 45% of the amino acids contained in the amino acid sequence thereof can be acidic amino acids.

(AC) In the peptide tag of the present disclosure, 30% or more and less than 45% of amino acids contained in the amino acid sequence thereof can be acidic amino acids, 10% or less of the amino acids contained in the amino acid sequence thereof can be basic amino acids, 30% or more and 70% or less of the amino acids contained in the amino acid sequence thereof can be the amino acids of the Element 3, 20% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A. In this embodiment, preferably 150 or less, 10% or less, or 5% or less of the amino acids contained in the amino acid sequence thereof can be G. In one embodiment, the peptide tag of the present disclosure does not contain G.

(AD) In the peptide tag of the present disclosure, 35% or more and less than 45% of amino acids contained in the amino acid sequence thereof can be acidic amino acids, 10% or less of the amino acids contained in the amino acid sequence thereof can be basic amino acids, 30% or more and 70% or less of the amino acids contained in the amino acid sequence thereof can be the amino acids of the Element 3, 10% or less of the amino acids contained in the amino acid sequence thereof can be the amino acids of the Element 4, 10% or less of the amino acids contained in the amino acid sequence thereof can be G, and 10% or less of the amino acids contained in the amino acid sequence thereof can be A.

(AE) The peptide tag of the present disclosure can have, for example, an amino acid sequence set forth in any of SEQ ID NOs: 2 to 11. The peptide tag of the present disclosure can have preferably an amino acid sequence of SEQ ID NO: 5.

(AF) The peptide tag of the present disclosure can have, for example, any one of amino acid sequences shown in Tables 1 to 11, Table 12-1, Table 12-2, Table 13-1, Table 13-2, Table 14-1, and Table 14-2.

(AG) The peptide tag of the present disclosure may have one or more selected from the group consisting of addition and insertion of one or more amino acids selected from the group consisting of N and P, and substitution with the amino acids (for example, substitution of one to about 30% of amino acids, such as substitution with 1 to 20, 10, or several amino acids) in any amino acids of any amino acid sequences of (AE) and (AF) described above (for example, the amino acids of the Element 1, the Element 2, the Element 3, or the Element 4, or for example, the amino acids of the Element 2, the Element 3, or the Element 4). The peptide tag of the present disclosure may have addition and insertion of S in any amino acids of any amino acid sequences of (AF) and (AG) described above (for example, the amino acids of the Element 1, the Element 2, the Element 3, or the Element 4, or for example, amino acids of the Element 2, the Element 3, or the Element 4), and substitution of S with the amino acids (for example, substitution of one to about 30% of amino acids, such as substitution with 1 to 20, 10, or several amino acids). The peptide tag of the present disclosure may have deletion of an arbitrary amino acid of any one of the amino acid sequences of (AF) and (AG) described above (for example, the amino acids of the Element 1, the Element 2, the Element 3, or the Element 4, for example, the amino acids of the Element 2, the Element 3 (particularly, F and/or Y), and the Element 4 (A or G).

(AH) In the peptide tag of the present disclosure, preferably 5% or more, 6% or more, 7% or more, 8% or more, 9% or more, 10% or more, 11% or more, 12% or more, 13% or more, 14% or more, 15% or more, 16% or more, 170 or more, 18% or more, 19% or more, or 20% or more of amino acids contained in the amino acid sequence thereof can be either N or P, or N and P. In the peptide tag of the present disclosure, preferably 5% or more, 6% or more, 7% or more, 8% or more, 9% or more, 10% or more, 11% or more, 12% or more, 13% or more, 14% or more, 15% or more, 16% or more, 170 or more, 18% or more, 19% or more, 20% or more, 210 or more, 25% or more, or 30% or more of the amino acids contained in the amino acid sequence thereof can be P. In the peptide tag of the present disclosure, preferably 5% or more, 6% or more, 7% or more, 8% or more, 9% or more, 10% or more, 11% or more, 12% or more, 13% or more, 14% or more, 15% or more, 16% or more, 170 or more, 18% or more, 19% or more, 20% or more, 210 or more, 25% or more, or 30% or more of the amino acids contained in the amino acid sequence thereof can be N.

(AI) In the peptide tag of the present disclosure, 45% or more of amino acids contained in the amino acid sequence thereof can be acidic amino acids, and 5% or more, 6% or more, 7% or more, 8% or more, 9% or more, 10% or more, 11% or more, 12% or more, 13% or more, 14% or more, 15% or more, 16% or more, 170 or more, 18% or more, 19% or more, 20% or more, 210 or more, 25% or more, or 30% or more thereof can be N or P. In this embodiment, less than 10% (preferably less than 5%, and more preferably 0%) of the amino acids contained in the amino acid sequence thereof can be G, less than 10% (preferably less than 5%, and more preferably 0%) thereof can be A, and/or less than 10% (preferably less than 5%, and more preferably 0%) thereof can be F and Y.

(AJ) In the peptide tag of the present disclosure,
(a) 5% or more and less than 45% of amino acids contained in the amino acid sequence can be acidic amino acids,
(b) 30% or more and less than 70% of the amino acids contained in the amino acid sequence can be amino acids selected from the group consisting of F, P, Y, G, S, Q, N, and A, and 60% or less of the amino acids contained in the amino acid sequence can be S,
(c) 10% or less of the amino acids contained in the amino acid sequence can be amino acids selected from the group consisting of M, T, W, C, I, V, and L, and
(d) each of A and G can constitute less than 10% of the amino acids contained in the amino acid sequence.
In this embodiment, the amino acid sequence can have a length of 10 to 200 amino acids (such as 10 to 90 amino acids).

(AK) In the peptide tag of the present disclosure,
(a) 5% or more and less than 45% of amino acids contained in the amino acid sequence can be acidic amino acids,
(b) 30% or more and less than 70% of the amino acids contained in the amino acid sequence can be amino acids selected from the group consisting of F, P, Y, G, S, Q, N, and A, 60% or less of the amino acids contained in the amino acid sequence can be S, and 10% or more of the amino acids contained in the amino acid sequence can be N or P,
(c) 10% or less of the amino acids contained in the amino acid sequence can be amino acids selected from the group consisting of M, T, W, C, I, V, and L, and
(d) each of A and G can constitute less than 10% of the amino acids contained in the amino acid sequence.
In this embodiment, the amino acid sequence can have a length of 10 to 200 amino acids (such as 10 to 90 amino acids).

(AJ) In the peptide tag of the present disclosure,
(a) 5% or more and less than 45% of amino acids contained in the amino acid sequence can be acidic amino acids,
(b) 30% or more and less than 70% of the amino acids contained in the amino acid sequence can be amino acids selected from the group consisting of F, P, Y, G, S, Q, N, and A, 60% or less of the amino acids contained in the amino acid sequence can be S, and 10% or less of the amino acids contained in the amino acid sequence can be F and/or Y,
(c) 10% or less of the amino acids contained in the amino acid sequence can be amino acids selected from the group consisting of M, T, W, C, I, V, and L, and
(d) each of A and G can constitute less than 10% of the amino acids contained in the amino acid sequence.
In this embodiment, the amino acid sequence can have a length of 10 to 200 amino acids (such as 10 to 90 amino acids).

(AM) In the peptide tag of the present disclosure,
(a) 5% or more and less than 45% of amino acids contained in the amino acid sequence can be acidic amino acids,
(b) 30% or more and less than 70% of the amino acids contained in the amino acid sequence can be amino acids selected from the group consisting of F, P, Y, G, S, Q, N, and A, 60% or less of the amino acids contained in the amino acid sequence can be S, 10% or more of the amino acids contained in the amino acid sequence can be N or P, and 10% or less of the amino acids contained in the amino acid sequence can be F and/or Y,
(c) 10% or less of the amino acids contained in the amino acid sequence can be amino acids selected from the group consisting of M, T, W, C, I, V, and L, and
(d) each of A and G can constitute less than 10% of the amino acids contained in the amino acid sequence.
In this embodiment, the amino acid sequence can have a length of 10 to 200 amino acids (such as 10 to 90 amino acids).

(AN) In the peptide tag of the present disclosure,
(a) 5% or more and less than 45% of amino acids contained in the amino acid sequence can be acidic amino acids,
(b) 30% or more and less than 65% of the amino acids contained in the amino acid sequence can be amino acids selected from the group consisting of F, P, Y, G, S, Q, N, and A, 60% or less of the amino acids contained in the amino acid sequence can be S, and 10% or less of the amino acids contained in the amino acid sequence can be F and/or Y,
(c) 10% or less of the amino acids contained in the amino acid sequence can be amino acids selected from the group consisting of M, T, W, C, I, V, and L, and
(d) each of A and G can constitute less than 10% of the amino acids contained in the amino acid sequence.
In this embodiment, the amino acid sequence can have a length of 10 to 200 amino acids (such as 10 to 90 amino acids).

(AN) In the peptide tag of the present disclosure,
(a) 5% or more and less than 45% of amino acids contained in the amino acid sequence can be acidic amino acids,
(b) 30% or more and less than 65% of the amino acids contained in the amino acid sequence can be amino acids selected from the group consisting of F, P, Y, G, S, Q, N, and A, 60% or less of the amino acids contained in the amino acid sequence can be S, 10% or more of the amino acids contained in the amino acid sequence can be N or P,
(c) 10% or less of the amino acids contained in the amino acid sequence can be amino acids selected from the group consisting of M, T, W, C, I, V, and L, and
(d) each of A and G can constitute less than 10% of the amino acids contained in the amino acid sequence.

In this embodiment, the amino acid sequence can have a length of 10 to 200 amino acids (such as 10 to 90 amino acids).

(AO) In the peptide tag of the present disclosure,
(a) 5% or more and less than 45% of amino acids contained in the amino acid sequence can be acidic amino acids,
(b) 30% or more and less than 65% of the amino acids contained in the amino acid sequence can be amino acids selected from the group consisting of F, P, Y, G, S, Q, N, and A, 60% or less of the amino acids contained in the amino acid sequence can be S, 10% or more of the amino acids contained in the amino acid sequence can be N or P, and 10% or less of the amino acids contained in the amino acid sequence can be F and/or Y,
(c) 10% or less of the amino acids contained in the amino acid sequence can be amino acids selected from the group consisting of M, T, W, C, I, V, and L, and
(d) each of A and G can constitute less than 10% of the amino acids contained in the amino acid sequence.

In this embodiment, the amino acid sequence can have a length of 10 to 200 amino acids (such as 10 to 90 amino acids).

(AP) In the peptide tag of the present disclosure,
(a) 5% or more and less than 45% of amino acids contained in the amino acid sequence can be acidic amino acids,
(b1) 55% or more and less than 90% of the amino acids contained in the amino acid sequence can be either of N and P, and
the rest of the amino acids contained in the amino acid sequence can be other amino acids. In this embodiment, 20% or less (preferably 150 or less, 10% or less, 5% or less, 4% or less, 3% or less, 2% or less, 1% or less, or 0%) of the amino acids contained in the amino acid sequence are neither an acidic amino acid nor N and P.

(AQ) In the peptide tag of the present disclosure,
(a) 45% or more of amino acids contained in the amino acid sequence can be acidic amino acids,
(b1) 210 or more of the amino acids contained in the amino acid sequence can be N, and/or 7% or more thereof can be P, and
20% or less (preferably 150 or less, 10% or less, 5% or less, 4% or less, 3% or less, 2% or less, 1% or less, or 0%) of the amino acids contained in the amino acid sequence can be other amino acids.

(AR) In the (AK) to (AQ) above, a rate of the Element 4 can be preferably 0%.

(AS) In the (AK) to (AQ) above, a rate of A or G can be 0%. In the (AK) to (AO) above, a rate of A and G is preferably 0%.

(AT) In the (AK) to (AQ) above, preferably, a rate of the Element 4 is 0%, and a rate of A and G is 0%.

(AU) In the (AR) to (AT) above, a rate of the Element 2 is preferably 0%.

In the (a) above, preferably 10% or more and less than 45% of the amino acids contained in the amino acid sequence are acidic amino acids, more preferably 150 or more and less than 45% thereof are acidic amino acids, further preferably 20% or more and less than 45% thereof are acidic amino acids, still further preferably 25% or more and less than 45% thereof are acidic amino acids, and particularly preferably 30% or more and less than 45% thereof are acidic amino acids. In these examples, the upper limit of the acidic amino acid content can be, for example, less than 45%, 40% or less, or 35% or less.

The peptide tag of the present disclosure can be added to a protein (such as an intracellular aggregating protein). Accordingly, the present disclosure provides a fusion protein of the peptide tag of the present disclosure and an intracellular aggregating protein. The peptide tag of the present disclosure may be added to an intracellular non-aggregating protein. When the tag is added thereto, toughness of the non-aggregating protein against a non-aggregation property can be increased. The protein can be, for example, an intracellular antibody. The intracellular antibody can be an antigen-binding fragment of an antibody. The peptide tag of the present disclosure may be added to a fusion protein of an intracellular antibody and a degradation-inducing sequence. Thus, selective degradation of a target to which the intracellular antibody binds can be induced. Examples of the intracellular antibody include the above-described antibody fragments. Other examples of the intracellular antibody include antibodies that bind to α-synuclein, LRRK2, Tau, β-amyloid, amyloid precursor protein (APP), C9orf72, superoxide dismutase 1 (SOD1), TAR DNA-binding protein 43 (TDP43), Fused in Sarcoma (FUS), and a prion protein, and pathological forms thereof. Another example of the intracellular antibody includes an antibody inhibiting protein-protein interaction (PPI). Still another example of the intracellular antibody includes one in the form of a fusion protein with a degradation-inducing sequence that binds to a target. Other examples of the intracellular antibody include intracellular antibodies described in Molecular Therapy, 29(2): 859-872, 2021 (such as CP13 iB, PHF1 iB, and Tau5 iB), and intracellular antibodies each having all CDR sequences of these intracellular antibodies. "iB" is an abbreviation of "intrabody", and specifically means an intracellular antibody. The present disclosure provides a fusion protein of, for example, such an intracellular antibody and the peptide tag of the present disclosure. The intracellular antibody may preferably further include a degradation-inducing sequence. Other examples of the intracellular antibody include intracellular antibodies described in Molecular Therapy, 30(4): 1484-1499, 2022 (such as VHH E4-1, and VHHZ70), and intracellular antibodies each having all CDR sequences of these intracellular antibodies. The intracellular antibody may preferably further include a degradation-inducing sequence. Still other examples of the intracellular antibody include an intracellular antibody described in J. Biol. Chem., 295(31): 10662-10676, 2020 (such as M204-scFv), and an intracellular antibody having all CDR sequences of this intracellular antibody. The intracellular antibody may preferably further include a degradation-inducing sequence. Still other examples of the intracellular antibody include an intracellular antibody described in WO2018/231254 (such as BIIB092 antibody), an intracellular antibody described in WO2016/207245, an antibody described in WO2018/011073 (such as C10-2), intracellular antibodies described in WO2015/114538 (such as VHH tau A2, VHH tau A2-SH, and VHH tau A2var-SH), intracellular antibodies described in WO2014/059442 (such as F9T, D11C, D4G, G12C, H2A and H7T), and JP2020/515233 (such as IE4, 9B11, 3A9, 10F10, 11F11, AC8, AE8, AA9, DG5, AD2, AD7, DG11, DG8, and DA9) and intracellular antibodies each having all CDR sequences of these intracellular antibodies. The intracellular antibody may preferably further include a degradation-inducing sequence. Examples of the intracellular antibody further include an intracellular antibodies capable of degrading and removing abnormal TDP-43 (such as SEQ ID NOs: 21 to 24) described in WO2019177138, and an intracellular antibody having all CDR sequences of this intracellular antibody. The intracellular antibody may preferably further include a degradation-inducing sequence. Thus, an intracellular antibody (such as a scFv or VHH) binding to tau, an intracellular antibody (such as a scFv or VHH) binding to α-synuclein, and other intracellular antibodies (such as a scFv or VHH) against amyloid causing cytotoxicity in a cell are preferred, and can be linked to the tag to form a fusion protein with the tag.

In one embodiment, the peptide tag of the present disclosure does not have a CAAX motif (such as SEQ ID NO: 58: KLNPPDESGPGCMSCKCVLS). In one embodiment, the peptide of the present disclosure does not have a membrane localization signal. In one embodiment, the peptide tag of the present disclosure does not have a signal peptide sequence for extracellular secretion from the viewpoint of expressing a protein of interest in a cell. In one embodiment, the peptide tag of the present disclosure may have a signal sequence for extracellular secretion from the viewpoint of promoting extracellular secretion of a protein of interest. When a secretory protein has an aggregation property, the peptide tag of the present disclosure containing a signal sequence in the sequence thereof, or the peptide tag of the present disclosure linked tandem to the signal sequence can be advantageous. In one embodiment, the peptide tag of the present disclosure can contain a nuclear localization signal. In one embodiment, the peptide tag of the present disclosure does not have a sequence preventing protein localization in the cytoplasm. In one embodiment, the peptide tag of the present disclosure promotes free distribution in a cell of the protein of interest. In one embodiment, the peptide tag of the present disclosure can promote intracellular bond of the protein of interest to an original binding partner, and co-localization with the binding partner. In one embodiment, the peptide tag of the present disclosure can have a sequence that imposes unique constraints on the distribution in a cell (or a sequence that prevents free distribution) of the protein of interest, but is possible not to have such a sequence.

In any embodiment, the peptide tag of the present disclosure does not have the following sequence (Enzymol. 326, 362-267 (2000)): S-tag: KETAAAKFERQHMDS (SEQ ID NO: 14). In one embodiment, the peptide tag of the present disclosure can have a sequence in which a rate of the Element 2 is 10% or less, and/or a rate of A is 10% or less.

In any embodiment, the peptide tag of the present disclosure does not have KLNPPDESGPGCMSCKCVLS (SEQ ID NO: 15) (Tanaka et al., 2007, EMBO Journal, 26: 3250-3259). In one embodiment, the peptide tag of the present disclosure does not have a sequence having 90% or more sequence identity to this sequence.

In any embodiment, the peptide tag of the present disclosure does not have EFGGAPEFPKPSTPPGSSGL (SEQ ID NO: 16), and a sequence having 90% or more sequence identity to this sequence (Paolo et al., 2003, Clinical Cancer Research, 9: 2837-2848). In one embodiment, the peptide tag of the present disclosure does not have a sequence having 90% or more sequence identity to this sequence.

In any embodiment, the peptide tag of the present disclosure does not have any one of the following sequences (Arimori et al., 2017, Structures, 25: 1611-1622) :
hMst1: DYEFLKSWTVEDLQKRLLALDPMMEQEIEEIRQKYQSKRQPILDAIEAK (SEQ ID NO: 17);
hMST2: DFDFLKNLSLEELQMRLKALDPMMEREIEELRQRYTAKRQPILDAMDAK (SEQ ID NO: 18);
hRaf1: GEVNWDAFSMPELHNFLRILQREEEEHLRQILQKYSYSRQKIQEALHAS (SEQ ID NO: 19);
hRaf5: GEVEWDAFSIPELQNFLTILEKEEQDKIQQVQKKYDKFRQKLEEALRES (SEQ ID NO: 20);
hSAV1: HILKWELFQLADLDTYQGMLKLLFMKELEQIVKMYEAYRQALLTELENR (SEQ ID NO: 21). In one embodiment, the peptide tag of the present disclosure does not have a sequence having 90% or more sequence identity to any one of these sequences.

In any embodiment, the peptide tag of the present disclosure have none of the following (Zhang et al., 2004, Protein Expression and Purification, 36(2): 207-216) :
T7C: 
LEDPFQSGVMLGVASTVAASPEEASVTSTEETLTPAQEAARTRAANKARKEAELAAA

TAEQ (SEQ ID NO: 22);
T7B: LEDPEEASVTSTEETLTPAQEAARTRAANKARKEAELAAATAEQ (SEQ ID NO: 23);
T7B1: LEDPEEASVTSTEETLTPAQEAARTRPPNKARKEAELAAATAEQ (SEQ ID NO: 24);
T7B2: LEDPEEASVTSTEETLTPAQEAARTRGGNKARKEAELAAATAEQ (SEQ ID NO: 25);
T7B3: LEDPEEASVTSTEETLTPAQEAARTRAANKARKEAELTAEQ (SEQ ID NO: 26) ;
T7B4: LEDPEEASVTSTEETLTPAQEAARTRAANKARKEAELEAETAEQ (SEQ ID NO: 27);
T7B5: LEDPEEASVTSTEETLTPAQEAARTRAAAKARKEAELAAATAEQ (SEQ ID NO: 28);
T7B6: LEDPEEASVTSTEETLTPAQEAARTRKARKEAELAAATAEQ (SEQ ID NO: 29);
T7B7: LEDPEEASVTSTEETLTPAQEAARTRAANKARKEAELAA (SEQ ID NO: 30) ;
T7B8: LEDPEEASVTSTEETLTPAQEAARTRAANKARKEAELAAA (SEQ ID NO: 31);
T7B9: LEDPEEASVTSTEETLTPAQEAAETEAANKARKEAELEAETAEQ (SEQ ID NO: 32);
T7B10: LEDPTPAQEAARTRAANKARKEAELAAATAEQ (SEQ ID NO: 33);
T7A: LEDPAANKARKEAELAAATAEQ (SEQ ID NO: 34);
T7A1: LEDPERNKERKEAELAAATAEQ (SEQ ID NO: 35);
T7A2: LEDPERNKERKEAELEAATAEQ (SEQ ID NO: 36);
T7A3: LEDPERNKERKEAELEAETAEQ (SEQ ID NO: 37);
T3: LEDPAVWEAGKVVAKGVGTADITATTSNGLIASCKVIVNAATS (SEQ ID NO: 38);
T3A: LEDPAVWEAGKVVAKGVGTADITATTSNGLIASSEEADNAATS (SEQ ID NO: 39). In one embodiment, the peptide tag of the present disclosure does not have a sequence having 90% or more sequence identity to any one of these sequences.

In any embodiment, the peptide tag of the present disclosure have none of the following (Japanese Patent Laid-Open No. 2015-97519):
Zif628: 
ERPYACPVESCDRRFSRSDELTRHIRIHTGQKPFQCRICMRNFSRSDHLTTHIRTHT

GEKPFACDICGRKFARSDERKRHTKIHLRQKD (SEQ ID NO: 40);
HinR: GRPRAITKHEQEQISRLLEKGHPRQQLAIIFGIGVSTLYRYFPASSIKKRMN (SEQ ID NO: 41); and
TrpR:

MAQQSPYSAAMAEQRHXXQEWLRFVDLLKNAYQNXXDLHLPLLNLMLTPDERXXEAL

GTRVRIVEELLRGEMSQRELKNELGAGIATITRGSNSLKAAPVELRQWLEEVLLKSD

(SEQ ID NO: 42).

In a preferable embodiment, the peptide tag of the present disclosure can be a natural sequence found in a non-human living thing. In a preferable embodiment, the peptide tag of the present disclosure can be a non-natural sequence or a part thereof. In either embodiment, the peptide tag of the present disclosure is none of the following (WO2010/034183):
NE-1: TKENPRSNQEESYDDNES (SEQ ID NO: 43);
NE-8: TKENPRTNQEESYDDNES (SEQ ID NO: 44);
NE-9: TKENPRSNQDESYDDNES (SEQ ID NO: 45);
NE-10: TKENPRSNQPPSYDDNES (SEQ ID NO: 46).

In one embodiment, the peptide tag of the present disclosure is none of the following (WO2011/034605): ACID.P1: GGSAQLEKELQALEKENAQLEWELQALEKELAQGAT (SEQ ID NO: 50) .

In one embodiment, the peptide tag of the present disclosure is none of the following (WO2009/023270): rPEG_K288-GFP: (GEGEGGGEG)₃₂ (SEQ ID NO: 51).

In one embodiment, the peptide tag of the present disclosure is none of the following (WO2020/059228):
Hero7: MTRGNQRELARQKNMKKQSDSVKGKRRDDGLSAAARKQRDSEIMQQKQKK ANEKKEEPK (SEQ ID NO: 1038);
Hero9: 
MSGPNGDLGMPVEAGAEGEEDGFGEAEYAAINSMLDQINSCLDHLEEKNDHLHARLQ

ELLESNRQTRLEFQQQLGEAPSDASP (SEQ ID NO: 1039);
Hero11: 
MAQGQRKFQAHKPAKSKTAAAASEKNRGPRKGGRVIAPKKARVVQQQKLKKNLEVGI

RKKIEHDVVMKASSSLPKKLALLKAPAKKKGAAAATSSKTPS (SEQ ID NO:
1040) .

In one embodiment, the peptide tag of the present disclosure is none of the following (Protein Engineering, Design & Selection, 26(8): 490-501, 2013):
PAS#1: ASPAAPAPASPAAPAPSAPAA (SEQ ID NO: 52);
1P2: ASAAAPAAASAAASAPSAAAA (SEQ ID NO: 53);
PAS#5: AASPAAPSAPPAAASPAAPSAPPAA (SEQ ID NO: 54);
   and repeated sequences of these (the number of repetition being, for example, 200 ± 20 times, 400 ± 40 times, or 600 ± 60 times).

In one embodiment, the peptide tag of the present disclosure is none of the following (Protein Engineering, Design & Selection, 17(11): 779-786, 2004):
Z (W) : 


 (SEQ ID NO: 55);
Z(a1): 
VDNKFNKEQQNAEYEIEHLPNLNEEQENAFIQSLEDDPSQSANLLAEAKKLNDAQAP

K (SEQ ID NO: 56);
Z(a2):

VDNKFNKEEEEAEEEIEHLPNLNEEQEEAFIESLEDDPSQSANLLAEAKKLNDAQAP

K (SEQ ID NO: 57)

In one embodiment, the peptide tag of the present disclosure may have a mutation selected from the group consisting of substitution, insertion, deletion, addition, and elimination of one or more, preferably two or more amino acids in any one of the amino acid sequences of SEQ ID NOs: 43 to 46 and 47 to 58. In one embodiment, the peptide tag of the present disclosure can have less than 90%, 85% or less, 80% or less, 75% or less, 70% or less, 60% or less, 50% or less, 40% or less, 30% or less, 20% or less, or 10% or less sequence identity to any one of the amino acid sequences of SEQ ID NOs: 44 to 47 and 47 to 58.

In any embodiment, the peptide tag of the present disclosure does not contain the following sequences (Protein Science (2019) 28, 823-836):
PA12-tag: GVAMPGAEDDW (SEQ ID NO: 47);
PA14-tag: EGGVAMPGAEDDVV (SEQ ID NO: 48).
In any embodiment, the peptide tag of the present disclosure does not contain the following sequences:

DYKDDDDVEAEESDNVDSADAEEDDSDVWWGGADTDYADGSEDKVVEVAEEEEVAEV
EEEEADDDEDDEDGDEVEEEAEEPYEEATERTTSIATTTTTTTESVEEVYPGQVGYP
GQVGYPGQV (SEQ ID NO: 49).

In one embodiment, the peptide tag of the present disclosure has less than 90%, 80% or less, 70% or less, 60% or less, 50% or less, 40% or less, 30% or less, 20% or less, or 10% or less sequence identity to any one of SEQ ID NOs: 14 to 48 and 49 to 58.

In one embodiment, the peptide tag of the present disclosure can have a sequence satisfying one, two or all of the following (i) to (iii): (i) a rate of the Element 2 is 10% or less, (ii) a rate of A is 10% or less, and (iii) a rate of G is 10% or less.

In a preferable embodiment, the peptide tag of the present disclosure does not have a sequence consecutively containing 5 or more As. In a preferable embodiment, the peptide tag of the present disclosure does not have a sequence consecutively containing 5 or more Qs. In a preferable embodiment, the peptide tag of the present disclosure does not have a sequence consecutively containing 5 or more Ss. In a preferable embodiment, the peptide tag of the present disclosure does not have a sequence consecutively containing 5 or more Ns. In a preferable embodiment, the content of a specific single amino acid in the amino acid sequence of the peptide tag does not exceed 50%, 40%, 35%, 30%, 25%, or 20%. In a preferable embodiment, the peptide tag of the present disclosure does not contain an amino acid sequence having a length of 3 to 8 amino acids described in Table 1 in WO2002/092132, or does not contain a consecutive repeat (for example, consecutive repeat of 2 or more, 3 or more, 4 or more, 5 or more, 6 or more, 7 or more, 8 or more, 9 or more, or 10 or more times) of the amino acid sequence.

For example, the peptide tag of the present disclosure can inhibit an intracellular antibody from forming aggregation to promote uniform distribution in a cell of the antibody, and/or inhibit aggregation formation to promote bond of the intracellular antibody to an antigen in a cell, and co-localization with the antigen. In one embodiment, the peptide tag of the present disclosure is possible not to have a sequence that imposes unique constraints on the distribution in a cell (or a sequence that prevents free distribution) of the intracellular antibody, and/or is possible not to have bond in a cell of the intracellular antibody to an antigen, and co-localization with the antigen.

All the peptide tags of the present disclosure can mitigate, inhibit, or improve aggregation tendency; increase, promote, or improve a non-aggregation property; or increase, promote, or improve stability of a tagged protein. The peptide tag of the present disclosure is possible not to have a sequence that imposes unique constraints on the distribution in a cell (or a sequence that prevents free distribution) of the intracellular antibody, and/or is possible not to have bond in a cell of the intracellular antibody to an antigen and co-localization with the antigen. When the protein of interest is an antigen-binding fragment of an antibody, the peptide tag of the present disclosure can promote co-localization with an antigen through bond of the protein to the antigen.

The peptide tag of the present disclosure can be, for example, a gene product encoded by a gene of a living thing, or a fragment thereof, and here, can be a gene product encoded by a gene of a non-human living thing (for example, a microorganism such as a bacteria, an alga, or a fungus, an animal such as a mammal, a bird, or fish, or a plant), or a fragment thereof. Alternatively, in a preferable embodiment, the peptide tag of the present disclosure can be a gene product encoded by a gene of a human, or a fragment thereof.

When fused with an aggregating protein, the peptide tag of the present disclosure can mitigate, inhibit or improve the aggregation tendency of the aggregating protein, or can increase, promote, or improve the non-aggregation property of the aggregating protein. Aggregation of a protein can adversely affect a cell in which the protein is expressed, and in addition, can adversely affect the protein production amount and functionality by the aggregation. Accordingly, the mitigation, inhibition, or improvement of the aggregation tendency reduces the influence of the aggregation on the cell, and can lead to reduction of the influence on the protein production amount and the functionality. In this manner, the peptide tag of the present disclosure can be beneficial in improvement of the expression level of an aggregating protein expressed in a cell and/or improvement of the functionality, and accordingly, can be useful for forced expression of the aggregating protein *in vivo.*

Accordingly, the peptide tag of the present disclosure may be fused with an aggregating protein. The aggregating protein can be a protein that forms aggregation in 30% or more, 40% or more, 50% or more, 60% or more, 70% or more, 80% or more, or 90% or more of cells when intracellularly expressed. The aggregating protein may be a protein that forms aggregation in 90% or less, 80% or less, 70% or less, 60% or more, or 50% or less of cells when intracellularly expressed. The fusion can be performed, for example, on the N terminal and/or the C terminal (preferably, both the N terminal and the C terminal) of the aggregating protein. The fusion can be conducted by, for example, linking a nucleic acid encoding the peptide tag of the present disclosure and a nucleic acid encoding the aggregating protein, in-frame (in such a manner as to match the reading frames of codons). The peptide tag of the present disclosure may be fused with a non-aggregating protein. Thus, the non-aggregation property of the non-aggregating protein can be further increased. The non-aggregating protein may be a protein that forms aggregation in 20% or less, 15% or less, 10% or less, 9% or less, 8% or less, 7% or less, 6% or less, 5% or less, 4% or less, 3% or less, 2% or less, or 1% or less of cells or a protein that does not form aggregation, when intracellularly expressed.

When fused with a protein, the peptide tag of the present disclosure can increase, promote, or improve the stability under an intracellular environment of the protein. The stability under an intracellular environment of a protein is beneficial in both an aggregating protein and a non-aggregating protein.

In one embodiment, the protein of interest may be linked to a second peptide tag. The second peptide tag can be added to the protein of interest for purpose of, for example, detection or purification. In this case, the peptide tag of the present disclosure can be used for reducing, inhibiting, or improving aggregation tendency; increasing, promoting, or improving a non-aggregation property; or increasing, promoting, or improving stability of a fusion protein of the protein of interest and the second peptide tag. As the second peptide tag, a usual peptide tag, such as an HA tag, can be used.

The aggregating protein is not especially limited, and can be, for example, an antigen-binding fragment of an antibody. The aggregating protein can be preferably a single chain Fv (scFv) or a VHH antibody. A scFv is a fusion protein containing a heavy chain variable region and a light chain variable region of an antibody in which the heavy chain variable region and the light chain variable region are linked via a linker (preferably, a flexible linker). For example, there is an undruggable therapeutic target in a cell. This is conspicuous, for example, when a site for binding to a low molecular weight compound cannot be found in a therapeutic target. An antibody can bind to the target with strong binding affinity with different principles from the low molecular weight compound, and hence can effectively work on the therapeutic target regarded as undruggable with the low molecular weight compound. An antibody is, however, usually extracellularly secreted, and extracellularly functions. Therefore, in order to express, in a cell, a secretory protein (protein extracellularly secreted), a gene can be designed to express a secretory protein (intracellular antibody) in a cell. For example, in order to express a secretory protein (intracellular antibody), a signal sequence of the protein can be disrupted, preferably removed or the like. In particular, a scFv can exhibit an aggregation property in a cell. Accordingly, the tag of the present disclosure can be fused with an aggregating protein, particularly a secretory protein, particularly an antigen-binding fragment of an antibody, and with preferably a scFv. A secretory protein exhibits an aggregation property in a cell in some cases. For stabilizing such a secretory protein before secretion in a cell, the tag of the present disclosure can be effective.

The antibody, or the antigen-binding fragment of the antibody can have binding affinity (KD) to an antigen thereof of, for example, 10⁻⁵ M or less, 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less. A test of the binding property and a test of the binding affinity can be performed, for example, in a buffered saline.

An example of the antigen includes an intracellular antigen such as an intracellular protein. Examples of the intracellular protein include an intracytoplasmic protein (such as an intracellular extravesicular cytoplasmic protein), a nuclear protein (in this case, the peptide tag or the fusion protein may contain a nuclear localization signal), a nuclear transcription factor, a protein binding to a transcription factor, a protein binding to a genomic DNA, a protein binding to a protein binding to a genomic DNA, a constituent protein of chromatin, a protein binding to chromatin, an intracellular cell skeleton, and a protein binding to an intracellular cell skeleton. The intracellular protein is not especially limited, and other examples include a gene product of a cancer driver gene, a protein in an activated signal cascade (particularly in an activated immune cell of a patient having a cancer cell or an immune-related disease), and a gene product of a tumor suppressor gene (particularly under regulation of a binding partner for negative regulation thereof). In one embodiment, the antigen can be Kras.

Introduction of the protein into a cell can be conducted by introducing, into the cell, a protein expression vector containing a nucleic acid encoding a fusion protein (a fusion protein of the peptide tag and the protein of interest) operably linked to a regulatory sequence. The protein expression vector is introduced into a protein-producing cell or a mammal cell, and can express the fusion protein in the cell.

The regulatory sequence can be a promoter capable of transcribing an mRNA, and for example, various types of pol II promoters can be used. The pol II promoters are not especially limited, and examples include a CMV promoter, an EF1 promoter (EF1α promoter), an SV40 promoter, an MSCV promoter, an hTERT promoter, a β actin promoter, a CAG promoter, and a CBh promoter. Further, a promoter driving bacteriophage-derived RNA polymerase, such as a T7 promoter, a T3 promoter, or an SP6 promoter, and a pol III promoter such as a U6 promoter can be used as the promoter. For a cyclic DNA, the T7 promoter can be preferably used, and for a linear DNA, the SP6 promoter can be preferably used. The promoter may be a promoter of a virus. Alternatively, the promoter may be an inducible promoter. The inducible promoter is a promoter capable of inducing expression of a polynucleotide functionally linked to the promoter only in the presence of an inducer driving the promoter. An example of the inducible promoter includes a promoter inducing gene expression by heat such as a heat shock promoter. Another example of the inducible promoter includes a promoter using a drug as the inducer driving the promoter. Examples of such a drug inducible promoter include a Cumate operator sequence, a λ operator sequence (such as 12 × λOp), and a tetracycline-based inducible promoter. An example of the tetracycline-based inducible promoter includes a promoter driving gene expression in the presence of tetracycline or a derivative thereof (such as doxycycline), or a reverse tetracycline controlled transactivator (rtTA). An example of the tetracycline-based inducible promoter includes a TRE3G promoter.

The protein expression vector is not especially limited, and can be a virus vector or a plasmid vector. The virus vector is not especially limited, and examples include a retrovirus vector, a lentivirus vector, an adenovirus vector, an adeno-associated virus vector, a herpes simplex virus vector, a vaccinia virus vector, a Sendai virus vector, and a vesicular stomatitis virus vector. From the viewpoint of changing infectiveness to a cell, these vectors may be of pseudo type. These vectors may be derived from attenuated strains. Such a vector can be appropriately prepared by known technique.

From the viewpoint of convenience in production of the protein expression vector, the protein expression vector may contain a nucleic acid encoding the regulatory sequence and the peptide tag of the present disclosure operably linked to the regulatory sequence, and have, on the downstream of the nucleic acid, a cloning site of a nucleic acid encoding the protein of interest. The cloning site has a restriction enzyme cleavage site uniquely present in the vector, and is suitable for introducing a fragment of a gene encoding the protein of interest. A gene encoding the fusion protein of the peptide tag and the protein of interest is obtained by linking a gene encoding the protein of interest in-frame to a gene encoding the peptide tag. Accordingly, the present disclosure provides a protein expression vector containing a nucleic acid encoding the regulatory sequence and the peptide tag of the present disclosure operably linked to the regulatory sequence. In one preferable embodiment, this vector has, on the downstream of the nucleic acid, a cloning site of a nucleic acid encoding the protein of interest. The present disclosure provides a protein expression vector containing: a nucleic acid encoding the regulatory sequence and the peptide tag of the present disclosure operably linked to the regulatory sequence; and a nucleic acid encoding the protein of interest linked in-frame to the former nucleic acid. In this manner, the fusion protein of the peptide tag and the protein of interest can be expressed in a cell.

The present disclosure provides a messenger RNA (mRNA) containing a nucleic acid encoding the peptide tag of the present disclosure. The mRNA further contains a nucleic acid encoding the protein of interest. The nucleic acid encoding the protein of interest is linked in-frame to the nucleic acid encoding the peptide tag. In one embodiment, at least one or more uridines may be changed to pseudouridines in the mRNA. The pseudouridine can be 1-methyl-pseudouridine. The mRNA can be one transcribed from a cDNA, namely, may not have an intron. The mRNA may have a cap structure at the 5' end (Furuichi Y. & Miura K., Nature, 1975; 253 (5490): 374-5). As the cap structure, a CapO structure can be added to the mRNA by Anti-Reverse Cap Analogues (ARCA) method using a cap analogue (Stepinski J. et al., RNA, 2001 Oct; 7(10): 1486-95). When 2'-O methyltransferase treatment is further performed, the CapO structure of the mRNA can be converted to a Cap1 structure. Such an operation can be performed by an ordinary method, and can be practiced using, for example, a commercially available kit, such as ScriptCap m7G Capping System, ScriptCap 2'-O-Methyltransferase Kit, or T7 mScript Standard mRNA Production System (AR Brown Co., Ltd.). The mRNA may have a poly A chain. The addition of a poly A chain can be performed by an ordinary method, and can be performed, for example, with A-Plus Poly(A) Polymerase Tailing Kit (AR Brown Co., Ltd.). Accordingly, in one embodiment, the mRNA can be an mRNA that has a cap structure at the 5' end, has a poly A chain at the 3' end, and preferably has pseudouridine (preferably 1-methyl-pseudouridine) as at least a part of uridines. The mRNA can be an isolated mRNA or a synthesized mRNA.

The mRNA can be encapsulated in a nanoparticle, such as a lipid nanoparticle (LNP). Thus, degradation of the mRNA in a living body is prevented, and efficiency of delivering the mRNA into a cell is improved. Accordingly, in one embodiment, the mRNA can be an mRNA that has a cap structure at the 5' end, has a poly A chain at the 3' end, and preferably has pseudouridine (preferably 1-methyl-pseudouridine) as at least a part of uridines. Such a lipid nanoparticle encapsulating the mRNA is also provided. The lipid nanoparticle is not especially limited, and lipid nanoparticles described in, for example, US9364435B, US8822668B, US8802644B, and US8058069B2 can be used. Alternatively, the mRNA may be encapsulated in a polyion complex micelle, or a polyion complex polymersome (Miyata et al., Chem. Soc. Rev., 2012, 41, 2562-2574). A nanoparticle refers to a particle having a diameter (for example, a hydrodynamic diameter) less than 1 µm.

Accordingly, the present disclosure provides a nanovesicle (such as a lipid nanovesicle, or a polyion complex polymersome) containing an mRNA at least containing a nucleic acid encoding the peptide tag of the present disclosure. The mRNA can contain a nucleic acid encoding the fusion protein of the peptide tag and the protein of interest.

The present disclosure provides a method for reducing, inhibiting, or improving aggregation tendency of a protein, including fusing, to the protein, the peptide tag of the present disclosure that reduces, inhibits, or improves the aggregation tendency. In this embodiment, the protein can be an aggregating protein. The aggregation tendency can be aggregation tendency under an intracellular environment. The fusion is conducted usually on the N terminal and/or the C terminal of the protein. The method can be an *in vitro* method.

The present disclosure provides a method for increasing, promoting, or improving a non-aggregation property a protein, including fusing, to the protein, the peptide tag of the present disclosure that reduces, inhibits, or improves the aggregation tendency. In this embodiment, the protein can be an aggregating protein. The non-aggregation property can be a non-aggregation property under an intracellular environment. The method can be an *in vitro* method.

The present disclosure provides a method for increasing, promoting, or improving stability of a protein, including fusing, to the protein, the peptide tag of the present disclosure that reduces, inhibits, or improves aggregation tendency. In this embodiment, the protein can be an aggregating protein. In this embodiment, the protein can be a non-aggregation property. The stability can be stability under an intracellular environment. The method can be an *in vitro* method.

The present disclosure provides use of the peptide tag of the present disclosure for reducing, inhibiting, or improving aggregation tendency of a protein. The present disclosure also provides use of the peptide tag of the present disclosure for increasing, promoting, or improving a non-aggregation property of a protein. The present disclosure also provides use of the peptide tag of the present disclosure for increasing, promoting, or improving stability of a protein. The use can be use *in vitro.*

It can be tested by *in vitro* assay how strongly the peptide tag of the present disclosure can reduce, inhibit, or improve the aggregation tendency of a protein; increase, promote, or improve the non-aggregation property; or increase, promote, or improve the stability. For example, a gene encoding the peptide tag of the present disclosure is fused, for introduction into a cell, to the N terminal or the C terminal of a gene encoding an aggregating protein such as a scFv having an amino acid sequence of SEQ ID NO: 1, and thus, the aggregating protein fused with the peptide tag of the present disclosure can be expressed in the cell. An aggregation formed by the aggregating protein is observed with an antibody against the aggregating protein, and thus, a rate (%) of cells having aggregations to cells expressing the aggregating protein can be calculated. This rate can be used for evaluating influence of the peptide tag of the present disclosure on the aggregation tendency, the non-aggregation tendency, and the stability of the protein.

The present disclosure provides a composition containing the peptide tag of the present disclosure. The peptide tag of the present disclosure can be linked to the protein of interest in a reaction solution by, for example, a click reaction. The click reaction can be a Huisgen reaction. One of the peptide tag and the protein of interest is modified with an alkyne and the other is modified with an azide compound, and thus, a 1,2,3-triazole ring is formed to obtain the link therebetween.

The present disclosure provides a composition containing the fusion protein of the peptide tag of the present invention and the protein of interest. The protein of interest can be an aggregating protein in one embodiment. The protein of interest can be an antigen-binding fragment of an antibody in one embodiment. The protein of interest can be a scFv in one embodiment.

The present disclosure provides an mRNA containing a nucleic acid encoding the fusion protein of the peptide tag of the present disclosure and the protein of interest, and a composition containing the mRNA. The present disclosure provides a vesicle containing an mRNA containing a nucleic acid encoding the fusion protein of the peptide tag of the present disclosure and the protein of interest, and a composition containing the vesicle.

The present disclosure provides a protein expression vector containing a nucleic acid encoding the fusion protein of the peptide tag of the present disclosure operably linked to a regulatory sequence and the protein of interest, and a composition containing the protein expression vector.

In one embodiment, the composition may further contain a pharmaceutically acceptable carrier, excipient, and/or additive. The composition can be a pharmaceutical composition in one embodiment.

In all embodiments of the present disclosure, the fusion protein having the peptide tag of the present disclosure linked thereto can be a non-natural protein.

In all embodiments of the present disclosure, the peptide and the protein can be respectively recombinant peptide and protein.

In all embodiments of the present disclosure, regarding a scFv having at least an amino acid sequence of SEQ ID NO: 1, the peptide tag of the present disclosure can reduce, inhibit, or improve aggregation tendency of the protein; increase, promote, or improve a non-aggregation property; or increase, promote, or improve stability.

A peptide, a protein, and a nucleic acid can be isolated, concentrated, or purified. Isolation means that one or more components of a system are separated from a given component. Purification means that a relative concentration of a given component is increased as compared with a concentration of one or more other components of a system. Concentration means that a concentration of a given component is increased.

One aspect of the present disclosure provides:
a method for acquiring (or selecting or identifying) an amino acid sequence (or a nucleic acid encoding the amino acid sequence), including acquiring an amino acid sequence in which:
   (a) 5% or more and less than 45% of amino acids contained in the amino acid sequence are acidic amino acids; and
   (b) 20% or more of the amino acids contained in the amino acid sequence are amino acids selected from the group consisting of F, P, Y, G, S, Q, N, and A.

One aspect of the present disclosure provides:
a method for acquiring (or selecting or identifying) an amino acid sequence (or a nucleic acid encoding the amino acid sequence) including:
acquiring an amino acid sequence satisfying any one of conditions (A) to (AE) and (AF) to (AU) described above, or any combination of these conditions.

In one aspect of the present disclosure, the method for acquiring an amino acid sequence may further include:
selecting or identifying an amino acid sequence of a peptide tag that, when a fusion protein of a peptide having the selected or identified amino acid sequence and a reference protein is expressed in a mammal cell (preferably in a human cell), provides reduction in a proportion of cells in which the fusion protein forms an aggregation (aggregation rate) (or the proportion which is not more than a predetermined value).

In one aspect of the present disclosure, the method for acquiring may further include obtaining a peptide having the amino acid sequence, or a nucleic acid encoding the peptide.

In the method for acquiring, the amino acid to be obtained may have a length of 10 to 200 amino acids (for example, a length of 10 to 90 amino acids).

One aspect of the present disclosure provides a method for acquiring (or selecting or identifying) an amino acid sequence (or a nucleic acid encoding the amino acid sequence) having a length of 10 to 200 amino acids (10 to 90 amino acids), including:
acquiring an amino acid sequence in which:
(a) 5% or more and less than 45% of amino acids contained in the amino acid sequence are acidic amino acids; and
(b) 20% or more of the amino acids contained in the amino acid sequence are amino acids selected from the group consisting of F, P, Y, G, S, Q, N, and A;

Selecting or identifying an amino acid sequence of a peptide tag that, when the fusion protein of a peptide having the selected or identified amino acid sequence and a reference protein (reference protein) is expressed in a mammal cell (preferably in a human cell), provides reduction of a proportion of cells in which a fusion protein forms an aggregation (aggregation rate) (or the proportion which is not more than a predetermined value); and
obtaining a peptide having the amino acid, or a nucleic acid encoding the peptide.

In this method, a peptide having a particularly excellent stabilizing action can be selected from peptides having a stabilizing action by increasing the extent of reduction or by reducing the predetermined value. In one aspect, the present method may further include expressing, in a mammal cell (preferably in a human cell), the fusion protein of the peptide having the selected or identified amino acid sequence and the reference protein. The present method may further include obtaining a nucleic acid encoding the selected or identified amino acid sequence. The predetermined value can be a numerical value based on a rate (%) of cells having an aggregation to cells expressing the aggregating protein. For example, the predetermined value can be a value of 30% or less, a value of 20% or less, a value of 15% or less, a value of 10% or less, a value of 5% or less, a value of 3% or less, a value of 2% or less, or a value of 1% or less, or 0%. The predetermined value can be a value in a range of 0% to 10%, a value in a range of 10% to 20%, or a value in a range of 20 to 30%. When a peptide having a higher effect is desired to be acquired, the predetermined value is preferably smaller. In this embodiment, a peptide tag exhibiting a stronger effect can be selected from, for example, the above-described peptide tags of the present disclosure (for example, any one of the peptide tags of (A) to (Z), (AA) to (AE), and (AF) to (AU) described above).

The reference protein can be, for example, a protein (aggregating protein, such as a scFv) in which the proportion of cells having an aggregation formed therein by the fusion protein is more than 30%, 40% to 50% or less, 50% to 60%, 60% to 70%, 70% to 80%, 80% to 90%, 90% to 95%, 95% to 99%, 99% to 99.9%, or 99.9% or more. The reference protein needs not have special functionality or binding property to an antigen but is used simply for evaluating the reduction of the aggregation rate, and therefore, the CDR sequence thereof may be any sequence. The aggregation rate of the scFv may be varied depending on the amino acid sequence of the CDR. It is possible to search for an amino acid sequence of a peptide tag that provides the aggregation rate not more than the predetermined value in the scFv having the varied aggregation rate. In one preferable embodiment, the reference protein can be a protein having an amino acid sequence of SEQ ID NO: 1.

In one embodiment, the amino acid sequence to be obtained further satisfies the condition defined in (B) described above. In one embodiment, the amino acid sequence to be acquired further satisfies the condition defined in (D) described above. In one embodiment, the amino acid sequence to be acquired further satisfies the condition defined in (E) described above. In one embodiment, the amino acid sequence to be acquired further satisfies the condition defined in (F) described above. In one embodiment, the amino acid sequence to be acquired further satisfies the condition defined in (G) described above. In one embodiment, the amino acid sequence to be acquired further satisfies the condition defined in (H) described above. In one embodiment, the amino acid sequence to be acquired further satisfies the condition defined in (I) described above. In one embodiment, the amino acid sequence to be acquired further satisfies the condition defined in (I) described above. In one embodiment, the amino acid sequence to be acquired further satisfies the condition defined in (J) described above. In one embodiment, the amino acid sequence to be acquired further satisfies the condition defined in (K) described above. In one embodiment, the amino acid sequence to be acquired further satisfies the condition defined in (L) described above. In one embodiment, the amino acid sequence to be acquired further satisfies the condition defined in (M) described above. In one embodiment, the amino acid sequence to be acquired further satisfies the condition defined in (N) described above. In one embodiment, the amino acid sequence to be acquired further satisfies the condition defined in (O) described above. In one embodiment, the amino acid sequence to be acquired further satisfies the condition defined in (P) described above. In one embodiment, the amino acid sequence to be acquired further satisfies the condition defined in (Q) described above. In one embodiment, the amino acid sequence to be acquired further satisfies the condition defined in (R) described above. In one embodiment, the amino acid sequence to be acquired further satisfies the condition defined in (S) described above. In one embodiment, the amino acid sequence to be acquired further satisfies the condition defined in (T) described above. In one embodiment, the amino acid sequence to be acquired further satisfies the condition defined in (U) described above. In one embodiment, the amino acid sequence to be acquired further satisfies the condition defined in (V) described above. In one embodiment, the amino acid sequence to be acquired further satisfies the condition defined in (W) described above. In one embodiment, the amino acid sequence to be acquired further satisfies the condition defined in (X) described above. In one embodiment, the amino acid sequence to be acquired further satisfies the condition defined in (Y) described above. In one embodiment, the amino acid sequence to be acquired further satisfies the condition defined in (Z) described above. In one embodiment, the amino acid sequence to be acquired further satisfies the condition defined in (AA) described above. In one embodiment, the amino acid sequence to be acquired further satisfies the condition defined in (AB) described above. In one embodiment, the amino acid sequence to be acquired further satisfies the condition defined in (AC) described above. In one embodiment, the amino acid sequence to be acquired further satisfies the condition defined in (AD) described above. In one embodiment, the amino acid sequence to be acquired further satisfies the condition defined in (AE) described above. In one embodiment, the amino acid sequence to be acquired further satisfies the condition defined in (AF) described above. In one embodiment, the amino acid sequence to be acquired further satisfies the condition defined in (AG) described above. In one embodiment, the amino acid sequence to be acquired further satisfies the condition defined in (AH) described above. In one embodiment, the amino acid sequence to be acquired further satisfies the condition defined in (AI) described above. In one embodiment, the amino acid sequence to be acquired further satisfies the condition defined in (AJ) described above. In one embodiment, the amino acid sequence to be acquired further satisfies the condition defined in (AK) described above. In one embodiment, the amino acid sequence to be acquired further satisfies the condition defined in (AL) described above. In one embodiment, the amino acid sequence to be acquired further satisfies the condition defined in (AM) described above. In one embodiment, the amino acid sequence to be acquired further satisfies the condition defined in (AN) described above. In one embodiment, the amino acid sequence to be acquired further satisfies the condition defined in (AO) described above. In one embodiment, the amino acid sequence to be acquired further satisfies the condition defined in (AP) described above. In one embodiment, the amino acid sequence to be acquired further satisfies the condition defined in (AQ) described above. In one embodiment, the amino acid sequence to be acquired further satisfies the condition defined in (AR) described above. In one embodiment, the amino acid sequence to be acquired further satisfies the condition defined in (AS) described above. In one embodiment, the amino acid sequence to be acquired further satisfies the condition defined in (AT) described above. In one embodiment, the amino acid sequence to be acquired further satisfies the condition defined in (AU) described above. In one embodiment, the amino acid sequence to be obtained satisfies the condition described in any one or more of (A) to (Z), (AA) to (AE), and (AF) to (AU) described above.

In one embodiment, the amino acid sequence to be acquired can be selected from an amino acid sequence group.

In one embodiment, the amino acid sequence group can be a group of the amino acid sequences defined in (A) above, or can include this group.

In one embodiment, the amino acid sequence group can be a group of the amino acid sequences defined in (B) above, or can include this group.

In one embodiment, the amino acid sequence group can be a group of the amino acid sequences defined in (C) above, or can include this group.

In one embodiment, the amino acid sequence group can be a group of the amino acid sequences defined in (D) above, or can include this group.

In one embodiment, the amino acid sequence group can be a group of the amino acid sequences defined in (E) above, or can include this group.

In one embodiment, the amino acid sequence group can be a group of the amino acid sequences defined in (F) above, or can include this group.

In one embodiment, the amino acid sequence group can be a group of the amino acid sequences defined in (G) above, or can include this group.

In one embodiment, the amino acid sequence group can be a group of the amino acid sequences defined in (H) above, or can include this group.

In one embodiment, the amino acid sequence group can be a group of the amino acid sequences defined in (I) above, or can include this group.

In one embodiment, the amino acid sequence group can be a group of the amino acid sequences defined in (J) above, or can include this group.

In one embodiment, the amino acid sequence group can be a group of the amino acid sequences defined in (K) above, or can include this group.

In one embodiment, the amino acid sequence group can be a group of the amino acid sequences defined in (L) above, or can include this group.

In one embodiment, the amino acid sequence group can be a group of the amino acid sequences defined in (M) above, or can include this group.

In one embodiment, the amino acid sequence group can be a group of the amino acid sequences defined in (N) above, or can include this group.

In one embodiment, the amino acid sequence group can be a group of the amino acid sequences defined in (O) above, or can include this group.

In one embodiment, the amino acid sequence group can be a group of the amino acid sequences defined in (P) above, or can include this group.

In one embodiment, the amino acid sequence group can be a group of the amino acid sequences defined in (Q) above, or can include this group.

In one embodiment, the amino acid sequence group can be a group of the amino acid sequences defined in (R) above, or can include this group.

In one embodiment, the amino acid sequence group can be a group of the amino acid sequences defined in (S) above, or can include this group.

In one embodiment, the amino acid sequence group can be a group of the amino acid sequences defined in (T) above, or can include this group.

In one embodiment, the amino acid sequence group can be a group of the amino acid sequences defined in (U) above, or can include this group.

In one embodiment, the amino acid sequence group can be a group of the amino acid sequences defined in (V) above, or can include this group.

In one embodiment, the amino acid sequence group can be a group of the amino acid sequences defined in (W) above, or can include this group.

In one embodiment, the amino acid sequence group can be a group of the amino acid sequences defined in (X) above, or can include this group.

In one embodiment, the amino acid sequence group can be a group of the amino acid sequences defined in (Y) above, or can include this group.

In one embodiment, the amino acid sequence group can be a group of the amino acid sequences defined in (Z) above, or can include this group.

In one embodiment, the amino acid sequence group can be a group of the amino acid sequences defined in (AA) above, or can include this group.

In one embodiment, the amino acid sequence group can be a group of the amino acid sequences defined in (AB) above, or can include this group.

In one embodiment, the amino acid sequence group can be a group of the amino acid sequences defined in (AC) above, or can include this group.

In one embodiment, the amino acid sequence group can be a group of the amino acid sequences defined in (AD) above, or can include this group.

In one embodiment, the amino acid sequence group can be a group of the amino acid sequences defined in (AE) above, or can include this group.

In one embodiment, the amino acid sequence group can be a group of the amino acid sequences defined in (AF) above, or can include this group.

In one embodiment, the amino acid sequence group can be a group of the amino acid sequences defined in (AG) above, or can include this group.

In one embodiment, the amino acid sequence group can be a group of the amino acid sequences defined in (AH) above, or can include this group.

In one embodiment, the amino acid sequence group can be a group of the amino acid sequences defined in (AI) above, or can include this group.

In one embodiment, the amino acid sequence group can be a group of the amino acid sequences defined in (AJ) above, or can include this group.

In one embodiment, the amino acid sequence group can be a group of the amino acid sequences defined in (AK) above, or can include this group.

In one embodiment, the amino acid sequence group can be a group of the amino acid sequences defined in (AL) above, or can include this group.

In one embodiment, the amino acid sequence group can be a group of the amino acid sequences defined in (AM) above, or can include this group.

In one embodiment, the amino acid sequence group can be a group of the amino acid sequences defined in (AN) above, or can include this group.

In one embodiment, the amino acid sequence group can be a group of the amino acid sequences defined in (AO) above, or can include this group.

In one embodiment, the amino acid sequence group can be a group of the amino acid sequences defined in (AP) above, or can include this group.

In one embodiment, the amino acid sequence group can be a group of the amino acid sequences defined in (AQ) above, or can include this group.

In one embodiment, the amino acid sequence group can be a group of the amino acid sequences defined in (AR) above, or can include this group.

In one embodiment, the amino acid sequence group can be a group of the amino acid sequences defined in (AS) above, or can include this group.

In one embodiment, the amino acid sequence group can be a group of the amino acid sequences defined in (AT) above, or can include this group.

In one embodiment, the amino acid sequence group can be a group of the amino acid sequences defined in (AU) above, or can include this group.

In one preferable embodiment, the aggregation rate of the reference protein is 5 to 10%, and the aggregation rate of the fusion protein is lower than this aggregation rate.

In one preferable embodiment, the aggregation rate of the reference protein is 5 to 10%, and the aggregation rate of the fusion protein is not more than a predetermined value, which is lower than this aggregation rate by 1% or more.

In one preferable embodiment, the aggregation rate of the reference protein is 5 to 10%, and the aggregation rate of the fusion protein is not more than a predetermined value, which is lower than this aggregation rate by 2% or more.

In one preferable embodiment, the aggregation rate of the reference protein is 5 to 10%, and the aggregation rate of the fusion protein is not more than a predetermined value, which is lower than this aggregation rate by 3% or more.

In one preferable embodiment, the aggregation rate of the reference protein is 5 to 10%, and the aggregation rate of the fusion protein is not more than a predetermined value, which is lower than this aggregation rate by 4% or more.

In one preferable embodiment, the aggregation rate of the reference protein is 6 to 10%, and the aggregation rate of the fusion protein is not more than a predetermined value, which is lower than this aggregation rate by 5% or more.

In one preferable embodiment, the aggregation rate of the reference protein is 7 to 10%, and the aggregation rate of the fusion protein is not more than a predetermined value, which is lower than this aggregation rate by 6% or more.

In one preferable embodiment, the aggregation rate of the reference protein is 8 to 10%, and the aggregation rate of the fusion protein is not more than a predetermined value, which is lower than this aggregation rate by 7% or more.

In one preferable embodiment, the aggregation rate of the reference protein is 9 to 10%, and the aggregation rate of the fusion protein is not more than a predetermined value, which is lower than this aggregation rate by 8% or more.

In a preferable embodiment, a ratio of the aggregation rate of the fusion protein to the aggregation rate of the reference protein is not more than a predetermined value, which is 0.9 or less, 0.8 or less, 0.7 or less, 0.6 or less, 0. or less, 0.4 or less, 0.3 or less, 0.2 or less, or 0.1 or less.

In one preferable embodiment, the aggregation rate of the reference protein is 5 to 10%, and the ratio of the aggregation rate of the fusion protein to this aggregation rate is not more than a predetermined value, which is 0.9 or less.

In one preferable embodiment, the aggregation rate of the reference protein is 5 to 10%, and the ratio of the aggregation rate of the fusion protein to this aggregation rate is not more than a predetermined value, which is 0.8 or less.

In one preferable embodiment, the aggregation rate of the reference protein is 5 to 10%, and the ratio of the aggregation rate of the fusion protein to this aggregation rate is not more than a predetermined value, which is 0.7 or less.

In one preferable embodiment, the aggregation rate of the reference protein is 5 to 10%, and the ratio of the aggregation rate of the fusion protein to this aggregation rate is not more than a predetermined value, which is 0.6 or less.

In one preferable embodiment, the aggregation rate of the reference protein is 5 to 10%, and the ratio of the aggregation rate of the fusion protein to this aggregation rate is not more than a predetermined value, which is 0.5 or less.

In one preferable embodiment, the aggregation rate of the reference protein is 5 to 10%, and the ratio of the aggregation rate of the fusion protein to this aggregation rate is not more than a predetermined value, which is 0.4 or less.

In one preferable embodiment, the aggregation rate of the reference protein is 5 to 10%, and the ratio of the aggregation rate of the fusion protein to this aggregation rate is not more than a predetermined value, which is 0.3 or less.

In one preferable embodiment, the aggregation rate of the reference protein is 5 to 10%, and the ratio of the aggregation rate of the fusion protein to this aggregation rate is not more than a predetermined value, which is 0.2 or less.

In one preferable embodiment, the aggregation rate of the reference protein is 5 to 10%, and the ratio of the aggregation rate of the fusion protein to this aggregation rate is not more than a predetermined value, which is 0.1 or less.

In one embodiment, the aggregation rate of the reference protein is less than 5%, and the aggregation rate of the fusion protein is lower than this aggregation rate.

In one preferable embodiment, the aggregation rate of the reference protein is less than 5%, and the ratio of the aggregation rate of the fusion protein to this aggregation rate is not more than a predetermined value, which is 0.9 or less.

In one preferable embodiment, the aggregation rate of the reference protein is less than 5%, and the ratio of the aggregation rate of the fusion protein to this aggregation rate is not more than a predetermined value, which is 0.8 or less.

In one preferable embodiment, the aggregation rate of the reference protein is less than 5%, and the ratio of the aggregation rate of the fusion protein to this aggregation rate is not more than a predetermined value, which is 0.7 or less.

In one preferable embodiment, the aggregation rate of the reference protein is less than 5%, and the ratio of the aggregation rate of the fusion protein to this aggregation rate is not more than a predetermined value, which is 0.6 or less.

In one preferable embodiment, the aggregation rate of the reference protein is less than 5%, and the ratio of the aggregation rate of the fusion protein to this aggregation rate is not more than a predetermined value, which is 0.5 or less.

In one preferable embodiment, the aggregation rate of the reference protein is less than 5%, and the ratio of the aggregation rate of the fusion protein to this aggregation rate is not more than a predetermined value, which is 0.4 or less.

In one preferable embodiment, the aggregation rate of the reference protein is less than 5%, and the ratio of the aggregation rate of the fusion protein to this aggregation rate is not more than a predetermined value, which is 0.3 or less.

In one preferable embodiment, the aggregation rate of the reference protein is less than 5%, and the ratio of the aggregation rate of the fusion protein to this aggregation rate is not more than a predetermined value, which is 0.2 or less.

In one preferable embodiment, the aggregation rate of the reference protein is less than 5%, and the ratio of the aggregation rate of the fusion protein to this aggregation rate is not more than a predetermined value, which is 0.1 or less.

In one preferable embodiment, the aggregation rate of the reference protein is more than 10%, and the aggregation rate of the fusion protein is not more than a predetermined value, which is lower than this aggregation rate by 10% or more.

In one preferable embodiment, the aggregation rate of the reference protein is more than 20%, and the aggregation rate of the fusion protein is not more than a predetermined value, which is lower than this aggregation rate by 10% or more.

In one preferable embodiment, the aggregation rate of the reference protein is more than 20%, and the aggregation rate of the fusion protein is not more than a predetermined value, which is lower than this aggregation rate by 20% or more.

In one preferable embodiment, the aggregation rate of the reference protein is more than 30%, and the aggregation rate of the fusion protein is not more than a predetermined value, which is lower than this aggregation rate by 10% or more.

In one preferable embodiment, the aggregation rate of the reference protein is more than 30%, and the aggregation rate of the fusion protein is not more than a predetermined value, which is lower than this aggregation rate by 20% or more.

In one preferable embodiment, the aggregation rate of the reference protein is more than 30%, and the aggregation rate of the fusion protein is not more than a predetermined value, which is lower than this aggregation rate by 30% or more.

In one preferable embodiment, the aggregation rate of the reference protein is more than 40%, and the aggregation rate of the fusion protein is not more than a predetermined value, which is lower than this aggregation rate by 10% or more.

In one preferable embodiment, the aggregation rate of the reference protein is more than 40%, and the aggregation rate of the fusion protein is not more than a predetermined value, which is lower than this aggregation rate by 20% or more.

In one preferable embodiment, the aggregation rate of the reference protein is more than 40%, and the aggregation rate of the fusion protein is not more than a predetermined value, which is lower than this aggregation rate by 30% or more.

In one preferable embodiment, the aggregation rate of the reference protein is more than 40%, and the aggregation rate of the fusion protein is not more than a predetermined value, which is lower than this aggregation rate by 40% or more.

In one preferable embodiment, the aggregation rate of the reference protein is more than 50%, and the aggregation rate of the fusion protein is not more than a predetermined value, which is lower than this aggregation rate by 10% or more.

In one preferable embodiment, the aggregation rate of the reference protein is more than 50%, and the aggregation rate of the fusion protein is not more than a predetermined value, which is lower than this aggregation rate by 20% or more.

In one preferable embodiment, the aggregation rate of the reference protein is more than 50%, and the aggregation rate of the fusion protein is not more than a predetermined value, which is lower than this aggregation rate by 30% or more.

In one preferable embodiment, the aggregation rate of the reference protein is more than 50%, and the aggregation rate of the fusion protein is not more than a predetermined value, which is lower than this aggregation rate by 40% or more.

In one preferable embodiment, the aggregation rate of the reference protein is more than 50%, and the aggregation rate of the fusion protein is not more than a predetermined value, which is lower than this aggregation rate by 50% or more.

In one preferable embodiment, the aggregation rate of the reference protein is more than 60%, and the aggregation rate of the fusion protein is not more than a predetermined value, which is lower than this aggregation rate by 10% or more.

In one preferable embodiment, the aggregation rate of the reference protein is more than 60%, and the aggregation rate of the fusion protein is not more than a predetermined value, which is lower than this aggregation rate by 20% or more.

In one preferable embodiment, the aggregation rate of the reference protein is more than 60%, and the aggregation rate of the fusion protein is not more than a predetermined value, which is lower than this aggregation rate by 30% or more.

In one preferable embodiment, the aggregation rate of the reference protein is more than 60%, and the aggregation rate of the fusion protein is not more than a predetermined value, which is lower than this aggregation rate by 40% or more.

In one preferable embodiment, the aggregation rate of the reference protein is more than 60%, and the aggregation rate of the fusion protein is not more than a predetermined value, which is lower than this aggregation rate by 50% or more.

In one preferable embodiment, the aggregation rate of the reference protein is more than 60%, and the aggregation rate of the fusion protein is not more than a predetermined value, which is lower than this aggregation rate by 60% or more.

In one preferable embodiment, the aggregation rate of the reference protein is more than 70%, and the aggregation rate of the fusion protein is not more than a predetermined value, which is lower than this aggregation rate by 10% or more.

In one preferable embodiment, the aggregation rate of the reference protein is more than 70%, and the aggregation rate of the fusion protein is not more than a predetermined value, which is lower than this aggregation rate by 20% or more.

In one preferable embodiment, the aggregation rate of the reference protein is more than 70%, and the aggregation rate of the fusion protein is not more than a predetermined value, which is lower than this aggregation rate by 30% or more.

In one preferable embodiment, the aggregation rate of the reference protein is more than 70%, and the aggregation rate of the fusion protein is not more than a predetermined value, which is lower than this aggregation rate by 40% or more.

In one preferable embodiment, the aggregation rate of the reference protein is more than 70%, and the aggregation rate of the fusion protein is not more than a predetermined value, which is lower than this aggregation rate by 50% or more.

In one preferable embodiment, the aggregation rate of the reference protein is more than 70%, and the aggregation rate of the fusion protein is not more than a predetermined value, which is lower than this aggregation rate by 60% or more.

In one preferable embodiment, the aggregation rate of the reference protein is more than 70%, and the aggregation rate of the fusion protein is not more than a predetermined value, which is lower than this aggregation rate by 70% or more.

In one preferable embodiment, the aggregation rate of the reference protein is more than 80%, and the aggregation rate of the fusion protein is not more than a predetermined value, which is lower than this aggregation rate by 10% or more.

In one preferable embodiment, the aggregation rate of the reference protein is more than 80%, and the aggregation rate of the fusion protein is not more than a predetermined value, which is lower than this aggregation rate by 20% or more.

In one preferable embodiment, the aggregation rate of the reference protein is more than 80%, and the aggregation rate of the fusion protein is not more than a predetermined value, which is lower than this aggregation rate by 30% or more.

In one preferable embodiment, the aggregation rate of the reference protein is more than 80%, and the aggregation rate of the fusion protein is not more than a predetermined value, which is lower than this aggregation rate by 40% or more.

In one preferable embodiment, the aggregation rate of the reference protein is more than 80%, and the aggregation rate of the fusion protein is not more than a predetermined value, which is lower than this aggregation rate by 50% or more.

In one preferable embodiment, the aggregation rate of the reference protein is more than 80%, and the aggregation rate of the fusion protein is not more than a predetermined value, which is lower than this aggregation rate by 60% or more.

In one preferable embodiment, the aggregation rate of the reference protein is more than 80%, and the aggregation rate of the fusion protein is not more than a predetermined value, which is lower than this aggregation rate by 70% or more.

In one preferable embodiment, the aggregation rate of the reference protein is more than 80%, and the aggregation rate of the fusion protein is not more than a predetermined value, which is lower than this aggregation rate by 80% or more.

In one preferable embodiment, the aggregation rate of the reference protein is more than 90%, and the aggregation rate of the fusion protein is not more than a predetermined value, which is lower than this aggregation rate by 10% or more.

In one preferable embodiment, the aggregation rate of the reference protein is more than 90%, and the aggregation rate of the fusion protein is not more than a predetermined value, which is lower than this aggregation rate by 20% or more.

In one preferable embodiment, the aggregation rate of the reference protein is more than 90%, and the aggregation rate of the fusion protein is not more than a predetermined value, which is lower than this aggregation rate by 30% or more.

In one preferable embodiment, the aggregation rate of the reference protein is more than 90%, and the aggregation rate of the fusion protein is not more than a predetermined value, which is lower than this aggregation rate by 40% or more.

In one preferable embodiment, the aggregation rate of the reference protein is more than 90%, and the aggregation rate of the fusion protein is not more than a predetermined value, which is lower than this aggregation rate by 50% or more.

In one preferable embodiment, the aggregation rate of the reference protein is more than 90%, and the aggregation rate of the fusion protein is not more than a predetermined value, which is lower than this aggregation rate by 60% or more.

In one preferable embodiment, the aggregation rate of the reference protein is more than 90%, and the aggregation rate of the fusion protein is not more than a predetermined value, which is lower than this aggregation rate by 70% or more.

In one preferable embodiment, the aggregation rate of the reference protein is more than 90%, and the aggregation rate of the fusion protein is not more than a predetermined value, which is lower than this aggregation rate by 80% or more.

In one preferable embodiment, the aggregation rate of the reference protein is more than 90%, and the aggregation rate of the fusion protein is not more than a predetermined value, which is lower than this aggregation rate by 90% or more.

A nucleic acid encoding the obtained peptide tag is linked in-frame to a nucleic acid encoding the protein of interest, and thus, a nucleic acid encoding the fusion protein of the peptide tag and the protein of interest can be obtained. From the nucleic acid encoding the fusion protein, the fusion protein can be expressed. A protein expression vector containing a nucleic acid encoding a fusion protein operably linked to a regulatory sequence may be prepared.

In one aspect of the present disclosure, the method can be employed for determining whether or not a peptide tag having an amino acid sequence with a length of 10 to 200 amino acids (for example, 10 to 90 amino acids) has an effect of improving an aggregation property not less than a predetermined intensity against a tagged protein. In other words, in one aspect of the present disclosure, a method for determining whether or not a peptide tag having an amino acid sequence with a length of 10 to 90 amino acids has an effect of improving an aggregation property not less than a predetermined intensity against a tagged protein, and the method including:
acquiring an amino acid sequence in which:
(α) 5% or more and less than 45% of amino acids contained in the amino acid sequence are acidic amino acids; and
(β) 20% or more of the amino acids contained in the amino acid sequence are amino acids selected from the group consisting of F, P, Y, G, S, Q, N, and A;
determining that an amino acid sequence of a peptide tag that, when the fusion protein of a peptide having the selected or identified amino acid sequence and a reference protein is expressed in a mammal cell (preferably in a human cell), provides reduction of a proportion of cells in which a fusion protein forms an aggregation (aggregation rate) (or the proportion which is not more than a predetermined value), has the effect of improving an aggregation property (for example, an aggregation property not less than a predetermined intensity).
In one embodiment, the method may further include expressing, in a mammal cell (preferably in a human cell), the fusion protein of the peptide having the selected or identified amino acid sequence and the reference protein. In one embodiment, the amino acid sequence to be acquired can be selected from an amino acid sequence group. The details of the amino acid group are the same as those described above. In one aspect, the amino acid sequence to be acquired satisfies another one or more conditions. The conditions are the same as those described above. In one embodiment, the aggregation rate of the reference protein and the predetermined value are the same as those described above.

In one embodiment, in the method for selecting or identifying an amino acid sequence having a length of 10 to 200 amino acids (for example, 10 to 90 amino acids), the amino acid sequence group having a length of 10 to 200 amino acids (for example, 10 to 90 amino acids) can be a group of amino acid sequences encoded by the coding region of the human genome. In one embodiment, the amino acid sequence group having a length of 10 to 90 amino acids can be a group of amino acid sequences encoded by the coding region of the genome of a non-human living thing.

In one embodiment, the amino acid sequence having a length of 10 to 200 amino acids (for example, 10 to 90 amino acids) can be a neo-antigen. A neo-antigen was discovered as a mutant antigen newly caused by gene mutation peculiar to a cancer cell. The neo-antigen is not expressed in a non-cancer cell. It is expected that immunity can be induced specifically to cancer by inducing immunity to a neo-antigen by administering a peptide containing the neo-antigen. The neo-antigen can be different among cancer cells. The neo-antigen can be used, for example, for tagging a protein of interest to be expressed in a cell having the neo-antigen, and can be thus usable because it is a peptide originally expressed, and hence the cell is not or little adversely affected. The neo-antigen can be a naturally occurring mutant. The neo-antigen has one or more mutations selected from the group consisting of addition, insertion, deletion, and substitution in, for example, a wild type sequence thereof. For example, a neo-antigen of a human typically has one or more (for example, 1 to 10, for example, 1 to several, 1 to 5, 1 to 4, 1 to 3, 2 or 1) mutations selected from the group consisting of addition, insertion, deletion, and substitution in a wild type sequence of a human. The neo-antigen of a human can have, for example, 80% or more identity, 85% or more identity, 90% or more identity, or 95% or more identity to the wild type sequence of a human.

In one preferable embodiment, the reference protein is a scFv. In one embodiment, the scFv has the amino acid sequence of SEQ ID NO: 1.

Still another aspect of the present invention provides a method for modifying an amino acid sequence of a peptide tag, including:
preparing a peptide tag (that may be any one of the peptide tags disclosed herein) for producing a fusion protein; and
obtaining a modified amino acid sequence by substituting, with either of P and N, one or more (preferably, a plurality of) amino acids of the Element 2, 3, or 4 (preferably, any one of the Element 2, the Element 4, and A, G, Y, and F).
The method of this aspect may further include:
determining that an amino acid sequence of a peptide tag that, when the fusion protein of a peptide having a selected or identified amino acid sequence and a reference protein is expressed in a mammal cell (preferably in a human cell), provides reduction of a proportion of cells in which a fusion protein forms an aggregation (aggregation rate) (or the proportion which is not more than a predetermined value), has the effect of improving an aggregation property (for example, an aggregation property not less than a predetermined intensity).
In this manner, modification having particularly strong aggregation reducing action can be performed.

### Examples

### Example 1

### [Method]

### - Construction of Gene Expression Vector

A gene fragment encoding a fusion protein containing a peptide tag and an aggregating protein was produced by Eurofins Genomics K.K. or VectorBuilder Japan, Inc. The thus synthesized gene fragment was cloned into a pEF-BOS vector (Mizushima and Nagata, Nucleic Acids Res. 1990 Sep 11; 18(17): 5322). As the aggregating protein, a protein (specifically, a scFv) having an amino acid sequence set forth in SEQ ID NO: 1 was used. This aggregating protein aggregates in the cytoplasm when expressed in a cell. The sequences of tags used here and SEQ ID NOs thereof were as shown in Table 1 below.

**[Table 1]**

| Table 1: List of Tags used in Experiment | | |
|---|---|---|
| Tag Name | Amino Acid Sequence | SEQ ID NO: |
| Tag-1-1 | AHSSSAESESTSDSDSSSDSESESSSSDSEGS | 2 |
| Tag-1-2 | AHSLSAELESTIDSDCSSDWESELSSSDSEGS | 3 |
| Tag-1-3 | AQSSSAESESGSDSDSSSDSESESSSSDSEGS | 4 |
| Tag-4-1 | NEGYREAFDEDYEQQDEDFAEQDPDGNEAFEGEYDGPNQDEYPDEAQNFE | 5 |
| Tag-2-1 | DEAGSSGAPADEAGSSGAPADEAGS SGAPADEAGSSGAPADEAGSSGAPA GS | 6 |
| Tag-2-2 | DEVGISLAPTDEVGISLAPTDEVGISLAPTDEVGISLAPTDEVGISLAPT GS | 7 |
| Tag-2-3 | DEVMISLWPTDEVMISLWPTDEVMI SLWPTDEVMISLWPTDEVMIS LWPT GS | 8 |
| Tag-3-1 | DFAGSSGAPADFAGSSGAPADFAGSSGAPADFAGSSGAPADFAGSSGAPA GS | 9 |
| Tag-3-2 | DTAVSSIAPLDTAVSSIAPLDTAVSSIAPLDTAVSSIAPLDTAVSSIAPL GS | 10 |
| Tag-3-3 | DTWVSLIAILDTWVSLIAILDTWVSLIAILDTWVSLIAILDTWVSLIAIL GS | 11 |
| Myc | EQKLISEEDL | 12 |

Specific content ratios of amino acids were as follows.

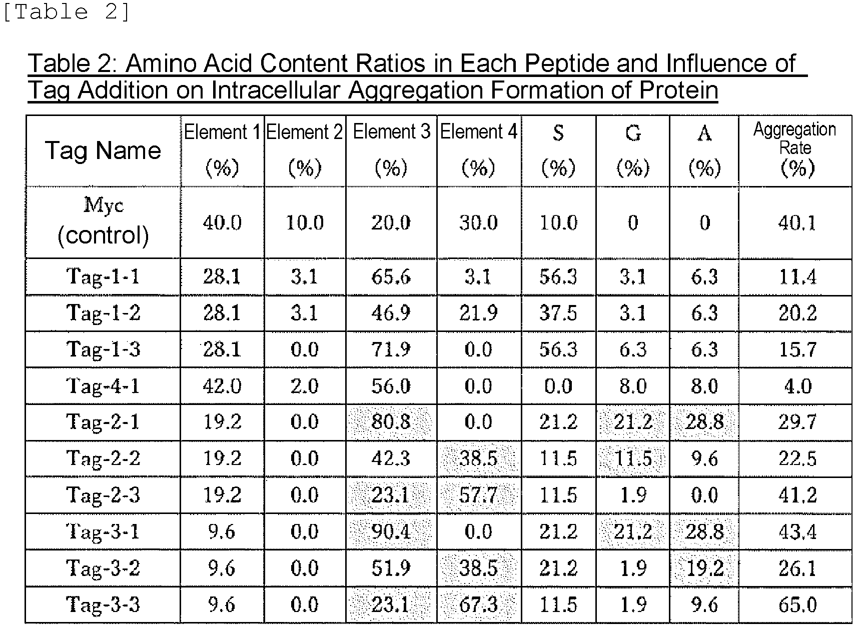

In Table 2, Element 1 refers to D and E, Element 2 refers to H, K, and R, Element 3 refers to F, P, Y, G, S, Q, N, and A, and Element 4 refers to the other amino acids.

The peptide tag was fused to the N terminal of an antibody fragment. The antibody fragment fused with the peptide tag was linked, for detection, to an HA tag of SEQ ID NO: 13 at the C terminal of the antibody fragment.

### - Measurement of Intracellular Aggregation Rate of Intracellular Antibody

### (Cell Culture)

A HeLa cell derived from human cervical epithelial cancer was prepared. The HeLa cell was purchased from JCRB Cell Bank, National Institutes of Biomedical Innovation, Health and Nutrition (JCRB9004), and cultured in DMEM (D-MEM, FUJIFILM Wako Pure Chemical Corporation, 4548995066251) containing 10% FBS.

### (Transfection)

In a 35 mm glass bottom dish (IWAKI 3911-035, glass hole, inner diameter: 12 mm) coated with poly-L-lysine (Sigma-Aldrich P1399 Poly-L-lysine hydrobromide mol. Wt. 150000-300000), 4 × 10⁵ HeLa cells were plated, and after 24 hours, the antibody fragment gene described above was introduced into the HeLa cells with Lipofectamine 3000 (Invitrogen, L3000-008) based on a use method provided by the manufacturer, and thus, a tagged antibody fragment was expressed in the cells. A culture fluid was removed 24 hours after the transfection, and the resultant cells were fixed with 4% PFA. 20 minutes after the fixation, the resultant cells were washed with PBS(-), and thereafter, the antibody expressed in the cells (hereinafter, simply referred to as the "intracellular antibody") was observed by immunostaining to measure the aggregation rate thereof.

### (Immunostaining)

The immunostaining was performed by a standard method. The cells were treated with 0.3% Triton X-100/PBS(-) for 2 minutes, and kept for 1 hour at room temperature in a blocking solution (1% BSA, 0.1% Triton X-100/PBS(-)). The cells were kept for 2 hours at room temperature in an anti-HA antibody (rabbit anti-Ha antibody: Sigma-Aldrich, H6908) diluted with the blocking solution, the resultant cells were washed with the blocking solution, and then the resultant cells were kept at room temperature in Alexa Fluor 488 Goat anti-rabbit IgG (H+L) (Invitrogen, A11034) for 2 hours. The resultant cells were washed with the blocking solution, kept for 15 minutes at room temperature in a nuclear staining probe (NucBlue Fixed cell stain ReadyProbes, Invitrogen, R37606), washed with PBS(-), and stored at 4°C until fluorescence imaging.

### (Fluorescence Imaging)

### The fluorescence imaging was performed with Keyence BZ

-X700 or BZ-X800 using a 40× objective lens. An image including 200 or more cells per dish was acquired to count the number of cells having aggregation of the intracellular antibody therein (intracellular antibody aggregating cells). The number of the intracellular antibody aggregating cells was normalized with the total number of cells expressing the intracellular antibody to quantify an intracellular aggregation rate of the intracellular antibody.

### [Results]

The peptides having the amino acid sequences shown in Table 1 were fused with antigen-binding fragments (specifically, scFvs) of antibodies, and the thus obtained fusion proteins were expressed in the cytoplasm of the HeLa cell. The amino acid ratio in each peptide tag are shown in Table 2. The intracellular aggregation rates obtained based on fluorescence images were as shown in Table 2. In Table 2, a molecule provided with a Myc tag was used as a control, and the effect of each peptide tag was evaluated based on a difference in the aggregation rate from that of the Myc tag.

As shown in Table 2,
- the aggregation rate tended to be reduced when the rate of the Element 3 was in a range of 40 to 75%;
- the aggregation rate tended to be reduced as the rate of the Element 4 was lower;
- the aggregation rate tended to be increased when the rate of alanine (A) was more than 10%;
- the aggregation rate tended to be increased when the rate of glycine (G) was more than 10%; and
- the intracellular aggregation rate tended to be reduced as the rate of the Element 1 was higher.

Serine (S) may not be present (see Tag-4-1), but did not adversely affect the reduction of the aggregation rate even present in a large amount (see Tag-1-1 to Tag-1-3) .

In either case, a peptide tag having an acidic amino acid content less than 45% did not exhibit non-specific adsorption (particularly, non-specific adsorption due to negative charge of the peptide tag) to an intracellular protein or the like. It was suggested that the peptide tag of the present disclosure is useful from the viewpoint that free intracellular localization of a protein of interest is not restricted peculiarly to the tag.

It is understood, through comparison between the Myc tag (control) and Tag-4-1, that reduction of the Element 4 with increase of the Element 3 makes a strong contribution to the aggregation rate reducing action of the peptide tag. Similarly, it is understood, also through comparison between Tag-1-2 and Tag-1-1, that the reduction of the Element 4 with increase of the Element 3 makes a contribution to the aggregation rate reducing action of the peptide tag.

In particular, when the rate of the Element 1 was 20% or more and less than 45%, that of the Element 2 was 10% or less, that of the Element 3 was 40 to 75%, that of the Element 4 was 10% or less, that of alanine was 10% or less, and that of glycine was 10% or less, the aggregation rate was favorably reduced.

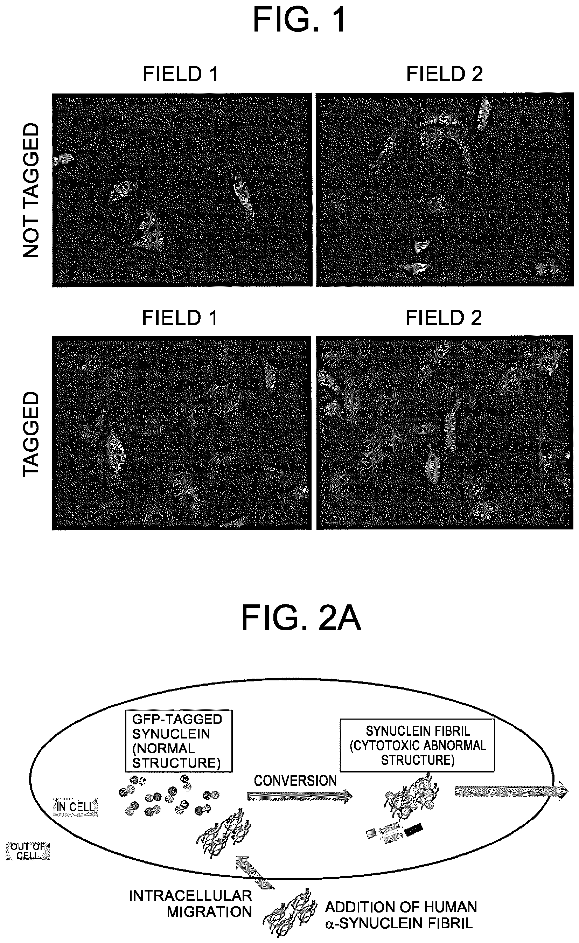

The HeLa cell expressing the fusion protein of the aggregating protein and the tag (Tag4-1) was observed under a fluorescence microscope. As a negative control, a HeLa cell expressing an aggregating protein without Tag4-1 was observed under a fluorescence microscope. Results were as illustrated in Figure 1. As illustrated in Figure 1, when tagged with Tag4-1 (fused with Tag-4-1), the aggregating protein homogeneously distributed in the cytoplasm, but the aggregating protein without Tag4-1 formed an aggregation in the cytoplasms of most of cells.

### Example 2: Experiment of Adding Amino Acid to Tag

Amino acid contents in a tag and the aggregation rate of a tagged protein were further analyzed. Serine (S) was randomly inserted into or added to Tag4-1 or Tag11-1, and the effect of the thus obtained modified tags on the protein aggregation rate was examined. The experiment was conducted in the same manner as described in Example above except that the tags were different. Results were as shown in Table 3. In Table 3, added or inserted amino acids are underlined.

**[Table 3]**

| Table 3: Sequences, S Contents, D/E Contents, and Aggregation Rates of Modified Tags | | | | |
|---|---|---|---|---|
| Tag | sequence | Aggregation Rate | S (%) | D,E(%) |
| Tag4-1 | NEGYREAFDEDYEQQDEDFAEQDPDGNEA FEGEYDGPNQDEYPDEAQNFE | 4.57 | 0 | 42 |
| (SEQ ID NO:59) | | | | |
| Tag4-1-S10 | NEGSYREAFDSEDYEQSQDSEDFSAEQDPD GNSEAFEGSEYSDGPNSQDEYPDEAQNFSE | 5.06 | 16.7 | 35 |
| (SEQ ID NO:60) | | | | |
| Tag4-1-S20 | NEGSYRESAFDSEDYEQSQDSSEDFSAEQDS SPDGNSSEAFEGSSEYSDGPNSQDEYPDEA SSQNFSSSE | 6.76 | 28.6 | 30 |
| (SEQ ID NO:61) | | | | |
| Tag11-1 | YDNPYFEPQYGFPPEEDEDE | 14.48 | 0 | 15 |
| (SEQ ID NO:62) | | | | |
| Tag11-1-S10 | SYDSNPSYFSEPSQYGSFPPSESEDSEDSE | 11.29 | 33.3 | 10 |
| (SEQ ID NO:63) | | | | |
| Tag11-1-S20 | SSYSDSNPSYFSEPSSQSSYGSSFPPSESEDSS ESDSSSE | 18.77 | 50 | 7.5 |
| (SEQ ID NO:64) | | | | |

As shown in Table 3 as results, increase of S in a tag did not largely affect the aggregation rate of the tagged peptide. Although it is known that increase of acidic amino acids in a tag largely affects reduction of the aggregation rate of the tagged peptide (US2020/0157210A), on the contrary, the reduction of the rate of acidic amino acids did not largely reduce the aggregation rate in this example.

Similarly, amino acid contents except for serine were changed to examine the influence of tagged peptides on the aggregation rate. In Tables 4 to 8, added or inserted amino acids are underlined.

**[Table 4]**

| Table 4: Sequences, Q Contents, D/E Contents, and Aggregation Rates of Modified Tags | | | | |
|---|---|---|---|---|
| Tag | sequence | Aggregation Rate | Q (%) | D,E(%) |
| Tag 4-1 | NEGYREAFDEDYEQQDEDFAEQDPDGNEAF EGEYDGPNQDEYPDEAQNFE | 4.88 | 10 | 42 |
| Tag4-1-Q10 | NEGQYREAFDQEDYEQQQDQEDFQAEQDP DGNQEAFEGQEYDGPNQQDEYQPDEAQNF QE | 8.31 | 25 | 35 |
| (SEQ ID NO:65) | | | | |
| Tag4-1-Q20 | NEGQYREQAFDQEDYEQQQDQQEDFQAEQ DQQPDGNQQEAFEGQQEYQDGPNQQDEY PDEAQQQNFQQQE | 8.40 | 35.7 | 30 |
| (SEQ ID NO:66) | | | | |
| Tag 11-1 | YDNPYFEPQYGFPPEEDEDE | 16.59 | 5 | 15 |
| Tag11-1-Q10 | QYDQNPQYFQEPQQYGQFPPQEQEDQEDQ E | 26.70 | 36.7 | 10 |
| (SEQ ID NO:67) | | | | |
| Tag11-1-Q20 | QQYQDQNPQYFQEPQQQQYGQQFPQPQE QEDQQEQDQQQE | 17.27 | 52.5 | 7.5 |
| (SEQ ID NO:68) | | | | |

**[Table 5]**

| Table 5: Sequences, N Contents, D/E Contents, and Aggregation Rates of Modified Tags | | | | |
|---|---|---|---|---|
| Tag | sequence | Aggregation Rate | N (%) | D,E(%) |
| Tag 4-1 | NEGYREAFDEDYEQQDEDFAEQDPDGNEAF EGEYDGPNQDEYPDEAQNFE | 4.88 | 8.00 | 42 |
| Tag4-1-N10 | NEGNYREAFDNEDYEQNQDNEDFNAEQDP DGNNEAFEGNEYNDGPNNQDEYPDEAQNF NE | 6.12 | 23.33 | 35 |
| (SEQ ID NO:69) | | | | |
| Tag4-1-N20 | NEGNYRENAFDNEDYEQNQDNNEDFNAEQ DNNPDGNNNEAFEGNNEYNDGPNNQDEYP DEANNQNFNNNE | 6.86 | 34.29 | 30 |
| (SEQ ID NO:70) | | | | |
| Tag 11-1 | YDNPYFEPQYGFPPEEDEDE | 16.59 | 5.00 | 15 |
| Tag11-1-N10 | NYDNNPNYFNEPNQYGNFPPNENEDNEDNE | 18.56 | 36.87 | 10 |
| (SEQ ID NO:71) | | | | |
| Tag11-1-N20 | NNYNDNNPNYFNEPNNQNNYGNNFPPNEN EDNNENDNNNE | 17.68 | 52.50 | 7.5 |
| (SEQ ID NO:72) | | | | |

**[Table 6]**

| Table 6: Sequences, P Contents, D/E Contents, and Aggregation Rates of Modified Tags | | | | |
|---|---|---|---|---|
| Tag | sequence | Aggregation Rate Rate | P (%) | D,E(%) |
| Tag 4-1 | NEGYREAFDEDYEQQDEDFAEQDPDGNEAF EGEYDGPNQDEYPDEAQNFE | 4.49 | 6.00 | 42 |
| Tag4-1-P10 | NEGPYREAFDPEDYEQPQDPEDFPAEQDPDG NPEAFEGPEYPDGPNPQDEYPDEAQNFPE | 8.84 | 21.67 | 35 |
| (SEQ ID NO:73) | | | | |
| Tag4-1-P20 | NEGPYREPAFDPEDYEQPQDPPEDFPAEQDP PPDGNPPEAFEGPPEYPDGPNPQDEYPDEAP PQNFPPPE | 6.44 | 32,86 | 30 |
| (SEQ ID NO:74) | | | | |
| Tag 11-1 | YDNPYFEPQYGFPPEEDEDE | 17.75 | 20.00 | 15 |
| Tag11-1-P10 | PYDPNPPYFPEPPQYGPFPPPEPEDPEDPE | 15.57 | 46.67 | 10 |
| (SEQ ID NO:75) | | | | |
| Tag 11-1-P2 0 | PPYPDPNPPYFPEPPPQPPYGPPFPPPEPEDP PEPDPPPE | 18.12 | 60.00 | 7.5 |
| (SEQ ID NO:76) | | | | |

**[Table 7]**

| Table 7: Sequences, F Contents, D/E Contents, and Aggregation Rates of Modified Tags | | | | |
|---|---|---|---|---|
| Tag | sequence | Aggregation Rate | F (%) | D,E(%) |
| Tag 4-1 | NEGYREAFDEDYEQQDEDFAEQDPDGNEAF EGEYDGPNQDEYPDEAQNFE | 4.90 | 8.00 | 42 |
| Tag4-1-F10 | NEGEYREAFDEEDYEQEQDEEDFEAEQDPDG NEEAFEGEEYEDGPNEQDEYPDEAQNFEE | 35.13 | 34.29 | 35 |
| (SEQ ID NO:77) | | | | |
| Tag4-1-F20 | NEGEYREEAFDEEDYEQEQDEEEDFEAEQDEE PDGNEEEAFEGEEEYEDGPNEQDEYPDEAEE QNFEEEE | 38.76 | 23.33 | 30 |
| (SEQ ID NO:78) | | | | |
| Tag 11-1 | YDNPYFEPQYGFPPEEDEDE | 16.59 | 10.00 | 15 |
| Tag11-1-F10 | EYDENPEYFEEPEQYGEFPPFEFEDFEDFE | 43.58 | 55.00 | 10 |
| (SEQ ID NO:79) | | | | |
| Tag11-1-F20 | EEYEDENPEYFEEPEEQEEYGEEFPPEEEEDEEE EDEEEE | 27.43 | 40.00 | 7.5 |
| (SEQ ID NO:80) | | | | |

**[Table 8]**

| Table 8: Sequences, Y Contents, D/E Contents, and Aggregation Rates of Modified Tags | | | | |
|---|---|---|---|---|
| | | | | |
| Tag | sequence | Aggregation Rate | Y (%) | D,E(%) |
| Tag 4-1 | NEGYREAFDEDYEQQDEDFAEQDPDGNEAF EGEYDGPNQDEYPDEAQNFE | 4.94 | 8.00 | 42 |
| Tag4-1-Y10 | NEGYYREAFDYEDYEQYQDYEDFYAEQDPDG NYEAFEGYEYYDGPNYQDEYPDEAQNFYE | 29.59 | 23.33 | 35 |
| (SEQ ID NO:81) | | | | |
| Tag4-1-Y20 | NEGYYREYAFDYEDYEQYQDYYEDFYAEQDYY PDGNYYEAFEGYYEYYDGPNYQDEYPDEAYY QNFYYYE | 37.93 | 34.29 | 30 |
| (SEQ ID NO:82) | | | | |
| Tag 11-1 | YDNPYFEPQYGFPPEEDEDE | 17.75 | 15.00 | 15 |
| Tag11.1.Y10 | YYDYN PYVFYEPYQYGYFPPYEYEDYE DYE | 17.28 | 43.33 | 10 |
| (SEQ ID NO:83) | | | | |
| Tag11-1-Y20 | YYYYDYNPYYFYEPYYQYYYGYYFPPYEYEDYY EYDYYYE | 30.53 | 57.50 | 7.5 |
| (SEQ ID NO:84) | | | | |

As described above, when the influence on the protein aggregation rate of tags obtained by randomly adding or inserting 10 to 20 specific amino acids was examined, the addition or insertion of F and Y tended to worsen the aggregation rate of the tagged proteins. The adverse effect of the addition or insertion of the other amino acids on the aggregation rates of tagged proteins was restrictive even if D and E contents were reduced.

### Example 3: Effect on Aggregation Rate of Protein by Amino Acid Content Change by Substitution of Amino Acids in Tag

N, P, S, Q, F, and Y were respectively substituted with other amino acids, and thus, attempts were made to specify, among these amino acids, an amino acid exhibiting the effect on the protein aggregation rate. In Tables 9 and 10, substituted amino acids are underlined.

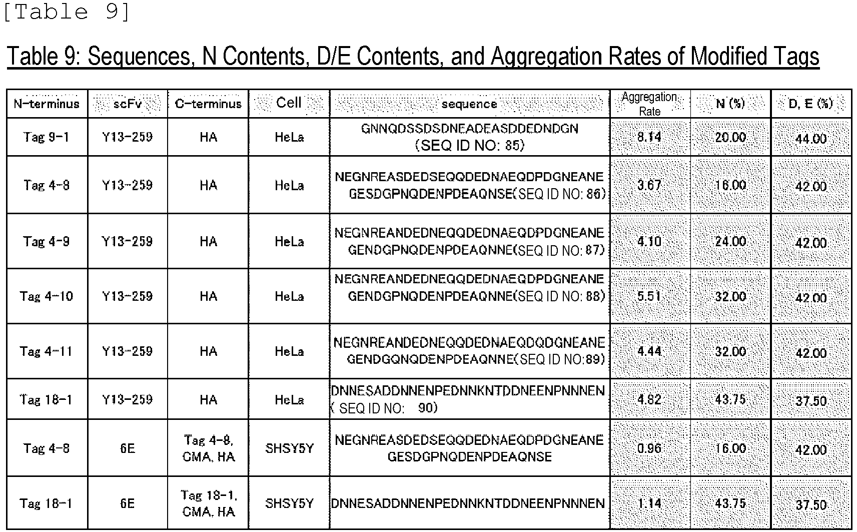

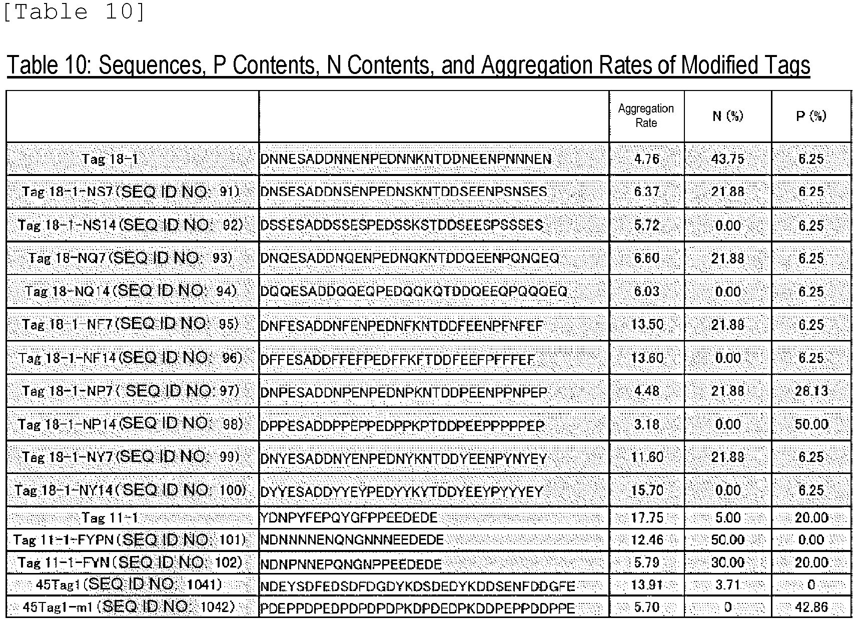

It was found, based on Tables 9 and 10, that the aggregation rate reducing action of a tagged protein is the greatest when the content of P or N in the tag was higher, and subsequently, the aggregation rate reducing action was exhibited in the order that the content of S and Q was higher, and the content of F and Y was higher. 45Tag1-m1 peptide tag, which was produced by substituting, with P, all of the Element 3 in 45Tag1 having an acidic amino acid content of about 51%, largely improved the aggregation inhibiting action thereof through the amino acid substitution with P. In this manner, it was revealed that the increase of the aggregation rate reducing action by adding P and N does not depend on the acidic amino acid content. This result shows that most of constituent amino acids can be acidic amino acids, and N or P.

### Example 4: Sequence Shuffle

Two tags, Tag4-8 (Scrl) and Tag4-8 (Scr2), were synthesized by randomly shuffling the amino acid sequence of Tag4-8, and were tested for the aggregation rate reducing action against the scFv in the same manner as described above. Results were as shown in Table 11.

**[Table 11]**

| Table 11: Sequence Shuffling | | |
|---|---|---|
| | Sequence | Aggregation Rate |
| Tag4-8 | NEGNREASDEDSEQQDEDNAEQDPD GNEANEGESDGPNQDENPDEAQNSE | 3.93 |
| . Tag4-8 (5er1) | ENESEDNDEEEPNQNADDGDPNANP AAEQGGDSSDDEEGQENDQSRENEQ | 3.25 |
| (SEQ ID NO: 103) | | |
| . Tag4-8(Scr2) | QNENNGDDQDQEEGSEEQQGESDRS NEESDNEPAADDNAPEGAEEPDDNN | 4.52 |
| (SEQ ID NO:104) | | |

As shown in Table 11, Tag4-8, Tag4-8 (Scrl) and Tag4-8 (Scr2) exhibited equivalent aggregation rate reducing actions. Even when only the order of amino acids was changed without changing the content ratios and the lengths of the amino acids, the aggregation rate reducing action was not affected.

### Example 5: Use as Tag of Human-derived Peptide

From human proteome database (Proteome ID: UP000005640), peptides having specific amino acid content ratios were all extracted, these peptides were randomly selected to be used as tags, and thus, the aggregation rate reducing action against a tagged protein was examined.

### Extraction Condition 1:

length: 20 to 70 amino acids
group [D, E]: content of [30] or more
group [D, E]: content of less than [45]
group [H, K, R]: content of [5] or less
group [C, T, V, L, I, W, M]: content of [5] or less
group [G]: content of less than [10]
group [A]: content of less than [10]
group [F, Y]: content of [5] or less
group [N]: content of [15] or more
* In the above-described extraction condition, the unit of each content is %. The amino acids are described by one letter codes.

**[Table 12]**

| Table 12: Aggregation Rates of scFvs having Tags Extracted under Extraction Condition 1 Added | | | | | | | | | |
|---|---|---|---|---|---|---|---|---|---|
| Tag Name | Sequence | Aggregation Rate % | DE Rate | N Rate | HKR Rate | CTVL/WM Rate | G Rate | A Rate | FY Rate |
| Tag 18-1(465-1) | DNNESADDNNENPEDNNKNTDDNEENPNNNEN | 4.68 | 37.50 | 43.75 | 3.13 | 3.13 | 0 | 3.13 | 0 |
| Teg 465-2 | GNNQDSSDSDNEADEASDDEDNDGN | 5.04 | 44.00 | 20 | 0 | 0 | 8 | 8 | 0 |
| Teg 465-3 | SHGNNQDSSDSDNEADEASDDEDNDGNEGDNE | 3.98 | 43.75 | 18.75 | 3.13 | 0 | 9.38 | 6.25 | 0 |
| Teg 465-4 | ESADDNNENPEDNNKNTDDNE | 9,99 | 42.86 | 33.33 | 4.76 | 4.76 | 0 | 4.76 | 0 |
| Tag 465-5 | NNENPEDNNKNTDDNEENPNN | 13.94 | 33.33 | 47.62 | 4.76 | 4.76 | 0 | 0 | 0 |
| Tag 465-6 | TDNNESADDNNENPEDNNKN | 8.95 | 35.00 | 40 | 5 | 5 | 0 | 5 | 0 |
| Tag 465-7 | DNNESADDNNENPEDNNKNT | 7.77 | 35.00 | 40 | 5 | 5 | 0 | 5 | 0 |
| Tag 465-8 | NNESADDNNENPEDNNKNTD | 7.39 | 35.00 | 40 | 5 | 5 | 0 | 5 | 0 |
| Tag 465-9 | FWGSHGNNQDSSDSDNEADEASDDEDNDGNE | 7.36 | 38.71 | 16.13 | 3.23 | 3.23 | 9.68 | 6.45 | 3.23 |
| Tag 1121-1 | KPNNSNAPNEDQEEEIQQSE | 14,73 | 30 | 20 | 5 | 5 | 0 | 5 | 0 |
| Tag 1121-2 | NNSNAPNEDQEEEIQQSEQH | 13.76 | 30 | 20 | 5 | 5 | 0 | 5 | 0 |
| Tag 1121-3 | SEGEQQLKPNNSNAPNEDQEEE | 14.15 | 31.82 | 18.18 | 4.55 | 4.55 | 4.55 | 4.55 | 0 |
| Tag 2408-1 | EQLNFSDDDEQGSNSPKENNSEDQ | 7.56 | 33.33 | 16.67 | 4.17 | 4.17 | 4.17 | 0 | 4.17 |
| Tag 2408-2 | EQLNFSDDDEQGSNSPKENNSEDQG | 7.53 | 32.00 | 16 | 4 | 4 | 8 | 0 | 4 |
| Tag 2408-3 | EQLNFSDDDEQGSNSPKENNSEDQGS | 9.42 | 30.77 | 15.38 | 3.85 | 3.85 | 7.69 | 0 | 3.85 |
| Tag 6301-1 | EEKNENDENSLSSSSDSSED | 12.66 | 40.00 | 15 | 5 | 5 | 0 | 0 | 0 |
| Tag 6301-2 | NENDENSLSSSSDSSEDKDE | 8.85 | 40.00 | 15 | 5 | 5 | 0 | 0 | 0 |
| Tag 6626-1 | KETNNSNAQNPSEEEGEGQDE | 12.50 | 33.33 | 19.05 | 4.76 | 4.76 | 9.52 | 4.76 | 0 |
| Tag 6626-2 | ETNNSNAQNPSEEEGEGQDED | 8.04 | 38.10 | 19.05 | 0 | 4.76 | 9.52 | 4.76 | 0 |
| Tag 6626-3 | KETNNSNAQNPSEEEGEGQDED | 13.56 | 36.36 | 18.18 | 4.55 | 4.55 | 9.09 | 4.55 | 0 |
| Tag 6915-1 | ENANDSSDDSGEETDESFNP | 9.52 | 40.00 | 15 | 0 | 5 | 5 | 5 | 5 |
| Tag 7128-1 | DDNESNSESAENGWDSGSNFSEE | 12.10 | 34.78 | 17.39 | 0 | 4.35 | 8.7 | 4.35 | 4.35 |
| Tag 7128-2 | SDDNESNSESAENGWDSGSNFSEE | 8.21 | 33.33 | 16.67 | 0 | 4.17 | 8.33 | 4.17 | 4.17 |
| Tag 7128-3 | SSDDNESNSESAENGWDSGSNFSEE | 8.06 | 32.00 | 16 | 0 | 4 | 8 | 4 | 4 |
| Tag 7315-1 | EENASSGDSEENTNSDHESE | 0.74 | 40.00 | 15 | 5 | 5 | 5 | 5 | 0 |
| Tag 7315-2 | SEENASSGDSEENTNSDHES | 11.72 | 35.00 | 15 | 5 | 5 | 5 | 5 | 0 |
| Tag 7315-3 | ENASSGDSEENTNSDHESEQ | 10.07 | 35.00 | 15 | 5 | 5 | 5 | 5 | 0 |
| Tag 8482-1 | DDDDENSENNWRNEYPEEESSDG | 8.17 | 42.86 | 19.05 | 4.76 | 4.76 | 4.76 | 0 | 4.78 |
| Tag 8482 2 | ENSENNWRNEYPEEESSDGDE | 711 | 42.86 | 19.05 | 4.76 | 4.76 | 4.76 | 0 | 4.78 |
| Tag 8482-3 | NSENNWRNEYPEEESSDGDEDS | 508 | 40.91 | 18.18 | 4.55 | 4.55 | 4.55 | 0 | 4.55 |
| Tag 8974-1 | EQQNEASEENNDQQSQEVPE | 7.84 | 35.00 | 15 | 0 | 5 | 0 | 5 | 0 |
| Tag 8974-2 | QQNEASEENNDQQSQEVPEK | 6.39 | 30.00 | 15 | 5 | 5 | 0 | 5 | 0 |
| Tag 9333-1 | MQEDEFDQGNQEQEDNSNAE | 15.37 | 40.00 | 15 | 0 | 5 | 5 | 5 | 5 |
| Tag 9333-2 | SKMQEDEFDQGNQEQEDNSN | 9.62 | 35.00 | 15 | 5 | 5 | 5 | 0 | 5 |
| Tag 9333-3 | QEDEFDQGNQEQEDNSNAEMEEENASN | 7.13 | 40.74 | 18.52 | 0 | 3.7 | 3.7 | 7.41 | 3.7 |
| Tag 10381-1 | PSENENSQSEDSVGGDNDSEN | 5.59 | 33.33 | 19.05 | 0 | 4.76 | 9.52 | 0 | 0 |
| Tag 11717-1 | EVEESNPSAKEDSNPNSSGE | 10.48 | 30.00 | 15 | 5 | 5 | 5 | 5 | 0 |
| Tag 11717-2 | VEESNPSAKEDSNPNSSGED | 12.10 | 30.00 | | 5 | 5 | 5 | 5 | 0 |
| Tag 12237-1 | KEENSESPLNENSDESYSEE | 9.98 | 40.00 | 15 | 5 | 5 | 0 | 0 | 5 |
| Tag 12809 1 | QPGPNHEEDADSYENMDNPD | 28.15 | 35.00 | 15 | 5 | 5 | 5 | 5 | 5 |
| Tag 12809-2 | PNHEEDADSYENMDNNPDGFD | 20.94 | 40.00 | 15 | 5 | 5 | 5 | 5 | 5 |
| Tag 12809-3 | NHEEDADSYENMDNPDGPDP | 23.41 | 40.00 | 15 | 5 | 5 | 5 | 5 | 5 |
| Tag 12885-1 | DDPNSSDESNGNDDANSESD | 14.62 | 40.00 | 20 | 0 | 0 | 5 | 5 | 0 |
| Tag 12885-2 | NSSDESNGNDDANSESDNNS | 17.00 | 30.00 | 30 | 0 | 0 | 5 | 5 | 0 |
| Tag 12885-3 | DESNGNDDANSESDNNSSSRGD | 13.93 | 31.82 | 22.73 | 4.55 | 0 | 9.09 | 5 | 0 |
| Tag 12968-1 | DNNENAGEDGDNDFSPSDEEL | 13.42 | 42.86 | 19.05 | 0 | 4.76 | 9.52 | 4.55 | 0 |
| Tag 12968-2 | AELEEDDNNENAGEDGDNDFSPS | 10.18 | 43.48 | 17.39 | 0 | 4.35 | 8.7 | 8.7 | 4.76 |
| Tag 12968-3 | DNNENAGEDGDNDFSPSDEELAN | 14.63 | 39.13 | 21.74 | 0 | 4.35 | 8.7 | 8.7 | 4.35 |
| Tag 13648-1 | NPADDPNNQGEDEFEEAEQVREEN | 15.03 | 41.67 | 16.67 | 4.17 | 4.35 | 4.17 | 8.7 | 4.17 |
| Tag 14056-1 | NEENTEPGAESSENADDPNKD | 13.22 | | 19.05 | 4.76 | 4.76 | 4.76 | | 0 |
| Tag 14056-2 | SSENADDPNKDTSENADGQSDEN | 11.45 | 34.78 | 17.39 | 4.35 | 4.35 | 4.35 | 8.7 | 0 |
| Tag 14056-3 | ESSENADDPNKDTSENADGQSDEN | 10.38 | 37.50 | 16.67 | 4.17 | 4.17 | 4.17 | 8.33 | 0 |
| Tag 14681-1 | DRDPEMENEEQPSSENDSQN | 5.63 | 40.00 | 15 | 5 | 5 | 0 | 0 | 0 |
| Tag 14681-2 | PEMENEEQPSSENDSQNQSG | 11.50 | 30.00 | 15 | 0 | 5 | 5 | 0 | 0 |
| Tag 14681-3 | ENEEQPSSENDSQNQSGEQI | 17.15 | 30.00 | 15 | 0 | 5 | 5 | 0 | 0 |
| Tag 14844-1 | DSESANVSDKEAGSNENDDON | 12.13 | 33.33 | 19.05 | 0 | 4.76 | 5 | 0 | 0 |
| Tag 15481-1 | NYNDGSQEDRDWQDDQSDNQ | 9.92 | 35.00 | 15 | 4.76 5 | 5 | 4.76 5 | 9.52 | 0 5 |
| Tag 16043-1 | RENTNEASSEGNSSDDSEDE | 13.45 | 40.00 | 15 | 5 | 5 | 5 | 5 | 0 |
| Tag 16043-2 | ENTNEASSEGNSSDDSEDER | 11.56 | 40.00 | 15 | 5 | 5 | | 5 | 0 |
| Tag 16400-1 | QENDNGNETESEQPKESNENQ | 15.9 | 33.33 | 23.81 | 4.76 | 4.76 | 4.76 | 0 | 0 |
| Tag 16400-2 | ENDNGNETESEQPKESNENQE | 18.1 | 38.1 | 23.81 | 4.76 | 4.76 | 4.76 | 0 | 0 |
| Tag 16400-3 | QENDNGNETESEQPKESNENQE | 8.84 | 36.36 | 22.73 | 4.55 | 4.55 | 4.55 | 0 | 0 |
| Tag 16417-1 | QNEENPGDEEAKNQVNSESDSDSEE | 12.18 | 40.00 | 16 | 4 | 4 | 4 | 4 | 0 |
| Tag 16417-2 | NEENPGDEEAKNQVNSESDSDSEES | 11.00 | 40.00 | 16 | 4 | 4 | 4 | 4 | 0 |
| Tag 16417-3 | QNEENPGDEEAKNQVNSESDSDSEES | 12.37 | 38.46 | 15.38 | 3.85 | 3.85 | 3.85 | 3.85 | 0 |
| Tag 18137-1 | YNGGNANPRPANNEEEEDEEDE | 7.47 | 40.91 | 22.73 | 4.55 | 0 | 9.09 | 9.09 | 4.55 |
| Tag 18137-2 | NGGNANPRPANNEEEEDEEDEY | 8.17 | 40.91 | 22.73 | 4.55 | 0 | 9.09 | 9.09 | 4.55 |
| Tag 18137-3 | NGGNANPRPANNEEEEDEEDEYD | 9.86 | 43.48 | 21.74 | 4.35 | 0 | 8.7 | 8.7 | 4.35 |
| Tag 18347-1 | GASENEEEDDDYNKPLDPNS | 16.06 | 40.00 | 15 | 5 | 5 | 5 | 5 | 5 |
| Tag 18478-1 | LQNQKEAEEPGPDSENSQEN | 16.82 | 30.00 | 15 | 5 | 5 | 5 | 5 | 0 |
| Tag 18478-2 | QNQKEAEEPGPDSENSQENP | 14.77 | 30.00 | 15 | 5 | 0 | 5 | 5 | 0 |
| Tag 18478-3 | NQKEAEEPGPDSENSQENPP | 12.74 | 30.00 | 15 | 5 | 0 | 5 | 5 | 0 |
| Tag 20166-1 | KESVSPENNEEGGNDNQDNEN | 18.95 | 33.33 | 28.57 | 4.76 | 4.76 | 9.52 | 0 | 0 |
| Tag 20166 2 | ESVSPENNEEGGNDNQDNENP | 13.67 | 33.33 | 28.57 | 0 | 4.76 | 9.52 | 0 | 0 |
| Tag 20166-3 | KESVSPENNEEGGNDNODNENP | 26.56 | 31.82 | 27.27 | 4.55 | 4.55 | 9.09 | 0 | 0 |
| Tag 41693-1 | ASPQPREPSDDENSDNSNEC | 7.83 | 30.00 | 15 | 5 | 5 | 0 | 5 | 0 |
| Tag 55443-1 | NNSQDEDGFQELNENGNAKDE | 19.76 | 33.33 | 23.81 | 4.76 | 4.76 | 9.52 | 4.76 | 4.76 |
| Tag 55443-2 | NSQDEDGFQELNENGNAKDEN | 22.25 | 33.33 | 23.81 | 4.76 | 4.76 | 9.52 | 4.76 | 4.76 |
| Tag 55443-3 | NNSQDEDGFQELNENGNAKDEN | 18.69 | 31.82 | 27.27 | 4.55 | 4.55 | 9.09 | 4.55 | 4.55 |

In Table 12, the N content ratio in the amino acid sequences of the extracted tags is 15% or more, and the content ratios of the other amino acids are as described above in Extraction Condition 1. In order to confirm that the aggregation inhibiting action does not depend on a specific amino acid sequence, tags were randomly selected from the tags extracted under Extraction Condition 1. As for some tags, an amino acid sequence satisfying the extraction condition was additionally selected from another portion of the same protein. The aggregation rates of scFvs tagged with the selected amino acid sequences were tested, and results as shown in Table 12 were obtained. As shown in Table 12, the aggregation rates of the tagged scFvs were low as a whole. Accordingly, it is obvious that the aggregation inhibiting action of a peptide tag largely depends on the amino acid content ratios thereof, and depends merely weakly on the specific amino acid sequence itself.

It is noted that human-derived amino acid sequences that can be extracted under Extraction Condition 1 were as follows.

### [Table 12-2]

**Table 12-2: Examples of human-derived amino acid sequences that can be extracted under Extraction Condition 1**

| SEQ ID NO: | Sequence |
|---|---|
| 105 | TDNNESADDNNENPEDNNKN (Tag465-6) |
| 106 | DNNESADDNNENPEDNNKNT (Tag465-7) |
| 107 | NNESADDNNENPEDNNKNTD (Tag465-8) |
| 108 | NESADDNNENPEDNNKNTDD |
| 109 | ESADDNNENPEDNNKNTDDN |
| 110 | SADDNNENPEDNNKNTDDNE |
| 111 | DNNENPEDNNKNTDDNEENP |
| 112 | NNENPEDNNKNTDDNEENPN |
| 113 | NENPEDNNKNTDDNEENPNN |
| 114 | ENPEDNNKNTDDNEENPNNN |
| 115 | NPEDNNKNTDDNEENPNNNE |
| 116 | PEDNNKNTDDNEENPNNNEN |
| 117 | DNNESADDNNENPEDNNKNTD |
| 118 | NNESADDNNENPEDNNKNTDD |
| 119 | NESADDNNENPEDNNKNTDDN |
| 120 | ESADDNNENPEDNNKNTDDNE (Tag 465-4) |
| 121 | SADDNNENPEDNNKNTDDNEE |
| 122 | ADDNNENPEDNNKNTDDNEEN |
| 123 | DDNNENPEDNNKNTDDNEENP |
| 124 | DNNENPEDNNKNTDDNEENPN |
| 125 | NNENPEDNNKNTDDNEENPNN (Tag 465-5) |
| 126 | NENPEDNNKNTDDNEENPNNN |
| 127 | ENPEDNNKNTDDNEENPNNNE |
| 128 | NPEDNNKNTDDNEENPNNNEN |
| 129 | DNNESADDNNENPEDNNKNTDD |
| 130 | NNESADDNNENPEDNNKNTDDN |
| 131 | NESADDNNENPEDNNKNTDDNE |
| 132 | SADDNNENPEDNNKNTDDNEEN |
| 133 | ADDNNENPEDNNKNTDDNEENP |
| 134 | DDNNENPEDNNKNTDDNEENPN |
| 135 | DNNENPEDNNKNTDDNEENPNN |
| 136 | NNENPEDNNKNTDDNEENPNNN |
| 137 | NENPEDNNKNTDDNEENPNNNE |
| 138 | ENPEDNNKNTDDNEENPNNNEN |
| 139 | DNNESADDNNENPEDNNKNTDDN |
| 140 | NNESADDNNENPEDNNKNTDDNE |
| 141 | NESADDNNENPEDNNKNTDDNEE |
| 142 | ESADDNNENPEDNNKNTDDNEEN |
| 143 | SADDNNENPEDNNKNTDDNEENP |
| 144 | ADDNNENPEDNNKNTDDNEENPN |
| 145 | DDNNENPEDNNKNTDDNEENPNN |
| 146 | DNNENPEDNNKNTDDNEENPNNN |
| 147 | NNENPEDNNKNTDDNEENPNNNE |
| 148 | NENPEDNNKNTDDNEENPNNNEN |
| 149 | HGNNQDSSDSDNEADEASDDEDN |
| 150 | DNNESADDNNENPEDNNKNTDDNE |
| 151 | NNESADDNNENPEDNNKNTDDNEE |
| 152 | NESADDNNENPEDNNKNTDDNEEN |
| 153 | ESADDNNENPEDNNKNTDDNEENP |
| 154 | SADDNNENPEDNNKNTDDNEENPN |
| 155 | ADDNNENPEDNNKNTDDNEENPNN |
| 156 | DDNNENPEDNNKNTDDNEENPNNN |
| 157 | DNNENPEDNNKNTDDNEENPNNNE |
| 158 | NNENPEDNNKNTDDNEENPNNNEN |
| 159 | SHGNNQDSSDSDNEADEASDDEDN |
| 160 | DNNESADDNNENPEDNNKNTDDNEE |
| 161 | NNESADDNNENPEDNNKNTDDNEEN |
| 162 | NESADDNNENPEDNNKNTDDNEENP |
| 163 | ESADDNNENPEDNNKNTDDNEENPN |
| 164 | SADDNNENPEDNNKNTDDNEENPNN |
| 165 | ADDNNENPEDNNKNTDDNEENPNNN |
| 166 | DDNNENPEDNNKNTDDNEENPNNNE |
| 167 | DNNENPEDNNKNTDDNEENPNNNEN |
| 168 | GSHGNNQDSSDSDNEADEASDDEDN |
| 169 | SHGNNQDSSDSDNEADEASDDEDND |
| 170 | HGNNQDSSDSDNEADEASDDEDNDG |
| 171 | GNNQDSSDSDNEADEASDDEDNDGN (Tag 465-2) |
| 172 | DNNESADDNNENPEDNNKNTDDNEEN |
| 173 | NNESADDNNENPEDNNKNTDDNEENP |
| 174 | NESADDNNENPEDNNKNTDDNEENPN |
| 175 | ESADDNNENPEDNNKNTDDNEENPNN |
| 176 | SADDNNENPEDNNKNTDDNEENPNNN |
| 177 | ADDNNENPEDNNKNTDDNEENPNNNE |
| 178 | DDNNENPEDNNKNTDDNEENPNNNEN |
| 179 | WGSHGNNQDSSDSDNEADEASDDEDN |
| 180 | GSHGNNQDSSDSDNEADEASDDEDND |
| 181 | SHGNNQDSSDSDNEADEASDDEDNDG |
| 182 | HGNNQDSSDSDNEADEASDDEDNDGN |
| 183 | DNNESADDNNENPEDNNKNTDDNEENP |
| 184 | NNESADDNNENPEDNNKNTDDNEENPN |
| 185 | NESADDNNENPEDNNKNTDDNEENPNN |
| 186 | ESADDNNENPEDNNKNTDDNEENPNNN |
| 187 | SADDNNENPEDNNKNTDDNEENPNNNE |
| 188 | ADDNNENPEDNNKNTDDNEENPNNNEN |
| 189 | SHGNNQDSSDSDNEADEASDDEDNDGN |
| 190 | HGNNQDSSDSDNEADEASDDEDNDGNE |
| 191 | DNNESADDNNENPEDNNKNTDDNEENPN |
| 192 | NNESADDNNENPEDNNKNTDDNEENPNN |
| 193 | NESADDNNENPEDNNKNTDDNEENPNNN |
| 194 | ESADDNNENPEDNNKNTDDNEENPNNNE |
| 195 | SADDNNENPEDNNKNTDDNEENPNNNEN |
| 196 | SHGNNQDSSDSDNEADEASDDEDNDGNE |
| 197 | DNNESADDNNENPEDNNKNTDDNEENPN N |
| 198 | NNESADDNNENPEDNNKNTDDNEENPNN N |
| 199 | NESADDNNENPEDNNKNTDDNEENPNNN E |
| 200 | ESADDNNENPEDNNKNTDDNEENPNNNE N |
| 201 | DNNESADDNNENPEDNNKNTDDNEENPN NN |
| 202 | NNESADDNNENPEDNNKNTDDNEENPNN NE |
| 203 | NESADDNNENPEDNNKNTDDNEENPNNN EN |
| 204 | DNNESADDNNENPEDNNKNTDDNEENPN NNE |
| 205 | NNESADDNNENPEDNNKNTDDNEENPNN NEN |
| 206 | FWGSHGNNQDSSDSDNEADEASDDEDN DGNE (Tag465-9) |
| 207 | SHGNNQDSSDSDNEADEASDDEDNDGNE GDN |
| 208 | DNNESADDNNENPEDNNKNTDDNEENPN NNEN (18-1) |
| 209 | SHGNNQDSSDSDNEADEASDDEDNDGNE GDNE (Tag 465-3) |
| 210 | LEDNNEEPRDPQSPPDPPNE (Tag656-1) |
| 211 | EDNNEEPRDPQSPPDPPNEF (Tag656-2) |
| 212 | DNNEEPRDPQSPPDPPNEFG (Tag656-3) |
| 213 | EGEQQLKPNNSNAPNEDQEE |
| 214 | GEQQLKPNNSNAPNEDQEEE |
| 215 | KPNNSNAPNEDQEEEIQQSE (Tag1121-1) |
| 216 | PNNSNAPNEDQEEEIQQSEQ |
| 217 | NNSNAPNEDQEEEIQQSEQH (Tag1121-2) |
| 218 | EGEQQLKPNNSNAPNEDQEEE |
| 219 | SEGEQQLKPNNSNAPNEDQEEE (Tag1121-3) |
| 220 | EQLNFSDDDEQGSNSPKENN |
| 221 | LNFSDDDEQGSNSPKENNSE |
| 222 | NFSDDDEQGSNSPKENNSED |
| 223 | FSDDDEQGSNSPKENNSEDQ |
| 224 | LNFSDDDEQGSNSPKENNSED |
| 225 | NFSDDDEQGSNSPKENNSEDQ |
| 226 | EQLNFSDDDEQGSNSPKENNSE |
| 227 | QLNFSDDDEQGSNSPKENNSED |
| 228 | LNFSDDDEQGSNSPKENNSEDQ |
| 229 | NFSDDDEQGSNSPKENNSEDQG |
| 230 | EQLNFSDDDEQGSNSPKENNSED |
| 231 | QLNFSDDDEQGSNSPKENNSEDQ |
| 232 | LNFSDDDEQGSNSPKENNSEDQG |
| 233 | NFSDDDEQGSNSPKENNSEDQGS |
| 234 | EQLNFSDDDEQGSNSPKENNSEDQ (Tag2408-1) |
| 235 | EQLNFSDDDEQGSNSPKENNSEDQG (Tag2408-2) |
| 236 | EQLNFSDDDEQGSNSPKENNSEDQGS (Tag2408-3) |
| 237 | EEKNENDENSLSSSSDSSED (Tag6301-1) |
| 238 | NENDENSLSSSSDSSEDKDE (Tag6301-1) |
| 239 | KETNNSNAQNPSEEEGEGQDE (Tag6626-1) |
| 240 | ETNNSNAQNPSEEEGEGQDED (Tag6626-2) |
| 241 | KETNNSNAQNPSEEEGEGQDED (Tag6626-3) |
| 242 | ENANDSSDDSGEETDESFNP (Tag6915-1) |
| 243 | DDNESNSESAENGWDSGSNFSE |
| 244 | DNESNSESAENGWDSGSNFSEE |
| 245 | SDDNESNSESAENGWDSGSNFSE |
| 246 | DDNESNSESAENGWDSGSNFSEE (Tag7128-1) |
| 247 | SDDNESNSESAENGWDSGSNFSEE (Tag7128-2) |
| 248 | SSDNESSESAENGWDSGSNFSEE (Tag7128-3) |
| 249 | SEENASSGDSEENTNSDHES (Tag7135-2) |
| 250 | EENASSGDSEENTNSDHESE (Tag 7315-1) |
| 251 | ENASSGDSEENTNSDHESEQ (Tag7135-3) |
| 252 | DENSENNWRNEYPEEESSDG |
| 253 | ENSENNWRNEYPEEESSDGD |
| 254 | NSENNWRNEYPEEESSDGDE |
| 255 | DDENSENNWRNEYPEEESSDG (Tag8482-1) |
| 256 | DENSENNWRNEYPEEESSDGD |
| 257 | ENSENNWRNEYPEEESSDGDE (Tag8482-2) |
| 258 | NSENNWRNEYPEEESSDGDED |
| 259 | NSENNWRNEYPEEESSDGDEDS (Tag8482-3) |
| 260 | ENSENNWRNEYPEEESSDGDEDS |
| 261 | EQQNEASEENNDQQSQEVPE (Tag8974-1) |
| 262 | QQNEASEENNDQQSQEVPEK (Tag8974-2) |
| 263 | SKMQEDEFDQGNQEQEDNSN (Tag9333-2) |
| 264 | KMQEDEFDQGNQEQEDNSNA |
| 265 | MQEDEFDQGNQEQEDNSNAE (Tag 9333-1) |
| 266 | QEDEFDQGNQEQEDNSNAEM |
| 267 | FDQGNQEQEDNSNAEMEEEN |
| 268 | EFDQGNQEQEDNSNAEMEEEN |
| 269 | FDQGNQEQEDNSNAEMEEENA |
| 270 | DQGNQEQEDNSNAEMEEENAS |
| 271 | QGNQEQEDNSNAEMEEENASN |
| 272 | EFDQGNQEQEDNSNAEMEEENA |
| 273 | FDQGNQEQEDNSNAEMEEENAS |
| 274 | DQGNQEQEDNSNAEMEEENASN |
| 275 | DEFDQGNQEQEDNSNAEMEEENA |
| 276 | EFDQGNQEQEDNSNAEMEEENAS |
| 277 | FDQGNQEQEDNSNAEMEEENASN |
| 278 | DEFDQGNQEQEDNSNAEMEEENAS |
| 279 | EFDQGNQEQEDNSNAEMEEENASN |
| 280 | QEDEFDQGNQEQEDNSNAEMEEENA |
| 281 | EDEFDQGNQEQEDNSNAEMEEENAS |
| 282 | DEFDQGNQEQEDNSNAEMEEENASN |
| 283 | QEDEFDQGNQEQEDNSNAEMEEENAS |
| 284 | EDEFDQGNQEQEDNSNAEMEEENASN |
| 285 | QEDEFDQGNQEQEDNSNAEMEEENASN (Tag9333-3) |
| 286 | PSENENSQSEDSVGGDNDSEN (Tag10381-1) |
| 287 | EVEESNPSAKEDSNPNSSGE (Tag11717-1) |
| 288 | VEESNPSAKEDSNPNSSGED (Tag11717-2) |
| 289 | KEENSESPLNENSDESYSEE (Tag12237-1) |
| 290 | QPGPNHEEDADSYENMDNPD (Tag12809-1) |
| 291 | PNHEEDADSYENMDNPDGPD (Tag12809-2) |
| 292 | NHEEDADSYENMDNPDGPDP (Tag12809-3) |
| 293 | DDPNSSDESNGNDDANSESD (Tag 12885-1) |
| 294 | DPNSSDESNGNDDANSESDN |
| 295 | PNSSDESNGNDDANSESDNN |
| 296 | NSSDESNGNDDANSESDNNS (Tag 12885-2) |
| 297 | SSDESNGNDDANSESDNNSS |
| 298 | SDESNGNDDANSESDNNSSS |
| 299 | DESNGNDDANSESDNNSSSR |
| 300 | MQGDDPNSSDESNGNDDANSE |
| 301 | QGDDPNSSDESNGNDDANSES |
| 302 | GDDPNSSDESNGNDDANSESD |
| 303 | DDPNSSDESNGNDDANSESDN |
| 304 | DPNSSDESNGNDDANSESDNN |
| 305 | DKSMQGDDPNSSDESNGNDDAN |
| 306 | SMQGDDPNSSDESNGNDDANSE |
| 307 | MQGDDPNSSDESNGNDDANSES |
| 308 | QGDDPNSSDESNGNDDANSESD |
| 309 | GDDPNSSDESNGNDDANSESDN |
| 310 | DDPNSSDESNGNDDANSESDNN |
| 311 | DPNSSDESNGNDDANSESDNNS |
| 312 | DESNGNDDANSESDNNSSSRGD (Tag12885-3) |
| 313 | DDKSMQGDDPNSSDESNGNDDAN |
| 314 | DKSMQGDDPNSSDESNGNDDANS |
| 315 | KSMQGDDPNSSDESNGNDDANSE |
| 316 | SMQGDDPNSSDESNGNDDANSES |
| 317 | MQGDDPNSSDESNGNDDANSESD |
| 318 | QGDDPNSSDESNGNDDANSESDN |
| 319 | GDDPNSSDESNGNDDANSESDNN |
| 320 | DDPNSSDESNGNDDANSESDNNS |
| 321 | DPNSSDESNGNDDANSESDNNSS |
| 322 | SDESNGNDDANSESDNNSSSRGD |
| 323 | DESNGNDDANSESDNNSSSRGDA |
| 324 | DDANSESDNNSSSRGDASYNSDE |
| 325 | FDDKSMQGDDPNSSDESNGNDDAN |
| 326 | DDKSMQGDDPNSSDESNGNDDANS |
| 327 | DKSMQGDDPNSSDESNGNDDANSE |
| 328 | SMQGDDPNSSDESNGNDDANSESD |
| 329 | MQGDDPNSSDESNGNDDANSESDN |
| 330 | QGDDPNSSDESNGNDDANSESDNN |
| 331 | GDDPNSSDESNGNDDANSESDNNS |
| 332 | DDPNSSDESNGNDDANSESDNNSS |
| 333 | DFDDKSMQGDDPNSSDESNGNDDAN |
| 334 | FDDKSMQGDDPNSSDESNGNDDANS |
| 335 | DDKSMQGDDPNSSDESNGNDDANSE |
| 336 | DKSMQGDDPNSSDESNGNDDANSES |
| 337 | KSMQGDDPNSSDESNGNDDANSESD |
| 338 | SMQGDDPNSSDESNGNDDANSESDN |
| 339 | MQGDDPNSSDESNGNDDANSESDNN |
| 340 | QGDDPNSSDESNGNDDANSESDNNS |
| 341 | GDDPNSSDESNGNDDANSESDNNSS |
| 342 | DDPNSSDESNGNDDANSESDNNSSS |
| 343 | DFDDKSMQGDDPNSSDESNGNDDANS |
| 344 | FDDKSMQGDDPNSSDESNGNDDANSE |
| 345 | DDKSMQGDDPNSSDESNGNDDANSES |
| 346 | DKSMQGDDPNSSDESNGNDDANSESD |
| 347 | KSMQGDDPNSSDESNGNDDANSESDN |
| 348 | SMQGDDPNSSDESNGNDDANSESDNN |
| 349 | MQGDDPNSSDESNGNDDANSESDNNS |
| 350 | QGDDPNSSDESNGNDDANSESDNNSS |
| 351 | GDDPNSSDESNGNDDANSESDNNSSS |
| 352 | DDPNSSDESNGNDDANSESDNNSSSR |
| 353 | DKSMQGDDPNSSDESNGNDDANSESDN |
| 354 | DDKSMQGDDPNSSDESNGNDDANSESD N |
| 355 | DKSMQGDDPNSSDESNGNDDANSESDN N |
| 356 | DDPNSSDESNGNDDANSESDNNSSSRGD |
| 357 | FDDKSMQGDDPNSSDESNGNDDANSESD N |
| 358 | DDKSMQGDDPNSSDESNGNDDANSESD NN |
| 359 | DKSMQGDDPNSSDESNGNDDANSESDN NS |
| 360 | DDPNSSDESNGNDDANSESDNNSSSRGD A |
| 361 | DESNGNDDANSESDNNSSSRGDASYNSD E |
| 362 | DFDDKSMQGDDPNSSDESNGNDDANSES DN |
| 363 | FDDKSMQGDDPNSSDESNGNDDANSESD NN |
| 364 | DDKSMQGDDPNSSDESNGNDDANSESD NNS |
| 365 | DKSMQGDDPNSSDESNGNDDANSESDN NSS |
| 366 | DDPNSSDESNGNDDANSESDNNSSSRGD AS |
| 367 | SDESNGNDDANSESDNNSSSRGDASYNS DE |
| 368 | DESNGNDDANSESDNNSSSRGDASYNSD ES |
| 369 | DFDDKSMQGDDPNSSDESNGNDDANSES DNN |
| 370 | FDDKSMQGDDPNSSDESNGNDDANSESD NNS |
| 371 | DDKSMQGDDPNSSDESNGNDDANSESD NNSS |
| 372 | DFDDKSMQGDDPNSSDESNGNDDANSES DNNS |
| 373 | FDDKSMQGDDPNSSDESNGNDDANSESD NNSS |
| 374 | DDKSMQGDDPNSSDESNGNDDANSESD NNSSS |
| 375 | DFDDKSMQGDDPNSSDESNGNDDANSES DNNSS |
| 376 | FDDKSMQGDDPNSSDESNGNDDANSESD NNSSS |
| 377 | DFDDKSMQGDDPNSSDESNGNDDANSES DNNSSS |
| 378 | DDPNSSDESNGNDDANSESDNNSSSRGD ASYNSDE |
| 379 | GDDPNSSDESNGNDDANSESDNNSSSRG DASYNSDE |
| 380 | DDPNSSDESNGNDDANSESDNNSSSRGD ASYNSDES |
| 381 | DSYDFDDKSMQGDDPNSSDESNGNDDA NSESDNNSSSRGD |
| 382 | SYDFDDKSMQGDDPNSSDESNGNDDANS ESDNNSSSRGDA |
| 383 | YDFDDKSMQGDDPNSSDESNGNDDANSE SDNNSSSRGDAS |
| 384 | DFDDKSMQGDDPNSSDESNGNDDANSES DNNSSSRGDASY |
| 385 | QGDDPNSSDESNGNDDANSESDNNSSSR GDASYNSDESKD |
| 386 | GDDPNSSDESNGNDDANSESDNNSSSRG DASYNSDESKDN |
| 387 | DDPNSSDESNGNDDANSESDNNSSSRGD ASYNSDESKDNG |
| 388 | DDKSMQGDDPNSSDESNGNDDANSESD NNSSSRGDASYNSDE |
| 389 | DFDDKSMQGDDPNSSDESNGNDDANSES DNNSSSRGDASYNSD |
| 390 | FDDKSMQGDDPNSSDESNGNDDANSESD NNSSSRGDASYNSDE |
| 391 | DDKSMQGDDPNSSDESNGNDDANSESD NNSSSRGDASYNSDES |
| 392 | DFDDKSMQGDDPNSSDESNGNDDANSES DNNSSSRGDASYNSDE |
| 393 | DFDDKSMQGDDPNSSDESNGNDDANSES DNNSSSRGDASYNSDES |
| 394 | DDPNSSDESNGNDDANSESDNNSSSRGD ASYNSDESKDNGNGSDSKGAEDDDSDST SDTN |
| 395 | DPNSSDESNGNDDANSESDNNSSSRGDA SYNSDESKDNGNGSDSKGAEDDDSDSTS DTNN |
| 396 | NSSDESNGNDDANSESDNNSSSRGDASY NSDESKDNGNGSDSKGAEDDDSDSTSDT NNSD |
| 397 | SSDESNGNDDANSESDNNSSSRGDASYN SDESKDNGNGSDSKGAEDDDSDSTSDTN NSDS |
| 398 | SDESNGNDDANSESDNNSSSRGDASYNS DESKDNGNGSDSKGAEDDDSDSTSDTNN SDSN |
| 399 | AEDDDSDSTSDTNNSDSNGNGNNGNDDN DKSDSGKGKSDSSDSDSSDSSNSSDSSD SSDS |
| 400 | EDDDSDSTSDTNNSDSNGNGNNGNDDN DKSDSGKGKSDSSDSDSSDSSNSSDSSD SSDSD |
| 401 | DDDSDSTSDTNNSDSNGNGNNGNDDND KSDSGKGKSDSSDSDSSDSSNSSDSSDS SDSDS |
| 402 | GDDPNSSDESNGNDDANSESDNNSSSRG DASYNSDESKDNGNGSDSKGAEDDDSDS TSDTN |
| 403 | DDPNSSDESNGNDDANSESDNNSSSRGD ASYNSDESKDNGNGSDSKGAEDDDSDST SDTNN |
| 404 | QGDDPNSSDESNGNDDANSESDNNSSSR GDASYNSDESKDNGNGSDSKGAEDDDSD STSDTN |
| 405 | GDDPNSSDESNGNDDANSESDNNSSSRG DASYNSDESKDNGNGSDSKGAEDDDSDS TSDTNN |
| 406 | DDPNSSDESNGNDDANSESDNNSSSRGD ASYNSDESKDNGNGSDSKGAEDDDSDST SDTNNS |
| 407 | DPNSSDESNGNDDANSESDNNSSSRGDA SYNSDESKDNGNGSDSKGAEDDDSDSTS DTNNSD |
| 408 | MQGDDPNSSDESNGNDDANSESDNNSS SRGDASYNSDESKDNGNGSDSKGAEDDD SDSTSDTN |
| 409 | QGDDPNSSDESNGNDDANSESDNNSSSR GDASYNSDESKDNGNGSDSKGAEDDDSD STSDTNN |
| 410 | GDDPNSSDESNGNDDANSESDNNSSSRG DASYNSDESKDNGNGSDSKGAEDDDSDS TSDTNNS |
| 411 | DDPNSSDESNGNDDANSESDNNSSSRGD ASYNSDESKDNGNGSDSKGAEDDDSDST SDTNNSD |
| 412 | DPNSSDESNGNDDANSESDNNSSSRGDA SYNSDESKDNGNGSDSKGAEDDDSDSTS DTNNSDS |
| 413 | [?] GDDPNSSDESNGNDDANSESDNNSSSRG DASYNSDESKDNGNGSDSKGAEDDDSDS TSDTNNSD |
| 414 | DDPNSSDESNGNDDANSESDNNSSSRGD ASYNSDESKDNGNGSDSKGAEDDDSDST SDTNNSDS |
| 415 | QGDDPNSSDESNGNDDANSESDNNSSSR GDASYNSDESKDNGNGSDSKGAEDDDSD STSDTNNSD |
| 416 | GDDPNSSDESNGNDDANSESDNNSSSRG DASYNSDESKDNGNGSDSKGAEDDDSDS TSDTNNSDS |
| 417 | DDPNSSDESNGNDDANSESDNNSSSRGD ASYNSDESKDNGNGSDSKGAEDDDSDST SDTNNSDSN |
| 418 | EDDDSDSTSDTNNSDSNGNGNNGNDDN DKSDSGKGKSDSSDSDSSDSSNSSDSSD SSDSDSSDSN |
| 419 | MQGDDPNSSDESNGNDDANSESDNNSS SRGDASYNSDESKDNGNGSDSKGAEDDD SDSTSDTNNSD |
| 420 | QGDDPNSSDESNGNDDANSESDNNSSSR GDASYNSDESKDNGNGSDSKGAEDDDSD STSDTNNSDS |
| 421 | GDDPNSSDESNGNDDANSESDNNSSSRG DASYNSDESKDNGNGSDSKGAEDDDSDS TSDTNNSDSN |
| 422 | DDPNSSDESNGNDDANSESDNNSSSRGD ASYNSDESKDNGNGSDSKGAEDDDSDST SDTNNSDSNG |
| 423 | AEDDDSDSTSDTNNSDSNGNGNNGNDDN DKSDSGKGKSDSSDSDSSDSSNSSDSSD SSDSDSSDSN |
| 424 | EDDDSDSTSDTNNSDSNGNGNNGNDDN DKSDSGKGKSDSSDSDSSDSSNSSDSSD SSDSDSSDSNS |
| 425 | LEEDDNNENAGEDGDNDFSPS |
| 426 | DNNENAGEDGDNDFSPSDEEL (Tag 12968-1) |
| 427 | NNENAGEDGDNDFSPSDEELA |
| 428 | NENAGEDGDNDFSPSDEELAN |
| 429 | DNNENAGEDGDNDFSPSDEELA |
| 430 | NNENAGEDGDNDFSPSDEELAN |
| 431 | AELEEDDNNENAGEDGDNDFSPS (Tag 12968-2) |
| 432 | DDNNENAGEDGDNDFSPSDEELA |
| 433 | DNNENAGEDGDNDFSPSDEELAN (Tag 12968-3) |
| 434 | DDNNENAGEDGDNDFSPSDEELAN |
| 435 | EDDNNENAGEDGDNDFSPSDEELAN |
| 436 | NPADDPNNQGEDEFEEAEQVREEN (Tag 13648-1) |
| 437 | NEENTEPGAESSENADDPNKD (Tag 14056-1) |
| 438 | ENADDPNKDTSENADGQSDEN |
| 439 | SENADDPN KDTSENADGQSDEN |
| 440 | SSENADDPNKDTSENADGQSDEN (Tag 14056-2) |
| 441 | ESSENADDPNKDTSENADGQSDEN (Tag 14056-3) |
| 442 | DRDPEMENEEQPSSENDSQN (Tag 14681-1) |
| 443 | RDPEMENEEQPSSENDSQNQ |
| 444 | DPEMENEEQPSSENDSQNQS |
| 445 | PEMENEEQPSSENDSQNQSG (Tag 14681-2) |
| 446 | EMENEEQPSSENDSQNQSGE |
| 447 | MENEEQPSSENDSQNQSGEQ |
| 448 | ENEEQPSSENDSQNQSGEQI (Tag 14681-3) |
| 449 | DSESANVSDKEAGSNENDDQN (Tag 14844-1) |
| 450 | NYNDGSQEDRDWQDDQSDNQ (Tag 15481-1) |
| 451 | RENTNEASSEGNSSDDSEDE (Tag 16043-1) |
| 452 | ENTNEASSEGNSSDDSEDER (Tag 16043-2) |
| 453 | QENDNGNETESEQPKESNEN |
| 454 | ENDNGNETESEQPKESNENQ |
| 455 | NDNGNETESEQPKESNENQE |
| 456 | QENDNGNETESEQPKESNENQ (Tag 16400-1) |
| 457 | ENDNGNETESEQPKESNENQE (Tag 16400-2) |
| 458 | QENDNGNETESEQPKESNENQE (Tag 16400-3) |
| 459 | QNEENPGDEEAKNQVNSESD |
| 460 | NEENPGDEEAKNQVNSESDS |
| 461 | EENPGDEEAKNQVNSESDSD |
| 462 | ENPGDEEAKNQVNSESDSDS |
| 463 | NPGDEEAKNQVNSESDSDSE |
| 464 | QNEENPGDEEAKNQVNSESDS |
| 465 | NEENPGDEEAKNQVNSESDSD |
| 466 | QNEENPGDEEAKNQVNSESDSD |
| 467 | NEENPGDEEAKNQVNSESDSDS |
| 468 | QNEENPGDEEAKNQVNSESDSDS |
| 469 | NEENPGDEEAKNQVNSESDSDSE |
| 470 | QNEENPGDEEAKNQVNSESDSDSE |
| 471 | NEENPGDEEAKNQVNSESDSDSEE |
| 472 | QNEENPGDEEAKNQVNSESDSDSEE (Tag 16417-1) |
| 473 | NEENPGDEEAKNQVNSESDSDSEES (Tag 16417-2) |
| 474 | QNEENPGDEEAKNQVNSESDSDSEES (Tag 16417-3) |
| 475 | YNGGNANPRPANNEEEEDEED |
| 476 | NGGNANPRPANNEEEEDEEDE |
| 477 | GGNANPRPANNEEEEDEEDEY |
| 478 | YNGGNANPRPANNEEEEDEEDE (Tag 18137-1) |
| 479 | NGGNANPRPANNEEEEDEEDEY (Tag 18137-2) |
| 480 | NGGNANPRPANNEEEEDEEDEYD (Tag 18137-3) |
| 481 | GASENEEEDDDYNKPLDPNS (Tag 18347-1) |
| 482 | LQNQKEAEEPGPDSENSQEN (Tag 18478-1) |
| 483 | QNQKEAEEPGPDSENSQENP (Tag 18478-2) |
| 484 | NQKEAEEPGPDSENSQENPP (Tag 18478-3) |
| 485 | KESVSPENNEEGGNDNQDNEN (Tag 20166-1) |
| 486 | ESVSPENNEEGGNDNQDNENP (Tag 20166-2) |
| 487 | KESVSPENNEEGGNDNQDNENP (Tag 20166-3) |
| 488 | ASPQPREPSDDENSDNSNEC (Tag 41693-1) |
| 489 | NNSQDEDGFQELNENGNAKDE (Tag 55443-1) |
| 490 | NSQDEDGFQELNENGNAKDEN (Tag 55443-2) |
| 491 | NNSQDEDGFQELNENGNAKDEN (Tag 55443-3) |

### Extraction Condition 2:

length: 20 to 70 amino acids
group [D, E]: content of [30] or more
group [D, E]: content of less than [45]
group [P]: content of [10] or more
group [H, K, R]: content of [5] or less
group [G]: content of less than [10]
group [A]: content of less than [10]
group [C, T, V, L, I, M, W]: content of [0]
group [F, Y]: content of [0]
* In the above-described extraction condition, the unit of each content is %. The amino acids are described by one letter codes.

**[Table 13]**

| Table 13: Aggregation Rates of scFvs having Tags Extracted under Extraction Condition 2 Added | | | | | | | |
|---|---|---|---|---|---|---|---|
| Tag Name | Sequence | Aggregation Rate % | DE Rate | P Rate | H K R Rate | G Rate | A Rate |
| tag47-1 | SSDSPKDQSPPEDSGESEAD | 9.48 | 35 | 15 | 5 | 5 | 5 |
| tag1784-1 | GPEPEPEPEPEPEPAPEPEPE | 15.91 | 42.86 | 47.62 | 0 | 4.76 | 4.76 |
| tag1784-2 | PEPEPEPEPEPEPAPEPEPEP | 14.03 | 42.86 | 52.38 | 0 | 0 | 4.76 |
| tag1784-3 | EPEPEPEPEPEPAPEPEPEPK | 23.67 | 42.86 | 47.62 | 4.76 | 0 | 4.76 |
| tag2257-1 | PEEDGSPDPEPSPEPEPKPS | 22.33 | 35 | 40 | 5 | 5 | 0 |
| tag2257-2 | EDPEEDGSPDPEPSPEPEPKP | 14.76 | 42.86 | 38.1 | 4.76 | 4.76 | 0 |
| tag2257-3 | DPEEDGSPDPEPSPEPEPKPS | 17.21 | 38.1 | 38.1 | 4.76 | 4.76 | 0 |
| tag4398-1 | PDDDGSDDSSPPSASPAESEP | 35.71 | 33.33 | 23.81 | 0 | 4.76 | 9.52 |
| tag4398-2 | DDDGSDDSSPPSASPAESEPQ | 18.64 | 33.33 | 19.05 | 0 | 4.76 | 9.52 |
| taq4398-3 | PDDDGSDDSSPPSASPAESEPQ | 18.58 | 31.82 | 22.73 | 0 | 4.55 | 9.09 |
| tag4898-1 | PPPEEQGQGDAPPQHEDEEPA | 9.9 | 33.33 | 28.57 | 4.76 | 9.52 | 9.52 |
| tag5533-1 | GAPSPAPSPDSDSDSDSDGEE | 19.43 | 33.33 | 19.05 | 0 | 9.52 | 9.52 |
| tag5533-2 | APSPAPSPDSDSDSDSDGEEE | 16.43 | 38.1 | 19.05 | 0 | 4.76 | 9.52 |
| tag5533-3 | PSPAPSPDSDSDSDSDGEEEE | 8.76 | 42.86 | 19.05 | 0 | 4.76 | 4.76 |
| tag5601-1 | PAPPPPPPPEEDPEQDSGPE | 33.03 | 30 | 50 | 0 | 5 | 5 |
| tag5601-2 | APPPPPPPEEDPEQDSGPED | 20.10 | 35 | 45 | 0 | 5 | 5 |
| tag5601-3 | PAPPPPPPPEEDPEQDSGPED | 24.34 | 33.33 | 47.62 | 0 | 4.76 | 4.76 |
| tag6354-1 | PPPPPQAPPEEENESEPEEPS | 7.87 | 33.33 | 42.86 | 0 | 0 | 4.76 |
| tag6354-2 | PPPPQAPPEEENESEPEEPSG | 10.68 | 33.33 | 38.1 | 0 | 4.76 | 4.76 |
| tag6354-3 | PPPPPQAPPEEENESEPEEPSG | 7.98 | 31.82 | 40.91 | 0 | 4.55 | 4.55 |
| tag6681-1 | PQDSSSKSPEPSADESPDND | 8.0/ | 30 | 20 | 5 | 0 | 5 |
| tag7124-1 | QSSDNSEDEEEPPDNADSKS | 5.56 | 40 | 10 | 5 | 0 | 5 |
| tag7702-1 | EEEEQPGKAPDPQDPQDAESD | 13.15 | 42.86 | 19.05 | 4.76 | 4.76 | 9.52 |
| tag7702-2 | EEEQPGKAPDPQDPQDAESDS | 17.29 | 38.1 | 19.05 | 4.76 | 4.76 | 9.52 |
| tag7702-3 | EEEEQPGKAPDPQDPQDAESDS | 17.57 | 40.91 | 18.18 | 4.55 | 4.55 | 9.09 |
| tag8341-1 | PPPSFFFGPFFPPKASPFSF | 23.65 | 35 | 35 | 5 | 5 | 5 |
| tag8341-2 | PPPPSEEEGPEEPPKASPESE | 23.58 | 33.33 | 38.1 | 4.76 | 4.76 | 4.76 |
| tag8341-3 | PPPSEEEGPEEPPKASPESEA | 24.91 | 33.33 | 33.33 | 4.76 | 4.76 | 9.52 |
| tag10102-1 | DDAEEPESPPPPPRSPSPEP | 19.00 | 30 | 45 | 5 | 0 | 5 |
| tag11508-1 | SGEASSSEEEPPSPDDKENQA | 14.91 | 33.33 | 14.29 | 4.76 | 4.76 | 9.52 |
| tag11508-2 | GEASSSEEEPPSPDDKENQAP | 14.49 | 33.33 | 19.05 | 4.76 | 4.76 | 9.52 |
| tag11508-3 | SGEASSSEEEPPSPDDKENQAP | 13.03 | 31.82 | 18.18 | 4.55 | 4.55 | 9.09 |
| tag13088-1 | PPPPPPPEESSDSEPEAEPG | 14.98 | 30 | 45 | 0 | 5 | 5 |
| tag13088-2 | PPPPPEESSDSEPEAEPGSP | 16.42 | 30 | 40 | 0 | 5 | 5 |
| tag13088-3 | PPPEESSDSEPEAEPGSPQK | 20.58 | 30 | 30 | 5 | 5 | 5 |
| tag13619-1 | SDPEPPDAGEDSKSENGENAP | 11.52 | 33.33 | 19.05 | 4.76 | 9.52 | 9.52 |
| tag 14205-1 | SDSESEDPPRNQASDSENEE | 10.73 | 40 | 10 | 5 | 0 | 5 |
| tag14666-1 | GPGEDAEPDEDPQSEDSEAPS | 10.21 | 42.86 | 19.05 | 0 | 9.52 | 9.52 |
| tag 14666-2 | PGEDAEPDEDPQSEDSEAPSS | 10.04 | 42.86 | 19.05 | 0 | 4.76 | 9.52 |
| tag14666-3 | GPGEDAEPDEDPQSEDSEAPSS | 12.08 | 40.91 | 18.18 | 0 | 9.09 | 9.09 |
| tag15430-1 | ESESSSSDSEANEPSQSASPEPE | 14.06 | 30.43 | 13.04 | 0 | 0 | 8.7 |
| tag15430-2 | SDSESESSSSDSEANEPSQSASPEPE | 10.87 | 30.77 | 11.54 | 0 | 0 | 7.69 |
| tag15430-3 | DSESESSSSDSEANEPSQSASPEPEP | 7.54 | 30.77 | 15.38 | 0 | 0 | 7.69 |
| tag16604-1 | HQEDSEEESQEEEAEGASEPPPP | 12.72 | 43.48 | 17.39 | 4.35 | 4.35 | 8.7 |
| tag16604-2 | GDHQEDSEEESQEEEAEGASEPPPP | 12.95 | 44 | 16 | 4 | 8 | 8 |
| tag17053-1 | PSQPPEEPEPDEAESSPDPQ | 15.35 | 35 | 35 | 0 | 0 | 5 |
| tag 17053-2 | PAPSQPPEEPEPDEAESSPDP | 16.53 | 33.33 | 38.1 | 0 | 0 | 9.52 |
| tag17053-3 | DPAPSQPPEEPEPDEAESSPDP | 14.03 | 36.36 | 36.36 | 0 | 0 | 9.09 |
| tag17170 | PGSQPQASSGPEAEEEEEDDE | 11.11 | 42.86 | 14.29 | 0 | 9.52 | 9.52 |
| tag17514-1 | SPDSQEEQKGESSASSPEEP | 13.45 | 30 | 15 | 5 | 5 | 5 |
| tag17514-2 | PADSPDSQEEQKGESSASSPEEP | 15.02 | 30.43 | 17.39 | 4.35 | 4.35 | 8.7 |
| tag17514-3 | ADSPDSQEEQKGLSSASSPEEPE | 16.14 | 34.78 | 13.04 | 4.35 | 4.35 | 8.7 |
| tag17603-1 | PSPEDESSSSSSSSSSEDEE | 6.01 | 35 | 10 | 0 | 0 | 0 |
| tag17603-2 | PRSPSPEDESSSSSSSSSSEDEE | 5.96 | 30.43 | 13.04 | 4.35 | 0 | 0 |
| tag17603-3 | PRSPSPEDESSSSSSSSSSEDEEE | 7.22 | 33.33 | 12.5 | 4.17 | 0 | 0 |
| tag18253-1 | SSSDSSDSDSSEDDEAPSKP | 3.59 | 35 | 10 | 5 | 0 | 5 |
| tag18453-1 | PSPGSPRGQPQDQDDDEDDEE | 6.12 | 42.86 | 19.05 | 4.76 | 9.52 | 0 |
| tag18467-1 | PAGDGEAGPQQAEDHPQNPPEDPNQDPPEDD | 9.87 | 32.26 | 25.81 | 3.23 | 9.68 | 9.68 |
| tag18467-2 | AGDGEAGPQQAEDHPQNPPEDPNQDPPEDDS | 5.73 | 32.26 | 22.58 | 3.23 | 9.68 | 9.68 |
| tag18467-3 | PAGDGEAGPQQAEDHPQNPPEDPNQDPPEDDS | 8.73 | 31.25 | 25 | 3.13 | 9.38 | 9.38 |
| tag18478-1 | QNQKEAEEPGPDSENSQENP | 14.94 | 30 | 15 | 5 | 5 | 5 |
| tag18478-2 | NQKEAEEPGPDSENSQENPP | 11.47 | 30 | 20 | 5 | 5 | 5 |
| tag19033-1 | DQNESQSPQEPEEGPSEDDKA | 12.69 | 38.1 | 14.29 | 4.76 | 4.76 | 4.76 |
| tag19033-2 | QNESQSPQEPEEGPSEDDKAE | 12.4 | 38.1 | 14.29 | 4.76 | 4.76 | 4.76 |
| tag19033-3 | NESQSPQEPEEGPSEDDKAEG | 12.28 | 38.1 | 14.29 | 4.76 | 9.52 | 4.76 |
| tag29487-1 | PASSSSNPEEGPEEDREAESE | 12.77 | 38.1 | 14.29 | 4.76 | 4.76 | 9.52 |
| tag34831-1 | DKPEEEDDEAQQPQPQSGPEEAE | 7.58 | 43.48 | 17.39 | 4.35 | 4.35 | 8.7 |
| tag34831-2 | KPEEEDDEAQQPQPQSGPEEAEE | 8.02 | 43.48 | 17.39 | 4.35 | 4.35 | 8.7 |
| tag34831-3 | PEEEDDEAQQPQPQSGPEEAEEG | 9.14 | 43.48 | 17.39 | 0 | 8.7 | 8.7 |
| tag34858-1 | PEEEHAPGEDESSPQPSQPS | 17.52 | 30 | 25 | 5 | 5 | 5 |

In Table 13, the P content ratio in the amino acid sequences of the extracted tags is 15% or more, and the content ratios of the other amino acids are as described above in Extraction Condition 2. In order to confirm that the aggregation inhibiting action does not depend on a specific amino acid sequence, tags were randomly selected from the tags extracted under Extraction Condition 2. As for some tags, an amino acid sequence satisfying the extraction condition was additionally selected from another portion of the same protein. The aggregation rates of scFvs tagged with the selected amino acid sequences were tested, and results as shown in Table 13 were obtained. As shown in Table 13, the aggregation rates of the tagged scFvs were low as a whole.

It is noted that human-derived amino acid sequences that can be extracted under Extraction Condition 2 were as follows.

### [Table 13-2]

**Table 13-2: Examples of human-derived amino acid sequences that can be extracted under Extraction Condition 2**

| SEQ ID NO: | Sequence |
|---|---|
| 492 | SSDSPKDQSPPEDSGESEAD (tag47-1) |
| 493 | PGPEPEPEPEPEPEPAPEPE |
| 494 | GPEPEPEPEPEPEPAPEPEP |
| 495 | PEPEPEPEPEPAPEPEPEPK |
| 496 | EPEPEPEPEPAPEPEPEPKP |
| 497 | PEPEPEPEPAPEPEPEPKPG |
| 498 | AGPGPEPEPEPEPEPEPAPEP |
| 499 | GPGPEPEPEPEPEPEPAPEPE |
| 500 | PGPEPEPEPEPEPEPAPEPEP |
| 501 | GPEPEPEPEPEPEPAPEPEPE (tag1784-1) |
| 502 | PEPEPEPEPEPEPAPEPEPEP (tag1784-2) |
| 503 | EPEPEPEPEPEPAPEPEPEPK (tag1784-3) |
| 504 | PEPEPEPEPEPAPEPEPEPKP |
| 505 | EPEPEPEPEPAPEPEPEPKPG |
| 506 | PEPEPEPEPAPEPEPEPKPGA |
| 507 | EPEPEPEPAPEPEPEPKPGAG |
| 508 | AGPGPEPEPEPEPEPEPAPEPE |
| 509 | GPGPEPEPEPEPEPEPAPEPEP |
| 510 | PGPEPEPEPEPEPEPAPEPEPE |
| 511 | GPEPEPEPEPEPEPAPEPEPEP |
| 512 | PEPEPEPEPEPEPAPEPEPEPK |
| 513 | EPEPEPEPEPEPAPEPEPEPKP |
| 514 | PEPEPEPEPEPAPEPEPEPKPG |
| 515 | EPEPEPEPEPAPEPEPEPKPGA |
| 516 | PEPEPEPEPAPEPEPEPKPGAG |
| 517 | AGPGPEPEPEPEPEPEPAPEPEP |
| 518 | GPGPEPEPEPEPEPEPAPEPEPE |
| 519 | PGPEPEPEPEPEPEPAPEPEPEP |
| 520 | GPEPEPEPEPEPEPAPEPEPEPK |
| 521 | PEPEPEPEPEPEPAPEPEPEPKP |
| 522 | EPEPEPEPEPEPAPEPEPEPKPG |
| 523 | PEPEPEPEPEPAPEPEPEPKPGA |
| 524 | EPEPEPEPEPAPEPEPEPKPGAG |
| 525 | AGPGPEPEPEPEPEPEPAPEPEPE |
| 526 | GPGPEPEPEPEPEPEPAPEPEPEP |
| 527 | PGPEPEPEPEPEPEPAPEPEPEPK |
| 528 | GPEPEPEPEPEPEPAPEPEPEPKP |
| 529 | PEPEPEPEPEPEPAPEPEPEPKPG |
| 530 | EPEPEPEPEPEPAPEPEPEPKPGA |
| 531 | PEPEPEPEPEPAPEPEPEPKPGAG |
| 532 | AGPGPEPEPEPEPEPEPAPEPEPEP |
| 533 | GPGPEPEPEPEPEPEPAPEPEPEPK |
| 534 | PGPEPEPEPEPEPEPAPEPEPEPKP |
| 535 | GPEPEPEPEPEPEPAPEPEPEPKPG |
| 536 | PEPEPEPEPEPEPAPEPEPEPKPGA |
| 537 | EPEPEPEPEPEPAPEPEPEPKPGAG |
| 538 | AGPGPEPEPEPEPEPEPAPEPEPEPK |
| 539 | GPGPEPEPEPEPEPEPAPEPEPEPKP |
| 540 | PGPEPEPEPEPEPEPAPEPEPEPKPG |
| 541 | GPEPEPEPEPEPEPAPEPEPEPKPGA |
| 542 | PEPEPEPEPEPEPAPEPEPEPKPGAG |
| 543 | AGPGPEPEPEPEPEPEPAPEPEPEPKP |
| 544 | PGPEPEPEPEPEPEPAPEPEPEPKPGA |
| 545 | DPEEDGSPDPEPSPEPEPKP |
| 546 | PEEDGSPDPEPSPEPEPKPS (tag2257-1) |
| 547 | EDPEEDGSPDPEPSPEPEPKP (tag2257-2) |
| 548 | DPEEDGSPDPEPSPEPEPKPS (tag2257-3) |
| 549 | EDPEEDGSPDPEPSPEPEPKPS |
| 550 | DEDPEEDGSPDPEPSPEPEPKPS |
| 551 | PDDDGSDDSSPPSASPAESEP (tag4398-1) |
| 552 | DDDGSDDSSPPSASPAESEPQ (tag4398-2) |
| 553 | PDDDGSDDSSPPSASPAESEPQ (tag4398-3) |
| 554 | PPPEEQGQGDAPPQHEDEEPA (tag4898-1) |
| 555 | PSPAPSPDSDSDSDSDGEEE |
| 556 | GAPSPAPSPDSDSDSDSDGEE (tag5533-1) |
| 557 | APSPAPSPDSDSDSDSDGEEE (tag5533-2) |
| 558 | PSPAPSPDSDSDSDSDGEEEE (tag5533-3) |
| 559 | NGAPSPAPSPDSDSDSDSDGEE |
| 560 | GAPSPAPSPDSDSDSDSDGEEE |
| 561 | APSPAPSPDSDSDSDSDGEEEE |
| 562 | QNGAPSPAPSPDSDSDSDSDGEE |
| 563 | NGAPSPAPSPDSDSDSDSDGEEE |
| 564 | GAPSPAPSPDSDSDSDSDGEEEE |
| 565 | APSPAPSPDSDSDSDSDGEEEEE |
| 566 | QNGAPSPAPSPDSDSDSDSDGEEE |
| 567 | NGAPSPAPSPDSDSDSDSDGEEEE |
| 568 | GAPSPAPSPDSDSDSDSDGEEEEE |
| 569 | QNGAPSPAPSPDSDSDSDSDGEEEE |
| 570 | NGAPSPAPSPDSDSDSDSDGEEEEE |
| 571 | GAPSPAPSPDSDSDSDSDGEEEEEE |
| 572 | QNGAPSPAPSPDSDSDSDSDGEEEEE |
| 573 | NGAPSPAPSPDSDSDSDSDGEEEEEE |
| 574 | QNGAPSPAPSPDSDSDSDSDGEEEEEE |
| 575 | NGAPSPAPSPDSDSDSDSDGEEEEEEE |
| 576 | QNGAPSPAPSPDSDSDSDSDGEEEEEEE |
| 577 | QNGAPSPAPSPDSDSDSDSDGEEEEEEE GER |
| 578 | QNGAPSPAPSPDSDSDSDSDGEEEEEEE GERD |
| 579 | EPPAPPPPPPPEEDPEQDSG |
| 580 | PAPPPPPPPEEDPEQDSGPE (tag5601-1) |
| 581 | APPPPPPPEEDPEQDSGPED (tag5601-2) |
| 582 | PAPPPPPPPEEDPEQDSGPED (tag5601-3) |
| 583 | EPPAPPPPPPPEEDPEQDSGPE |
| 584 | PPAPPPPPPPEEDPEQDSGPED |
| 585 | AEPPAPPPPPPPEEDPEQDSGPE |
| 586 | EPPAPPPPPPPEEDPEQDSGPED |
| 587 | AEPPAPPPPPPPEEDPEQDSGPED |
| 588 | PPPPPQAPPEEENESEPEEP |
| 589 | PPPPQAPPEEENESEPEEPS |
| 590 | PPPQAPPEEENESEPEEPSG |
| 591 | PPPPPQAPPEEENESEPEEPS (tag6354-1) |
| 592 | PPPPQAPPEEENESEPEEPSG (tag6354-2) |
| 593 | PPPPPQAPPEEENESEPEEPSG (tag6354-3) |
| 594 | PQDSSSKSPEPSADESPDND (tag6681-1) |
| 595 | QSSDNSEDEEEPPDNADSKS (tag7124-1) |
| 596 | EEEEEQPGKAPDPQDPQDAES |
| 597 | EEEEQPGKAPDPQDPQDAESD (tag7702-1) |
| 598 | EEEQPGKAPDPQDPQDAESDS (tag7702-2) |
| 599 | EEEEQPGKAPDPQDPQDAESDS (tag7702-3) |
| 600 | EEEEEQPGKAPDPQDPQDAESDS |
| 601 | PPPPPSEEEGPEEPPKASPE |
| 602 | PPPPSEEEGPEEPPKASPES |
| 603 | PPPSEEEGPEEPPKASPESE (tag8341-1) |
| 604 | PPPPSEEEGPEEPPKASPESE (tag8341-2) |
| 605 | PPPSEEEGPEEPPKASPESEA (tag8341-3) |
| 606 | PPPPPSEEEGPEEPPKASPESE |
| 607 | PPPPSEEEGPEEPPKASPESEA |
| 608 | PPPPPSEEEGPEEPPKASPESEA |
| 609 | DDAEEPESPPPPPRSPSPEP (tag10102-1) |
| 610 | SGEASSSEEEPPSPDDKENQ |
| 611 | SGEASSSEEEPPSPDDKENQA (tag11508-1) |
| 612 | GEASSSEEEPPSPDDKENQAP (tag11508-2) |
| 613 | SGEASSSEEEPPSPDDKENQAP (tag11508-3) |
| 614 | PQPPPPPPPEESSDSEPEAE |
| 615 | QPPPPPPPEESSDSEPEAEP |
| 616 | PPPPPPPEESSDSEPEAEPG (tag13088-1) |
| 617 | PPPPPPEESSDSEPEAEPGS |
| 618 | PPPPPEESSDSEPEAEPGSP (tag13088-2) |
| 619 | PPPPEESSDSEPEAEPGSPQ |
| 620 | PPPEESSDSEPEAEPGSPQK (tag13088-3) |
| 621 | SDPEPPDAGEDSKSENGENAP (tag13619-1) |
| 622 | SDSESEDPPRNQASDSENEE (tag14205-1) |
| 623 | GPGEDAEPDEDPQSEDSEAPS (tag14666-1) |
| 624 | PGEDAEPDEDPQSEDSEAPSS (tag14666-2) |
| 625 | GPGEDAEPDEDPQSEDSEAPSS (tag14666-3) |
| 626 | ESESSSSDSEANEPSQSASPEPE (tag15430-1) |
| 627 | DSESESSSSDSEANEPSQSASPEPE |
| 628 | SDSESESSSSDSEANEPSQSASPEPE (tag15430-2) |
| 629 | DSESESSSSDSEANEPSQSASPEPEP (tag15430-3) |
| 630 | HQEDSEEESQEEEAEGASEPPPP (tag16604-1) |
| 631 | GDHQEDSEEESQEEEAEGASEPPPP (tag16604-2) |
| 632 | PSQPPEEPEPDEAESSPDPQ (tag17053-1) |
| 633 | DPAPSQPPEEPEPDEAESSPD |
| 634 | PAPSQPPEEPEPDEAESSPDP (tag17053-2) |
| 635 | APSQPPEEPEPDEAESSPDPQ |
| 636 | PSQPPEEPEPDEAESSPDPQA |
| 637 | DPAPSQPPEEPEPDEAESSPDP (tag17053-3) |
| 638 | PAPSQPPEEPEPDEAESSPDPQ |
| 639 | DPAPSQPPEEPEPDEAESSPDPQ |
| 640 | PGSQPQASSGPEAEEEEEDDE (tag17170) |
| 641 | DSPDSQEEQKGESSASSPEE |
| 642 | SPDSQEEQKGESSASSPEEP (tag17514-1) |
| 643 | PDSQEEQKGESSASSPEEPE |
| 644 | DSQEEQKGESSASSPEEPEE |
| 645 | DSPDSQEEQKGESSASSPEEP |
| 646 | SPDSQEEQKGESSASSPEEPE |
| 647 | PDSQEEQKGESSASSPEEPEE |
| 648 | PADSPDSQEEQKGESSASSPEE |
| 649 | ADSPDSQEEQKGESSASSPEEP |
| 650 | DSPDSQEEQKGESSASSPEEPE |
| 651 | SPDSQEEQKGESSASSPEEPEE |
| 652 | PADSPDSQEEQKGESSASSPEEP (tag17514-2) |
| 653 | ADSPDSQEEQKGESSASSPEEPE (tag17514-3) |
| 654 | DSPDSQEEQKGESSASSPEEPEE |
| 655 | PADSPDSQEEQKGESSASSPEEPE |
| 656 | ADSPDSQEEQKGESSASSPEEPEE |
| 657 | PADSPDSQEEQKGESSASSPEEPEE |
| 658 | SPSPEDESSSSSSSSSSEDE |
| 659 | PSPEDESSSSSSSSSSEDEE (tag17603-1) |
| 660 | PRSPSPEDESSSSSSSSSSEDEE (tag17603-2) |
| 661 | PRSPSPEDESSSSSSSSSSEDEEE (tag17603-3) |
| 662 | SSSDSSDSDSSEDDEAPSKP (tag18253-1) |
| 663 | PSPGSPRGQPQDQDDDEDDEE (tag18453-1) |
| 664 | QQAEDHPQNPPEDPNQDPPE |
| 665 | QAEDHPQNPPEDPNQDPPED |
| 666 | AEDHPQNPPEDPNQDPPEDD |
| 667 | EDHPQNPPEDPNQDPPEDDS |
| 668 | QQAEDHPQNPPEDPNQDPPED |
| 669 | QAEDHPQNPPEDPNQDPPEDD |
| 670 | AEDHPQNPPEDPNQDPPEDDS |
| 671 | PQQAEDHPQNPPEDPNQDPPED |
| 672 | QQAEDHPQNPPEDPNQDPPEDD |
| 673 | QAEDHPQNPPEDPNQDPPEDDS |
| 674 | DGEAGPQQAEDHPQNPPEDPNQD |
| 675 | GPQQAEDHPQNPPEDPNQDPPED |
| 676 | PQQAEDHPQNPPEDPNQDPPEDD |
| 677 | QQAEDHPQNPPEDPNQDPPEDDS |
| 678 | GPQQAEDHPQNPPEDPNQDPPEDD |
| 679 | PQQAEDHPQNPPEDPNQDPPEDDS |
| 680 | EAGPQQAEDHPQNPPEDPNQDPPED |
| 681 | AGPQQAEDHPQNPPEDPNQDPPEDD |
| 682 | GPQQAEDHPQNPPEDPNQDPPEDDS |
| 683 | DGEAGPQQAEDHPQNPPEDPNQDPPE |
| 684 | GEAGPQQAEDHPQNPPEDPNQDPPED |
| 685 | EAGPQQAEDHPQNPPEDPNQDPPEDD |
| 686 | AGPQQAEDHPQNPPEDPNQDPPEDDS |
| 687 | DGEAGPQQAEDHPQNPPEDPNQDPPED |
| 688 | GEAGPQQAEDHPQNPPEDPNQDPPEDD |
| 689 | EAGPQQAEDHPQNPPEDPNQDPPEDDS |
| 690 | DGEAGPQQAEDHPQNPPEDPNQDPPED D |
| 691 | GEAGPQQAEDHPQNPPEDPNQDPPEDD S |
| 692 | DGEAGPQQAEDHPQNPPEDPNQDPPED DS |
| 693 | PAGDGEAGPQQAEDHPQNPPEDPNQDP PEDD (tag18467-1) |
| 694 | AGDGEAGPQQAEDHPQNPPEDPNQDPP EDDS (tag18467-2) |
| 695 | PAGDGEAGPQQAEDHPQNPPEDPNQDP PEDDS (tag18467-3) |
| 696 | QNQKEAEEPGPDSENSQENP (tag18478-1) |
| 697 | NQKEAEEPGPDSENSQENPP (tag18478-2) |
| 698 | DQNESQSPQEPEEGPSEDDK |
| 699 | QNESQSPQEPEEGPSEDDKA |
| 700 | NESQSPQEPEEGPSEDDKAE |
| 701 | DQNESQSPQEPEEGPSEDDKA(tag19033-1) |
| 702 | QNESQSPQEPEEGPSEDDKAE (tag19033-2) |
| 703 | NESQSPQEPEEGPSEDDKAEG (tag19033-3) |
| 704 | ESQSPQEPEEGPSEDDKAEGE |
| 705 | SQSPQEPEEGPSEDDKAEGEE |
| 706 | DQNESQSPQEPEEGPSEDDKAE |
| 707 | QNESQSPQEPEEGPSEDDKAEG |
| 708 | NESQSPQEPEEGPSEDDKAEGE |
| 709 | DQNESQSPQEPEEGPSEDDKAEG |
| 710 | QNESQSPQEPEEGPSEDDKAEGE |
| 711 | NESQSPQEPEEGPSEDDKAEGEE |
| 712 | DQNESQSPQEPEEGPSEDDKAEGE |
| 713 | QNESQSPQEPEEGPSEDDKAEGEE |
| 714 | DQNESQSPQEPEEGPSEDDKAEGEE |
| 715 | QNESQSPQEPEEGPSEDDKAEGEEE |
| 716 | XEASSSEEEPPSPDDKENQAP (tag25919-1) |
| 717 | PASSSSNPEEGPEEDREAESE (tag29487-1) |
| 718 | DKPEEEDDEAQQPQPQSGPE |
| 719 | KPEEEDDEAQQPQPQSGPEE |
| 720 | AGEGDKPEEEDDEAQQPQPQS |
| 721 | EGDKPEEEDDEAQQPQPQSGP |
| 722 | GDKPEEEDDEAQQPQPQSGPE |
| 723 | DKPEEEDDEAQQPQPQSGPEE |
| 724 | KPEEEDDEAQQPQPQSGPEEA |
| 725 | PEEEDDEAQQPQPQSGPEEAE |
| 726 | EEDDEAQQPQPQSGPEEAEEG |
| 727 | EDDEAQQPQPQSGPEEAEEGE |
| 728 | DDEAQQPQPQSGPEEAEEGEE |
| 729 | DEAQQPQPQSGPEEAEEGEEE |
| 730 | EAQQPQPQSGPEEAEEGEEEE |
| 731 | QQPQPQSGPEEAEEGEEEEAE |
| 732 | QPQPQSGPEEAEEGEEEEAER |
| 733 | EGDKPEEEDDEAQQPQPQSGPE |
| 734 | GDKPEEEDDEAQQPQPQSGPEE |
| 735 | DKPEEEDDEAQQPQPQSGPEEA |
| 736 | KPEEEDDEAQQPQPQSGPEEAE |
| 737 | QQPQPQSGPEEAEEGEEEEAER |
| 738 | EGDKPEEEDDEAQQPQPQSGPEE |
| 739 | GDKPEEEDDEAQQPQPQSGPEEA |
| 740 | DKPEEEDDEAQQPQPQSGPEEAE (tag34831-1) |
| 741 | KPEEEDDEAQQPQPQSGPEEAEE (tag34831-2) |
| 742 | PEEEDDEAQQPQPQSGPEEAEEG (tag34831-3) |
| 743 | EGDKPEEEDDEAQQPQPQSGPEEA |
| 744 | GDKPEEEDDEAQQPQPQSGPEEAE |
| 745 | KPEEEDDEAQQPQPQSGPEEAEEG |
| 746 | EGDKPEEEDDEAQQPQPQSGPEEAE |
| 747 | GDKPEEEDDEAQQPQPQSGPEEAEE |
| 748 | DKPEEEDDEAQQPQPQSGPEEAEEG |
| 749 | KPEEEDDEAQQPQPQSGPEEAEEGE |
| 750 | PEEEHAPGEDESSPQPSQPS (tag34858-1) |
| 751 | XPPPEESSDSEPEAEPGSPQ (tag) |
| 752 | PPPEESSDSEPEAEPGSPQK (tag) |

### Extraction Condition 3:

length: 20 to 70 amino acids
group [D, E]: content of [45] or more
group [G]: content of less than [10]
group [A]: content of less than [10]
group [F, Y]: content of [0]
group [C, M, L, I, W, T, V]: content of [0]
group [P]: content of [15] or more

**[Table 14]**

| Table 14: Aggregation Rates of scFvs havinq Tags Extracted under Extraction Condition 3 Added | | | | | | |
|---|---|---|---|---|---|---|
| Tag Name | Sequence | Aggregation Rate % | DE Rate | P Rate | G Rate | A Rate |
| Tag 167-1 | KEPKEEKKDDDEEAPKPSSD | 8.02 | 45 | 15 | 0 | 5 |
| Tag 1034-1 | JSEEEKPPEEDKEEEEEKKAP | 8.91 | 55 | 15 | 0 | 5 |
| Tag 1409-1 | PAEEDEDDPEQEKEAGEPGRP | 5.17 | 47.62 | 19.05 | 9.52 | 9.52 |
| Tag 1784-1 | EPEPEPEPEPEPAPEPEPEP | 12.97 | 45 | 50 | 0 | 5 |
| Tag 2257-1 | EDPEEDGSPDPEPSPEPEPK | 12.61 | 45 | 35 | 5 | 0 |
| Tag 2257-2 | DEDPEEDGSPDPEPSPEPEPK | 11.50 | 47.62 | 33.33 | 4.76 | 0 |
| Tag 2257-3 | DEDPEEDGSPDPEPSPEPEPKP | 8.62 | 45.45 | 36.36 | 4.55 | 0 |
| Tag 2740-1 | KPEDKDPRDPEESKEPKEEK | 13.22 | 45 | 20 | 0 | 0 |
| Tag 2740-2 | PEDKDPRDPEESKEPKEEKQ | 11.68 | 45 | 20 | 0 | 0 |
| Tag 2740-3 | EDKDPRDPEESKEPKEEKQR | 8.79 | 45 | 15 | 0 | 0 |
| Tag 3227-1 | KRNDSEEEERERDEEQEPPP | 20.63 | 0 | 15 | 0 | 0 |
| Tag 4898-1 | PEEEPDDQDAPDEHEPSPSED | 10.71 | 52.38 | 23.81 | 0 | 4.76 |
| Tag 4898-2 | EEEPDDQDAPDEHEPSPSEDA | 7.01 | 52.38 | 19.05 | 0 | 9.52 |
| Tag 4898-3 | EEPDDQDAPDEHEPSPSEDAP | 19.90 | 47.62 | 23.81 | 0 | 9.52 |
| Tag 5533-1 | PSPAPSPDSDSDSDSDGEEEEEEE | 1.89 | 50 | 16.67 | 4.17 | 4.17 |
| Tag 5533-2 | APSPAPSPDSDSDSDSDGEEEEEEE | 1.00 | 48 | 16 | 4 | 8 |
| Tag 5533-3 | PSPAPSPDSDSDSDSDGEEEEEEEG | 0.90 | 48 | 16 | 8 | 4 |
| Tag 6236-1 | EKNDEDEPQKPEDKGDPEGPE | 10.19 | 47.62 | 19.05 | 9.52 | 0 |
| Tag 6236-2 | EKNDEDEPQKPEDKGDPEGPEA | 8.40 | 45.45 | 18.18 | 9.09 | 4.55 |
| Tag 6755-1 | EDEEEEEEEEEEEDEGPAPP | 4.10 | 75 | 15 | 5 | 5 |
| Tag 6755-2 | DEEEEEEEEEEEDEGPAPPS | 2.38 | 70 | 15 | 5 | 5 |
| Tag 7167-1 | IGEREPDPPDDRDASDGEDEKP | 12.37 | 47.62 | 19.05 | 9.52 | 4.76 |
| Tag 7167-2 | EREPDPPDDRDASDGEDEKPP | 8.71 | 47.62 | 23.81 | 4.76 | 4.76 |
| Tag 7167-3 | EGEREPDPPDDRDASDGEDEKP | 9.97 | 50 | 18.18 | 9.09 | 4.55 |
| Tag 7702-1 | EEEEEQPGKAPDPQDPQDAESD | 13.30 | 45.45 | 18.18 | 4.55 | 9.09 |
| Tag 8243-1 | EPEEKQEPEEKQEPEEKQKPE | 11.90 | 47.62 | 19.05 | 0 | 0 |
| Tag 8243-2 | QEPEEKQEPEEKQEPEEKQKPE | 12.44 | 45.45 | 18.18 | 0 | 0 |
| Tag 8243-3 | EPEEKQEPEEKQEPEEKQKPEA | 12.60 | 45.45 | 18.18 | 0 | 4.55 |
| Tag 8818-1 | DDDDDDDSPDPESPDDSESD | 2.58 | 65 | 15 | 0 | 0 |
| Tag 8818-2 | DDDDDDSPDPESPDDSESDS | 2.19 | 60 | 15 | 0 | 0 |
| Tag 8818-3 | DDSPDPESPDDSESDSESEK | 8.33 | 50 | 15 | 6 | 0 |
| Tag 9050-1 | DPDQPREDPAEEEKEEKDAPE | 15.60 | 52.38 | 19.05 | 0 | 9.52 |
| Tag 9166-1 | PENESEPKHEEEPKPEEKPEEE | 17.13 | 50 | 22.73 | 0 | 0 |
| Tag 9168-2 | NESEPKHEEEPKPEEKPEEEEK | 7.62 | 50 | 18.18 | 0 | 0 |
| Tag 9166-3 | PENESEPKHEEEPKPEEKPEEE | 13.00 | 52.17 | 21.74 | 0 | 0 |
| Tag 9590-1 | PPEEDPEEQAEEMPEGEQPE | 13.99 | 50 | 25 | 5 | 5 |
| Tag 9590-2 | KPPEEDPEEQAEENPEGEQPE | 10.18 | 47.62 | 23.81 | 4.76 | 4.76 |
| Tag 9590-3 | KPPEEDPEEQAEENPEGEQPEE | 9.67 | 50 | 22.73 | 4.55 | 4.55 |
| Tag 9704-1 | PDDDDESEDHDDPDNAHESP | 7.65 | 55 | 15 | 0 | 5 |
| Tag 9749-1 | PEPEPEPEPEPESEPEPEPE | 15.74 | 50 | 45 | 0 | 0 |
| Tag 9749-2 | GGEPEPEPEPEPEPEPESEPEPE | 12.24 | 47.83 | 39.13 | 8.7 | 0 |
| Tag 9749-3 | GGEPEPEPEPEPEPEPESEPEPEPE | 14.29 | 48 | 40 | 8 | 0 |
| Tag 10346-1 | PEEEPDDQDAPDEHESPPPE | 8.16 | 50 | 30 | 0 | 5 |
| Tag 10346-2 | EEFPDDQDAPDEHESPPPEDA | 6.88 | 52.38 | 23.81 | 0 | 9.52 |
| Tag 10346-3 | PEEEPDDQDAPDEHESPPPEDAP | 8.97 | 47.83 | 30.43 | 0 | 8.7 |
| Tag 11099-1 | GPSSDDENEEESKPEKEDEP | 7.21 | 50 | 15 | 5 | 0 |
| Tag 11099-2 | PSSDDENEEESKPEKEDEPQ | 5.09 | 50 | 15 | 0 | 0 |
| Tag 12127-1 | SDDSDSEKRRPEEQEEEPQP | 17.51 | 45 | 15 | 0 | 0 |
| Tag 12127-2 | DDSDSEKRRPEEQEEEPQPR | 17.44 | 45 | 15 | 0 | 0 |
| Tag 13036-1 | PEEEDEEPGDPREGEEEEEEDEPD | 3.17 | 68 | 20 | 8 | 0 |
| Tag 13036-2 | PEEEDEEPGDPREGEEEEEEDEPDPEAP | 5.60 | 64.29 | 2743 | 7.14 | 3.57 |
| Tag 13038-3 | AAPEEEDEEPGDPREGEEEEEEDEPDPEAPENGS | 6.19 | 55.88 | 17.65 | 8.82 | 8.82 |
| Tag 14128-1 | PPPSEGSDEEEEEEDEEDEE | 2.43 | 70 | 15 | 5 | 0 |
| Tag 14128-2 | KPPPSEGSDEEEEEEDEEDEEERKP | 10.86 | 60 | 16 | 4 | 0 |
| Tag 4128-3 | KPPPSEGSDEEEEEEDEEDEEERKPQ | 9.76 | 57.69 | 15.38 | 3.85 | 0 |
| Tag 16549-1 | EEEEEEEEEEEEEEEAPPPP | 4.95 | 75 | 20 | 0 | 5 |
| Tag 16549-2 | EEEEEEEEEEEEEEEEEAPPPPR | 3.01 | 73.91 | 17.39 | 0 | 4.35 |
| Tag 16549-3 1 | PDDDEEDEEEEEEEEEEEEEEEEEEEEEAPPPP | 0.89 | 81.82 | 15.15 | 0 | 3.03 |
| Tag 16604-1 | EDSEEESQEEEAEGASEPPPP | 6.00 | 47.62 | 19.05 | 4.76 | 9.52 |
| Tag 16604-2 | QEDSEEESQEEEAEGASEPPPP | 7.39 | 45.45 | 18.18 | 4.55 | 9.09 |
| Tag 16604-3 | DHQEDSEEESQEEEAEGASEPPPP | 10.59 | 45.83 | 16.67 | 4.17 | 8.33 |
| Tag 16741-1 | DQSEEEEEEEKHPPKPAKPE | 10.62 | 45 | 20 | 0 | 5 |
| Tag 16991-1 | PAPAHRPPEDEGEENEGEEDE | 14.97 | 47.62 | 19.05 | 9.52 | 9.52 |
| Tag 16991-2 | PAPAHRPPEDEGEENEGEEDEE | 7.12 | 50 | 18.18 | 9.09 | 9.09 |
| Tag 17199-1 | QENGQREEEEEEKEPEAEPP | 9.09 | 50 | 15 | 5 | 5 |
| Tag 17199-2 | PAEGQENGQREEEEEEKEPEAEPP | 10.29 | 45.83 | 16.67 | 8.33 | 8.33 |
| Tag 17936-1 | EQEPEPEPEPEPEPEPEPEP | 6.37 | 50 | 45 | 0 | 0 |
| Tag 17938-2 | EPEPEPEPEPEPEPEPEPEQ | 10.94 | 50 | 45 | 0 | 0 |
| Tag 17936-3 | EQEPEPEPEPEPEPEPEPEPEQ | 16.61 | 50 | 40.91 | 0 | 0 |
| Tag 13132-1 | PEEEEEEEEEEEPASPPERK | 3.36 | 60 | 20 | 0 | 5 |
| Tag 18453-1 | PSPGSPRGQPQDQDDDEDDEED | 4.31 | 45.45 | 18.18 | 9.09 | 0 |
| Tag 18453-2 | JRPSPGSPRGQPQDQDDDEDDEEDE | 13.65 | 45.83 | 16.67 | 8.33 | 0 |
| Tag 18453-3 | PSPGSPRGQPQDQDDDEDDEEDEA | 6.54 | 45.83 | 16.67 | 8.33 | 4.17 |
| Tag 1866-1 | AEDDDEEDEEEEEEEPDPDP | 2.24 | 80 | 15 | 0 | 5 |
| Tag 18866-2 | EDDDEEDEEEEEEEPDPDPE | 2.29 | 85 | 15 | 0 | 0 |
| Tag 19350-1 | KQEPPDPEEDKEENKDDSAS | 14.73 | 45 | 15 | 0 | 5 |
| Tag 19350-2 | QEPPDPEEDKEENKDDSASK | 14.12 | 45 | 15 | 0 | 5 |
| Tag 19511-1 | EDEDEDESSEEDSEDEEPPP | 1.74 | 70 | 15 | 0 | 0 |
| Tag 19511-2 | PDDSRDEDEDEDESSEEDSEDEEPPP | 4.37 | 65.38 | 15.38 | 0 | 0 |
| Tag 19511-3 | PKKEPDDSRDEDEDEDESSEEDSEDEEPPPKRR | 3.87 | 54.55 | 15.15 | 0 | 0 |
| Tag 22900-1 | PEEEAAEEEEEEEERPKPSRP | 10.93 | 52.38 | 19.05 | 0 | 9.52 |
| Tag 22900-2 | EQPEEEAAEEEEEEEERPKPSRP | 6.90 | 52.17 | 17.39 | 0 | 8.7 |
| Tag 22900-3 | EEEQPEEEAAEEEEEEEERPKPSRP | 9.67 | 56 | 16 | 0 | 8 |
| Tag 34831-1 | PEEEDDEAQQPQPQSGPEEAEE | 10.16 | 45.45 | 18.18 | 4.55 | 9.09 |
| Tag 34831-2 | EGDKPEEEDDEAQQPQPQSGPEEAEE | 10.78 | 46.15 | 15.38 | 7.69 | 7.69 |
| Tag 34831-3 | KPEEEDDEAQQPQPQSGPEEAEEGEEEEAERGP | 8.33 | 45.45 | 15.15 | 9.09 | 9.09 |
| Tag 39056-1 | NNSEEEEDDDDEEEEPDKPP | 0.44 | 65 | 15 | 0 | 0 |
| Tag 39056-2 | NSEEEEDDDDEEEEPDKPPA | 0.99 | 65 | 15 | 0 | 5 |
| Tag 39056-3 | SEEEEDDDDEEEEPDKPPAN | 1.23 | 65 | 15 | 0 | 5 |

In Table 14, the acidic amino acid ratio in the amino acid sequences of the extracted tags is 45% or more, the P content ratio is 15% or more, and the content ratios of the other amino acids are as described above in Extraction Condition 3. In order to confirm that the aggregation inhibiting action does not depend on a specific amino acid sequence, tags were randomly selected from the tags extracted under Extraction Condition 3. As for some tags, an amino acid sequence satisfying the extraction condition was additionally selected from another portion of the same protein. The aggregation rates of scFvs tagged with the selected amino acid sequences were tested, and results as shown in Table 14 were obtained. As shown in Table 14, the aggregation rates of the tagged scFvs were low as a whole. It is understood, from Table 14-1, that a strategy of reducing the G, A, F, Y, C, M, L, I, W, T, and V contents and increasing the P content works on a peptide tag having a high acidic amino acid content.

It is noted that human-derived amino acid sequences that can be extracted under Extraction Condition 3 were as follows.

### [Table 14-2]

**Table 14-2: Examples of human-derived amino acid sequences that can be extracted under Extraction Condition 3**

| SEQ ID NO: | Sequence |
|---|---|
| 753 | KEPKEEKKDDDEEAPKPSSD (Tag 167-1) |
| 754 | SEEEKPPEEDKEEEEEKKAP (Tag 1034-1) |
| 755 | PAEEDEDDPEQEKEAGEPGRP (Tag 1409-1) |
| 756 | PEPEPEPEPEPEPAPEPEPE |
| 757 | EPEPEPEPEPEPAPEPEPEP (Tag 1784-1) |
| 758 | DEDPEEDGSPDPEPSPEPEP |
| 759 | EDPEEDGSPDPEPSPEPEPK (Tag 2257-1) |
| 760 | DEDPEEDGSPDPEPSPEPEPK (Tag 2257-2) |
| 761 | DEDPEEDGSPDPEPSPEPEPKP (Tag 2257-3) |
| 762 | KPEDKDPRDPEESKEPKEEK (Tag 2740-1) |
| 763 | PEDKDPRDPEESKEPKEEKQ (Tag 2740-2) |
| 764 | EDKDPRDPEESKEPKEEKQR (Tag 2740-3) |
| 765 | KRNDSEEEERERDEEQEPPP (Tag 3227-1) |
| 766 | PEEEPDDQDAPDEHEPSPSE |
| 767 | EEEPDDQDAPDEHEPSPSED |
| 768 | PEEEPDDQDAPDEHEPSPSED (Tag 4898-1) |
| 769 | EEEPDDQDAPDEHEPSPSEDA (Tag 4898-2) |
| 770 | EEPDDQDAPDEHEPSPSEDAP (Tag 4898-3) |
| 771 | PEEEPDDQDAPDEHEPSPSEDA |
| 772 | EEEPDDQDAPDEHEPSPSEDAP |
| 773 | PEEEPDDQDAPDEHEPSPSEDAP |
| 774 | SPAPSPDSDSDSDSDGEEEE |
| 775 | PAPSPDSDSDSDSDGEEEEE |
| 776 | PSPAPSPDSDSDSDSDGEEEEE |
| 777 | PSPAPSPDSDSDSDSDGEEEEEE |
| 778 | APSPAPSPDSDSDSDSDGEEEEEE |
| 779 | PSPAPSPDSDSDSDSDGEEEEEEE (Tag 5533-1) |
| 780 | APSPAPSPDSDSDSDSDGEEEEEEE(Tag 5533-2) |
| 781 | PSPAPSPDSDSDSDSDGEEEEEEEG (Tag 5533-3) |
| 782 | GAPSPAPSPDSDSDSDSDGEEEEEEE |
| 783 | APSPAPSPDSDSDSDSDGEEEEEEEG |
| 784 | PSPAPSPDSDSDSDSDGEEEEEEEGE |
| 785 | EKNDEDEPQKPEDKGDPEGPE (Tag 6236-1) |
| 786 | EKNDEDEPQKPEDKGDPEGPEA(Tag 6236-2) |
| 787 | EDEEEEEEEEEEEDEGPAPP (Tag 6755-1) |
| 788 | DEEEEEEEEEEEDEGPAPPS (Tag 6755-2) |
| 789 | EREPDPPDDRDASDGEDEKP |
| 790 | REPDPPDDRDASDGEDEKPP |
| 791 | GEREPDPPDDRDASDGEDEKP (Tag 7167-1) |
| 792 | EREPDPPDDRDASDGEDEKPP (Tag 7167-2) |
| 793 | EGEREPDPPDDRDASDGEDEKP (Tag 7167-3) |
| 794 | GEREPDPPDDRDASDGEDEKPP |
| 795 | EGEREPDPPDDRDASDGEDEKPP |
| 796 | EEEEEQPGKAPDPQDPQDAESD (Tag 7702-1) |
| 797 | QEPEEKQEPEEKQEPEEKQK |
| 798 | EPEEKQEPEEKQEPEEKQKP |
| 799 | PEEKQEPEEKQEPEEKQKPE |
| 800 | EEKQEPEEKQEPEEKQKPEA |
| 801 | EPEEKQEPEEKQEPEEKQKPE (Tag 8243-1) |
| 802 | QEPEEKQEPEEKQEPEEKQKPE (Tag 8243-2) |
| 803 | EPEEKQEPEEKQEPEEKQKPEA (Tag 8243-3) |
| 804 | AGDDDDDDDDSPDPESPDDS |
| 805 | GDDDDDDDDSPDPESPDDSE |
| 806 | DDDDDDDDSPDPESPDDSES |
| 807 | DDDDDDDSPDPESPDDSESD (Tag 8818-1) |
| 808 | DDDDDDSPDPESPDDSESDS (Tag 8818-2) |
| 809 | DDDDDSPDPESPDDSESDSE |
| 810 | DDDDSPDPESPDDSESDSES |
| 811 | DDDSPDPESPDDSESDSESE |
| 812 | DDSPDPESPDDSESDSESEK (Tag 8818-3) |
| 813 | DSPDPESPDDSESDSESEKE |
| 814 | SPDPESPDDSESDSESEKEE |
| 815 | PDPESPDDSESDSESEKEES |
| 816 | DPDQPREDPAEEEKEEKDAPE (Tag 9050-1) |
| 817 | EGKPENESEPKHEEEPKPEE |
| 818 | PENESEPKHEEEPKPEEKPE |
| 819 | ENESEPKHEEEPKPEEKPEE |
| 820 | NESEPKHEEEPKPEEKPEEE |
| 821 | ESEPKHEEEPKPEEKPEEEE |
| 822 | SEPKHEEEPKPEEKPEEEEK |
| 823 | PENESEPKHEEEPKPEEKPEE |
| 824 | ENESEPKHEEEPKPEEKPEEE |
| 825 | NESEPKHEEEPKPEEKPEEEE |
| 826 | ESEPKHEEEPKPEEKPEEEEK |
| 827 | KPENESEPKHEEEPKPEEKPEE |
| 828 | PEN ESEPKH EEEPKPEEKPEEE (Tag 9166-1) |
| 829 | ENESEPKHEEEPKPEEKPEEEE |
| 830 | NESEPKHEEEPKPEEKPEEEEK (Tag 9166-2) |
| 831 | KPENESEPKHEEEPKPEEKPEEE |
| 832 | PENESEPKHEEEPKPEEKPEEEE (Tag 9166-3) |
| 833 | ENESEPKHEEEPKPEEKPEEEEK |
| 834 | EGKPENESEPKHEEEPKPEEKPEE |
| 835 | GKPENESEPKHEEEPKPEEKPEEE |
| 836 | KPENESEPKHEEEPKPEEKPEEEE |
| 837 | PENESEPKHEEEPKPEEKPEEEEK |
| 838 | EGKPENESEPKHEEEPKPEEKPEEE |
| 839 | GKPENESEPKHEEEPKPEEKPEEEE |
| 840 | KPENESEPKHEEEPKPEEKPEEEEK |
| 841 | EGKPENESEPKHEEEPKPEEKPEEEE |
| 842 | GKPENESEPKHEEEPKPEEKPEEEEK |
| 843 | EGKPENESEPKHEEEPKPEEKPEEEEK |
| 844 | KPPEEDPEEQAEENPEGEQP |
| 845 | PPEEDPEEQAEENPEGEQPE (Tag 9590-1) |
| 846 | PEEDPEEQAEENPEGEQPEE |
| 847 | KPPEEDPEEQAEENPEGEQPE (Tag 9590-2) |
| 848 | PPEEDPEEQAEENPEGEQPEE |
| 849 | KPPEEDPEEQAEENPEGEQPEE (Tag 9590-3) |
| 850 | PDDDDESEDHDDPDNAHESP (Tag 9704-1) |
| 851 | GEPEPEPEPEPEPEPESEPE |
| 852 | EPEPEPEPEPEPEPESEPEP |
| 853 | PEPEPEPEPEPEPESEPEPE |
| 854 | EPEPEPEPEPEPESEPEPEP |
| 855 | PEPEPEPEPEPESEPEPEPE (Tag 9749-1) |
| 856 | GGEPEPEPEPEPEPEPESEPE |
| 857 | GEPEPEPEPEPEPEPESEPEP |
| 858 | EPEPEPEPEPEPEPESEPEPE |
| 859 | PEPEPEPEPEPEPESEPEPEP |
| 860 | EPEPEPEPEPEPESEPEPEPE |
| 861 | GGEPEPEPEPEPEPEPESEPEP |
| 862 | GEPEPEPEPEPEPEPESEPEPE |
| 863 | EPEPEPEPEPEPEPESEPEPEP |
| 864 | PEPEPEPEPEPEPESEPEPEPE |
| 865 | GGEPEPEPEPEPEPEPESEPEPE (Tag 9749-2) |
| 866 | GEPEPEPEPEPEPEPESEPEPEP |
| 867 | EPEPEPEPEPEPEPESEPEPEPE |
| 868 | GGEPEPEPEPEPEPEPESEPEPEP |
| 869 | GEPEPEPEPEPEPEPESEPEPEPE |
| 870 | GGEPEPEPEPEPEPEPESEPEPEPE (Tag 9749-3) |
| 871 | PEEEPDDQDAPDEHESPPPE (Tag 10346-1) |
| 872 | EEEPDDQDAPDEHESPPPED |
| 873 | PEEEPDDQDAPDEHESPPPED |
| 874 | EEEPDDQDAPDEHESPPPEDA (Tag 10346-2) |
| 875 | EEPDDQDAPDEHESPPPEDAP |
| 876 | PEEEPDDQDAPDEHESPPPEDA |
| 877 | EEEPDDQDAPDEHESPPPEDAP |
| 878 | PEEEPDDQDAPDEHESPPPEDAP (Tag 10346-3) |
| 879 | GPSSDDENEEESKPEKEDEP (Tag 11099-1) |
| 880 | PSSDDENEEESKPEKEDEPQ (Tag 11099-2) |
| 881 | SDDSDSEKRRPEEQEEEPQP (Tag 12127-1) |
| 882 | DDSDSEKRRPEEQEEEPQPR (Tag 12127-2) |
| 883 | DPREGEEEEEEDEPDPEAPE |
| 884 | PREGEEEEEEDEPDPEAPEN |
| 885 | DEEPGDPREGEEEEEEDEPDP |
| 886 | EEPGDPREGEEEEEEDEPDPE |
| 887 | EPGDPREGEEEEEEDEPDPEA |
| 888 | PGDPREGEEEEEEDEPDPEAP |
| 889 | GDPREGEEEEEEDEPDPEAPE |
| 890 | DPREGEEEEEEDEPDPEAPEN |
| 891 | PREGEEEEEEDEPDPEAPENG |
| 892 | EDEEPGDPREGEEEEEEDEPDP |
| 893 | DEEPGDPREGEEEEEEDEPDPE |
| 894 | EEPGDPREGEEEEEEDEPDPEA |
| 895 | EPGDPREGEEEEEEDEPDPEAP |
| 896 | PGDPREGEEEEEEDEPDPEAPE |
| 897 | GDPREGEEEEEEDEPDPEAPEN |
| 898 | DPREGEEEEEEDEPDPEAPENG |
| 899 | PREGEEEEEEDEPDPEAPENGS |
| 900 | PEEEDEEPGDPREGEEEEEEDEP |
| 901 | EEDEEPGDPREGEEEEEEDEPDP |
| 902 | EDEEPGDPREGEEEEEEDEPDPE |
| 903 | DEEPGDPREGEEEEEEDEPDPEA |
| 904 | EEPGDPREGEEEEEEDEPDPEAP |
| 905 | EPGDPREGEEEEEEDEPDPEAPE |
| 906 | PGDPREGEEEEEEDEPDPEAPEN |
| 907 | DPREGEEEEEEDEPDPEAPENGS |
| 908 | APEEEDEEPGDPREGEEEEEEDEP |
| 909 | PEEEDEEPGDPREGEEEEEEDEPD |
| 910 | EEEDEEPGDPREGEEEEEEDEPDP |
| 911 | EEDEEPGDPREGEEEEEEDEPDPE |
| 912 | EDEEPGDPREGEEEEEEDEPDPEA |
| 913 | DEEPGDPREGEEEEEEDEPDPEAP |
| 914 | EEPGDPREGEEEEEEDEPDPEAPE |
| 915 | EPGDPREGEEEEEEDEPDPEAPEN |
| 916 | AAPEEEDEEPGDPREGEEEEEEDEP |
| 917 | APEEEDEEPGDPREGEEEEEEDEPD |
| 918 | PEEEDEEPGDPREGEEEEEEDEPDP (Tag 13036-1) |
| 919 | EEEDEEPGDPREGEEEEEEDEPDPE |
| 920 | EEDEEPGDPREGEEEEEEDEPDPEA |
| 921 | EDEEPGDPREGEEEEEEDEPDPEAP |
| 922 | DEEPGDPREGEEEEEEDEPDPEAPE |
| 923 | EEPGDPREGEEEEEEDEPDPEAPEN |
| 924 | AAPEEEDEEPGDPREGEEEEEEDEPD |
| 925 | APEEEDEEPGDPREGEEEEEEDEPDP |
| 926 | PEEEDEEPGDPREGEEEEEEDEPDPE |
| 927 | EEEDEEPGDPREGEEEEEEDEPDPEA |
| 928 | EEDEEPGDPREGEEEEEEDEPDPEAP |
| 929 | EDEEPGDPREGEEEEEEDEPDPEAPE |
| 930 | DEEPGDPREGEEEEEEDEPDPEAPEN |
| 931 | AAPEEEDEEPGDPREGEEEEEEDEPDP |
| 932 | APEEEDEEPGDPREGEEEEEEDEPDPE |
| 933 | PEEEDEEPGDPREGEEEEEEDEPDPEA |
| 934 | EEEDEEPGDPREGEEEEEEDEPDPEAP |
| 935 | EEDEEPGDPREGEEEEEEDEPDPEAPE |
| 936 | EDEEPGDPREGEEEEEEDEPDPEAPEN |
| 937 | AAPEEEDEEPGDPREGEEEEEEDEPDPE |
| 938 | APEEEDEEPGDPREGEEEEEEDEPDPEA |
| 939 | PEEEDEEPGDPREGEEEEEEDEPDPEAP (Tag 13036-2) |
| 940 | EEEDEEPGDPREGEEEEEEDEPDPEAPE |
| 941 | EEDEEPGDPREGEEEEEEDEPDPEAPEN |
| 942 | APEEEDEEPGDPREGEEEEEEDEPDPEA P |
| 943 | PEEEDEEPGDPREGEEEEEEDEPDPEAP E |
| 944 | EEEDEEPGDPREGEEEEEEDEPDPEAPE N |
| 945 | APEEEDEEPGDPREGEEEEEEDEPDPEA PE |
| 946 | PEEEDEEPGDPREGEEEEEEDEPDPEAP EN |
| 947 | PRGAAAPEEEDEEPGDPREGEEEEEED EPDP |
| 948 | RGAAAPEEEDEEPGDPREGEEEEEEDE PDPE |
| 949 | AAPEEEDEEPGDPREGEEEEEEDEPDPE APE |
| 950 | APEEEDEEPGDPREGEEEEEEDEPDPEA PEN |
| 951 | PEEEDEEPGDPREGEEEEEEDEPDPEAP ENG |
| 952 | EEEDEEPGDPREGEEEEEEEDEPDPEAPE NGS |
| 953 | PRGAAAPEEEDEEPGDPREGEEEEEED EPDPE |
| 954 | AAPEEEDEEPGDPREGEEEEEEDEPDPE APEN |
| 955 | APEEEDEEPGDPREGEEEEEEDEPDPEA PENG |
| 956 | PEEEDEEPGDPREGEEEEEEDEPDPEAP ENGS |
| 957 | AAPEEEDEEPGDPREGEEEEEEDEPDPE APENG |
| 958 | APEEEDEEPGDPREGEEEEEEDEPDPEA PENGS |
| 959 | AAPEEEDEEPGDPREGEEEEEEEDEPDPE APENGS (Tag 13036-3) |
| 960 | KPPPSEGSDEEEEEEDEEDE |
| 961 | PPPSEGSDEEEEEEDEEDEE (Tag 14128-1) |
| 962 | PPPSEGSDEEEEEEDEEDEEERKP |
| 963 | KPPPSEGSDEEEEEEDEEDEEERKP (Tag 14128-2) |
| 964 | PPPSEGSDEEEEEEDEEDEEERKPQ |
| 965 | KPPPSEGSDEEEEEEDEEDEEERKPQ (Tag 14128-3) |
| 966 | EEEEEEEEEEEEEEEEAPPP |
| 967 | EEEEEEEEEEEEEEEAPPPP (Tag 16549-1) |
| 968 | EEEEEEEEEEEEEEAPPPPR |
| 969 | EEEEEEEEEEEEEEEEAPPPP |
| 970 | EEEEEEEEEEEEEEEAPPPPR |
| 971 | EEEEEEEEEEEEEEEEEAPPPP |
| 972 | EEEEEEEEEEEEEEEEAPPPPR |
| 973 | EEEEEEEEEEEEEEEEEEAPPPP |
| 974 | EEEEEEEEEEEEEEEEEAPPPPR (Tag 16549-2) |
| 975 | EEEEEEEEEEEEEEEEEEEAPPPP |
| 976 | EEEEEEEEEEEEEEEEEEAPPPPR |
| 977 | EEEEEEEEEEEEEEEEEEEEAPPPP |
| 978 | EEEEEEEEEEEEEEEEEEEAPPPPR |
| 979 | EEEEEEEEEEEEEEEEEEEEEAPPPP |
| 980 | EEEEEEEEEEEEEEEEEEEEAPPPPR |
| 981 | PDDDEEDEEEEEEEEEEEEEEEEEEEEE APPPP (Tag 16549-3) |
| 982 | EDSEEESQEEEAEGASEPPPP (Tag 16604-1) |
| 983 | QEDSEEESQEEEAEGASEPPPP (Tag 16604-2) |
| 984 | DHQEDSEEESQEEEAEGASEPPPP (Tag 16604-3) |
| 985 | DQSEEEEEEEKHPPKPAKPE (Tag 16741-1) |
| 986 | PAPAHRPPEDEGEENEGEEDE (Tag16991-1) |
| 987 | PAPAHRPPEDEGEENEGEEDEE (Tag16991-2) |
| 988 | QENGQREEEEEEKEPEAEPP (Tag 17199-1) |
| 989 | PAEGQENGQREEEEEEKEPEAEPP (Tag 17199-2) |
| 990 | EQEPEPEPEPEPEPEPEPEP (Tag 17936-1) |
| 991 | QEPEPEPEPEPEPEPEPEPE |
| 992 | EPEPEPEPEPEPEPEPEPEQ (Tag 17936-2) |
| 993 | EQEPEPEPEPEPEPEPEPEPE |
| 994 | QEPEPEPEPEPEPEPEPEPEQ |
| 995 | EQEPEPEPEPEPEPEPEPEPEQ (Tag 17936-3) |
| 996 | PEEEEEEEEEEEPASPPERK (Tag 18132-1) |
| 997 | PSPGSPRGQPQDQDDDEDDEED (Tag 18453-1) |
| 998 | PSPGSPRGQPQDQDDDEDDEEDE |
| 999 | RPSPGSPRGQPQDQDDDEDDEEDE (Tag 18453-2) |
| 1000 | PSPGSPRGQPQDQDDDEDDEEDEA (Tag 18453-3) |
| 1001 | AEDDDEEDEEEEEEEPDPDP (Tag 18866-1) |
| 1002 | EDDDEEDEEEEEEEPDPDPE (Tag 18866-2) |
| 1003 | KQEPPDPEEDKEENKDDSAS (Tag 19350-1) |
| 1004 | QEPPDPEEDKEENKDDSASK (Tag 19350-2) |
| 1005 | EDEDEDESSEEDSEDEEPPP (Tag 19511-1) |
| 1006 | DEDEDESSEEDSEDEEPPPK |
| 1007 | EDEDESSEEDSEDEEPPPKR |
| 1008 | DEDESSEEDSEDEEPPPKRR |
| 1009 | PDDSRDEDEDEDESSEEDSEDEEPPP (Tag19511-2) |
| 1010 | PKKEPDDSRDEDEDEDESSEEDSEDEEP PP |
| 1011 | PKKEPDDSRDEDEDEDESSEEDSEDEEP PPK |
| 1012 | PKKEPDDSRDEDEDEDESSEEDSEDEEP PPKR |
| 1013 | PKKEPDDSRDEDEDEDESSEEDSEDEEP PPKRR (Tag 19511-3) |
| 1014 | PEEEAAEEEEEEEERPKPSRP (Tag 22900-1) |
| 1015 | QPEEEAAEEEEEEEERPKPSRP |
| 1016 | EQPEEEAAEEEEEEEERPKPSRP (Tag 22900-2) |
| 1017 | EEQPEEEAAEEEEEEEERPKPSRP |
| 1018 | EEEQPEEEAAEEEEEEEERPKPSRP (Tag 22900-3) |
| 1019 | PEEEDDEAQQPQPQSGPEEAEE (Tag 34831-1) |
| 1020 | DKPEEEDDEAQQPQPQSGPEEAEE |
| 1021 | PEEEDDEAQQPQPQSGPEEAEEGE |
| 1022 | PEEEDDEAQQPQPQSGPEEAEEGEE |
| 1023 | EGDKPEEEDDEAQQPQPQSGPEEAEE (Ta 34831-2) |
| 1024 | DKPEEEDDEAQQPQPQSGPEEAEEGE |
| 1025 | KPEEEDDEAQQPQPQSGPEEAEEGEE |
| 1026 | PEEEDDEAQQPQPQSGPEEAEEGEEE |
| 1027 | PEEEDDEAQQPQPQSGPEEAEEGEEEE AERGP |
| 1028 | KPEEEDDEAQQPQPQSGPEEAEEGEEE EAERGP (Tag 34831-3) |
| 1029 | NNSEEEEDDDDEEEEPDKPP (Tag 39056-1) |
| 1030 | NSEEEEDDDDEEEEPDKPPA(Tag 39056-2) |
| 1031 | SEEEEDDDDEEEEPDKPPAN (Tag 39056-3) |

As shown by these examples, the protein aggregation rate reducing action of a peptide tag had low dependency on a specific amino acid sequence, also had low dependency on a protein from which it is derived, but had high dependency on amino acid contents.

### Example 6: Addition to Various Proteins and Aggregation Inhibiting Action

In the above Example, Y13-259 was used as the scFv. In this example, a VHH antibody (a heavy chain variable domain of a heavy chain antibody) was used. As the VHH antibody, iDab#6 binding to Ras was used. As the tag, Tag4-8 was used. The other conditions were the same as those employed in Example 1. As a result, the aggregation rate of the VHH antibody not having the tag was 57.89%, the aggregation rate of the VHH antibody having Tag4-8 at the C terminal was 8.77%, and thus, a strong aggregation inhibiting action was exhibited by the tag addition.

When SHSY5Y (human dopamine-like cell) that is a neuroblastoma cell line was used as the cell, scFv-6E (6E) was used as the scFv, and Tag4-8 or Tag18-1 was added as the tag to the C terminal and the N terminal, the aggregation rate of the Tag4-8 added scFv was 0.96%, the aggregation rate of the Tag18-1 added scFv was 1.14%, and thus, it was revealed that the tag addition makes a contribution to the low aggregation rate.

When D4 binding to botulinum toxin type A (SEQ ID NO: 1032:
QVQLQQSGGGLVQPGGSLRLSCAASGFTLDYYAIGWFRQAPGKEREGVLCISSSGGS TNYADSVKGRFTISRDNAKNTVYLQMNSLKPEDTAVYYCAADDLRCGSNWSSYFRGS WGQGTQVTVSS) was used as the VHH antibody, and the Tag4-8 was added as the tag to the C terminal of the VHH antibody, the aggregation rate of the Tag4-8 added D4 was 7.8%, and the aggregation rate of D4 not having the tag was 81.5%. This result reveals that the tag addition makes a contribution to the reduction of the aggregation rate of the VHH antibody.

### Example 7: Action Enhancement by Stabilization of Intracellular Antibody (Effect of Enhancement of Antibody Action on Amyloid Accumulation)

It is known that a central nervous system disease is caused by accumulation of amyloid in a nerve cell. When human α-synuclein fibril is extracellularly introduced into a nerve cell, synuclein fibril is formed with synuclein having a normal structure in the cell involved. When GFP-tagged synuclein has been expressed in the cell, the synuclein forms synuclein fibril together with the introduced human α-synuclein fibril, and the thus formed synuclein fibril can be observed with fluorescence of GFP. In this example, GFP-tagged synuclein was expressed in SHSY-5Y cell, and α-synuclein fibril (Cosmo Bio Co., Ltd., SYNO3) was extracellularly introduced into the SHSY-5Y cell. In this example, the ability of an antibody to reduce the synuclein fibril was tested. Specifically, as the antibody, scFv-6E binding to fibrilized synuclein (SEQ ID NO: 1033:
AEVQLLESGGGLVQPGGSLRLSCAASGFTFSSYAMSWVRQAPGKGLEWVSYIASGGD TTNYADSVKGRFTISRDNSKNTLYLQMNSLRAEDTAVYYCAKGASAFDYWGQGTLVT VSSGGGGSGGGGSGGGGSTDIQMTQSPSSLSASVGDRVTITCRASQSISSYLNWYQQ KPGKAPKLLIYAASYLQSGVPSRFSGSGSGTDFTLTISSLQPEDFATYYCQQSSNDP YTFGQGTKVEIKR) was used. The scFv-6E is an antibody not having a significant binding property to a monomer or oligomer of synuclein, and such an antibody selective or specific to synuclein fibril is suitable for selectively removing synuclein fibril. To the scFv-6E, a tag having the aggregation rate reducing action (Tag4-8 or Tagl8-1) and a degradation-inducing sequence (CMA (SEQ ID NO: 1034): MARVKKDQAEPLHRKFERQPPG) were added. An expression plasmid vector for the protein and the synuclein fibril were introduced into the cell respectively with X-trem GENE9 and Multifectam (Merck). The antibody was provided with an HA tag or a myc tag, and detected with an anti-HA tag antibody. The synuclein fibril was detected with an anti-phosphorylated α-synuclein antibody. These antibodies were specifically detected with Alexa 555 labeled antibody and Alexa 633 labeled antibody. Fluorescent stained cells were observed with Keyence BZ-X800.

The tag was added to the N terminal and the C terminal. In consideration that the aggregation rate of one having Tag4-8 added to the N terminal and the C terminal was 0.96% in the SHSY-5Y cell, that the aggregation rate of one having TAG18-1 added to the N terminal and the C terminal was 1.14% in the SHSY-5 cell, and that the aggregation rate of the scFv-E6 having no tag added was 41.6% in the HeLa cell, it seems that favorable aggregation inhibition was exhibited.

When a scFv binds to synuclein fibril, the lysosome is caused to target the synuclein fibril for a degradation-inducing sequence to degrade the synuclein fibril, and as a result, the amount of synuclein fibril in the cell is expected to be reduced. It was evaluated whether or not the reduction amount of the synuclein fibril is increased when the aggregation rate of the scFv was reduced by using the tag to increase the amount of functional scFv in the cell.

The antibody was expressed in the cell where the synuclein fibril was formed, and the number of synuclein fibril-positive cells was counted. A positive rate of the synuclein fibril was compared between a cell in which the antibody was expressed and a cell in which it was not expressed. The result was obtained as a rate of synuclein fibril-positive cells in antibody-positive cells/a rate of synuclein fibril-positive cells in antibody-negative cells (P/N).

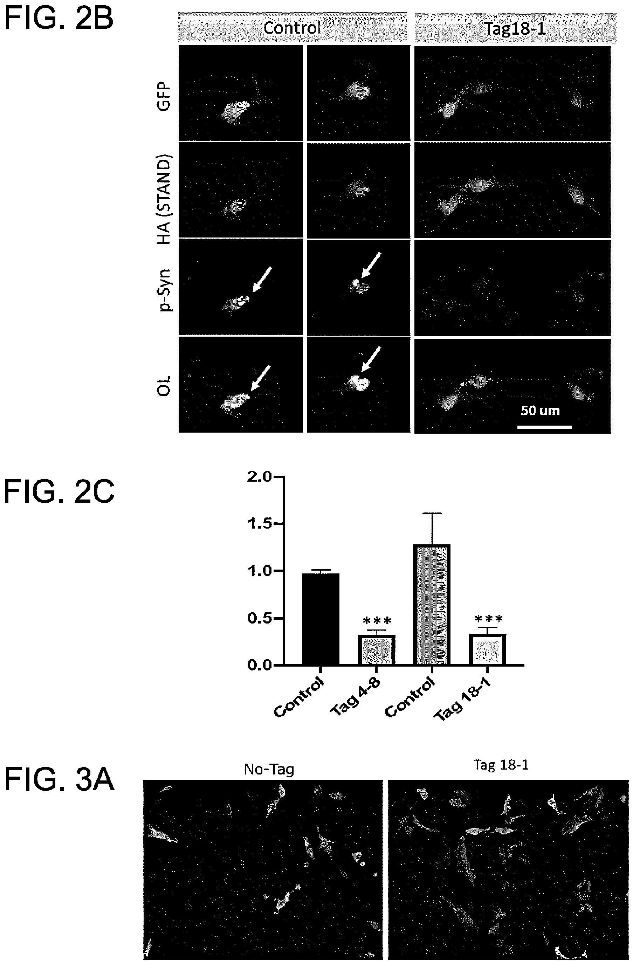

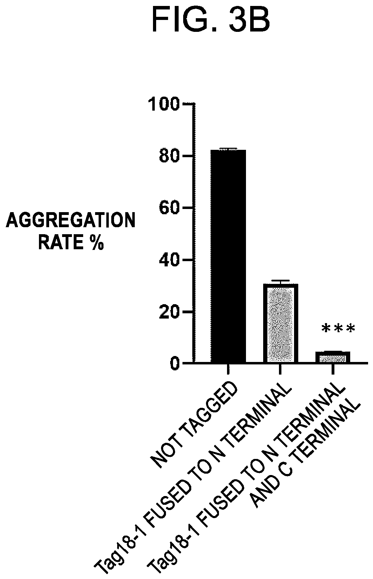

As illustrated in Figure 2, phosphorylated synuclein was lost in the cell into which the tagged scFv-E6 was introduced. The P/N was as illustrated in Figure 3. As illustrated in Figure 3, both the scFv-E6 tagged with Tag4-8 and the scFv-E6 tagged with Tag18-1 largely reduced the rate of synuclein-positive cells.

### Example 8: Enhancement of Action by Stabilization of Intracellular Antibody (Effect of Enhancement of Antibody Action for Recovering Function of CFTR)

The cystic fibrosis transmembrane conductance regulator (CFTR; UniprotKB/Swiss-Prot: P13569.3) is a negative ion channel expressed in epithelial membrane cells of the whole body, and abnormality thereof causes cystic fibrosis. F508 deletion mutation of CFTR (CFTRΔF508) is known as the most common mutation of CFTR, in which the 508th phenylalanine is deleted due to deletion of three nucleotides. As a result, CFTRΔF508 cannot be normally folded, strongly tends to form an aggregation, and is deemed to move onto the membrane in a smaller amount than the wild type. The scFv-C2 (SEQ ID NO: 1035:
EVQLLESGGGLVQPGGSLRLSCAASGFTFSSYAMSWVRQAPGKGLEWVSAISGSGGS TYYADSVKGRFTISRDNSKNTLYLQMNSLRAEDTAVYYCAKMRLGLFDYWGQGTLVT VSSGGGGSGGGGSGGGGEIVLTQSPGTLSLSPGERATLSCRASQSVSSSYLAWYQQK PGQAPRLLIYGASSRATGIPDRFSGSGSGTDFTLTISRLEPEDFAVYYCQQRGDVPP TFGQGTKVEIKAAA) binds to the NBD1 domain of CFTR, and thus can inhibit formation of an aggregation by the NBD1 (Lovato et al., Protein Engineering, Design and Selection, 20(12): 607-614, 2007). The scFv-C2 exhibits an effect of increasing the amount of CFTRΔF508 moving onto the membrane. In this example, it was evaluated whether or not aggregation of the mutant ΔF508 in the NBD1 domain in the HeLa cell can be inhibited by adding a tag (Tag18-1) to the scFv-C2. Plasmid vectors for expressing these proteins were introduced into HeLa cell with Lipo3000(TM). An antibody was tagged with a myc tag to be detected with a rabbit anti-myc tag antibody, and the NBD1 was tagged with a His tag to be detected with a mouse anti-His tag antibody. The mouse anti-His tag antibody was detected with Alexa 633-labeled anti-mouse IgG antibody, and the rabbit anti-myc tag antibody was detected with Alexa 488-labeled anti-rabbit IgG antibody. Fluorescent stained cells were observed with Keyence BZ-X800.

First, the aggregation rate of the scFv-C2 in HeLa cell was 82%. On the contrary, the aggregation rate of the scFv-C2 having Tag18-1 at the N terminal was 31%, and the aggregation rate of the scFv-C2 having Tag18-1 at the N terminal and the C terminal was 4.6% (see Figure 3A and Figure 3B). The aggregation rate of wild type NBD1 domain was 74% in a cell expressing scFv-C2, and the aggregation rate of the ΔF508 mutant of the NBD1 was 85% in a cell expressing scFv-C2. On the contrary, in a cell expressing scFv-C2 having Tag18-1 at the N terminal and the C terminal, the aggregation rate of the wild type NBD1 domain was 32%, and the aggregation rate of the ΔF508 mutant of NBD1 was 43%. In this manner, aggregation formation of the scFv-C2 itself could be inhibited by tagging the scFv-C2, and thus, the formation of an aggregation by the NBD1 could be inhibited. The inhibition of the aggregation of the NBD1 is expected to make a contribution to improvement of expression level of the NBD1 in cell membrane.

### Example 9: Effect of Amino Acid Substitution in Existing Tag

Through Examples described above, it was revealed that amino acid substitution for satisfying the condition needed for the peptide tag of the present disclosure, particularly amino acid substitution with P or N increases the aggregation rate reducing action of the tag. In this example, with some amino acids of PEST sequence substituted with N, the aggregation rates of the scFvs (Y14-259) having tags before and after the substitution were examined.

**[Table 15]**

| Table 15: Comparison of sequence between before and after substitution | |
|---|---|
| PEST(before substitution) SEQ ID NO: 1036 | YPYDVPDYAGSPQPVEDGEDEFCTPMACEANIQSGDSAAPMSAVHRHRL |
| PEST(after substitution) SEQ ID NO: 1037 | NNYDVPDNAGSPQPQEDGEDEFNNPQANEANQQSGDSNNPNSAVNRHNN |

After these scFvs were expressed in a cell, the aggregation rates of the scFvs were evaluated in the same manner as in Example 1, and the aggregation rate of the scFv having the tag before the substitution was 50.3%, but the aggregation rate of the scFv having the tag after the substitution was 18.0%. In this manner, it was revealed that the amino acid substitution for satisfying the condition needed for the peptide tag of the present disclosure, particularly the amino acid substitution with P or N increases the effect of inhibiting protein aggregation of the tag.

As described so far, various proteins including antibodies form aggregation in a cell, and thus, the functions can be partially or entirely impaired. A protein tag for inhibiting the formation of an aggregation can inhibit the aggregation formation of these proteins, and thus, can cause the proteins to exhibit their actions to be originally exhibited. A tag in which an acidic amino acid ratio is relatively low can be helpful in a scene where a tag having a high acidic amino acid ratio is difficult to use.

## Claims

1. A peptide, wherein
(a) 5% or more and less than 45% of amino acids contained in an amino acid sequence thereof are acidic amino acids, and
(b) 20% or more, and preferably 30% or more of the amino acids contained in the amino acid sequence are amino acids selected from the group consisting of F, P, Y, G, S, Q, N, and A, and
the peptide is capable of reducing an aggregation property in a cell of a protein linked to the peptide.

2. The peptide according to claim 1, wherein
(c) 30% or less, preferably 20% or less, more preferably 15% or less, and further preferably 10% or less of the amino acids contained in the amino acid sequence are amino acids selected from the group consisting of M, T, W, C, I, V, and L.

3. The peptide according to claim 1 or 2, wherein
(d) each of A and G constitutes less than 10% of the amino acids contained in the amino acid sequence.

4. The peptide according to any one of claims 1 to 3, wherein
(a) 20% or more and less than 45% of the amino acids contained in the amino acid sequence are acidic amino acids,
(b) 30% or more and less than 70% of the amino acids contained in the amino acid sequence are amino acids selected from the group consisting of F, P, Y, G, S, Q, N, and A,
(c) 20% or less of the amino acids contained in the amino acid sequence are amino acids selected from the group consisting of M, T, W, C, I, V, and L, and
(d) each A and G constitutes less than 10% of the amino acids contained in the amino acid sequence.

5. A peptide having an amino acid sequence set forth in any one of SEQ ID NOs: 2 to 11.

6. A nucleic acid encoding the peptide according to any one of claims 1 to 5.

7. A protein expression vector comprising: the nucleic acid according to claim 6 operably linked to a regulatory sequence; and a nucleic acid encoding a protein of interest in-frame to the nucleic acid according to claim 6.

8. The protein expression vector according to claim 7, wherein the protein of interest is an antibody, or an antigen-binding fragment of an antibody.

9. The protein expression vector according to claim 8, wherein the antigen-binding fragment of the antibody is a single chain Fv (scFv).

10. A fusion protein of the peptide according to any one of claims 1 to 5 and a protein of interest.

11. The fusion protein according to claim 10, wherein the protein of interest is an antibody, or an antigen-binding fragment of an antibody.

12. The fusion protein according to claim 11, wherein the antigen-binding fragment of the antibody is a single chain Fv (scFv).

13. A protein-producing cell comprising: the nucleic acid according to claim 6 operably linked to a regulatory sequence; and a nucleic acid encoding a protein of interest in-frame to the nucleic acid according to claim 6.

14. A method for selecting or identifying an amino acid sequence, comprising:
acquiring an amino acid sequence in which:
(a) 5% or more and less than 45% of amino acids contained in the amino acid sequence are acidic amino acids; and
(b) 20% or more of the amino acids contained in the amino acid sequence are amino acids selected from the group consisting of F, P, Y, G, S, Q, N, and A;
selecting or identifying an amino acid sequence of a peptide tag that, when a fusion protein of the peptide tag having the selected or identified amino acid sequence and a reference protein is expressed in a mammal cell (preferably in a human cell), provides reduction of a proportion of cells in which the fusion protein forms an aggregation, or the proportion which is not more than a predetermined value; and
obtaining the peptide tag having the amino acid sequence or a nucleic acid encoding the peptide tag.

15. The method according to claim 14, wherein the amino acid sequence to be acquired is the peptide according to any one of claims 1 to 5.

16. The method according to claim 14 or 15, wherein the amino acid sequence to be acquired is a group of amino acid sequences encoded by coding regions of human genome.

17. The method according to any one of claims 14 to 16, wherein the amino acid sequence to be acquired contains a neo-antigen.
